(19) **Europäisches Patentamt / European Patent Office / Office européen des brevets**

(11) **EP 4 089 681 B1**

(12) **EUROPEAN PATENT SPECIFICATION**

(45) Date of publication and mention
of the grant of the patent:
**12.03.2025  Bulletin 2025/11**

(21) Application number: **21823091.0**

(22) Date of filing: **07.06.2021**

(51) International Patent Classification (IPC):
**G16C 60/00** (2019.01)   **C10G 9/00** (2006.01)
**C10G 11/18** (2006.01)   **C10G 35/24** (2006.01)
**C10G 45/72** (2006.01)   **C10G 47/36** (2006.01)
**G16C 20/10** (2019.01)   **G16C 20/30** (2019.01)
**G16C 20/70** (2019.01)   **G06N 20/00** (2019.01)

(52) Cooperative Patent Classification (CPC):
**G16C 60/00; C10G 9/005; C10G 11/187;**
**C10G 35/24; C10G 45/72; C10G 47/36;**
G06N 20/00; G16C 20/10; G16C 20/30;
G16C 20/70; Y02P 90/30

(86) International application number:
**PCT/CN2021/098571**

(87) International publication number:
**WO 2021/249330 (16.12.2021 Gazette 2021/50)**

(54) **METHOD, APPARATUS AND SYSTEM FOR WHOLE-PROCESS OPTIMIZATION OF MOLECULAR-LEVEL OIL REFINERY PROCESSING, AND STORAGE MEDIUM**

VERFAHREN, VORRICHTUNG UND SYSTEM ZUR OPTIMIERUNG VON GANZEN PROZESSEN DER MOLEKULAREN ERDÖLRAFFINATIONSVERARBEITUNG UND SPEICHERMEDIUM

PROCÉDÉ, APPAREIL ET SYSTÈME POUR L'OPTIMISATION SUR TOUT LE PROCESSUS D'UN TRAITEMENT DE RAFFINERIE DE PÉTROLE AU NIVEAU MOLÉCULAIRE, ET SUPPORT DE STOCKAGE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.06.2020  CN 202010533876**

(43) Date of publication of application:
**16.11.2022  Bulletin 2022/46**

(73) Proprietor: **PetroChina Company Limited**
**Dongcheng District**
**Beijing 100007 (CN)**

(72) Inventors:
• **WANG, Hangzhou**
  **Beijing 100724 (CN)**
• **LIU, Yixin**
  **Beijing 100724 (CN)**
• **JI, Ye**
  **Beijing 100724 (CN)**

• **DUAN, Wei**
  **Beijing 100724 (CN)**
• **WANG, Lu**
  **Beijing 100724 (CN)**

(74) Representative: **Slingsby Partners LLP**
**1 Kingsway**
**London WC2B 6AN (GB)**

(56) References cited:
**CN-A- 104 484 714    CN-A- 106 444 672**
**CN-A- 109 777 528    CN-A- 109 859 805**
**CN-A- 111 899 795    US-A1- 2014 107 941**

• **LI J. ET AL: "Data-driven mathematical modeling and global optimization framework for entire petrochemical planning operations", AICHE JOURNAL, vol. 62, no. 9, 1 September 2016 (2016-09-01), US, pages 3020 - 3040, XP055465448, ISSN: 0001-1541, DOI: 10.1002/ aic.15220**

**(Cont. next page)**

- KHOR C. S. ET AL: "Petroleum refinery optimization", OPTIMIZATION AND ENGINEERING, SPRINGER NEW YORK LLC, US, vol. 18, no. 4, 3 November 2016 (2016-11-03), pages 943 - 989, XP036352192, ISSN: 1389-4420, [retrieved on 20161103], DOI: 10.1007/S11081-016-9338-X
- GUEDDAR T. ET AL: "Disaggregationaggregation based model reduction for refinery-wide optimization", COMPUTERS & CHEMICAL ENGINEERING, PERGAMON PRESS, OXFORD, GB, vol. 35, no. 9, 28 April 2011 (2011-04-28), pages 1838 - 1856, XP028245865, ISSN: 0098-1354, [retrieved on 20110505], DOI: 10.1016/J.COMPCHEMENG.2011.04.016
- CHERNYSHEVA E. A. ET AL: "Enhancing the Efficiency of Refinery Crude Oil Distillation Process by Optimized Preliminary Feedstock Blending (Review)", PETROLEUM CHEMISTRY, PLEIADES PUBLISHING, MOSCOW, vol. 60, no. 1, 1 January 2020 (2020-01-01), pages 1 - 15, XP037025633, ISSN: 0965-5441, [retrieved on 20200218], DOI: 10.1134/S0965544120010053
- REN Y. ET AL: "Molecular reconstruction: Recent progress toward composition modeling of petroleum fractions", CHEMICAL ENGENEERING JOURNAL, vol. 357, 11 September 2018 (2018-09-11), AMSTERDAM, NL, pages 761 - 775, XP055776630, ISSN: 1385-8947, DOI: 10.1016/j.cej.2018.09.083
- JOO E. ET AL: "CRACKER - a PC based simulator for industrial cracking furnaces", COMPUTERS & CHEMICAL ENGINEERING, PERGAMON PRESS, OXFORD, GB, vol. 24, no. 2-7, 15 July 2000 (2000-07-15), pages 1523 - 1528, XP026981867, ISSN: 0098-1354, [retrieved on 20000715]

**Description**

Technical Field

The present disclosure relates to the technical field of petroleum processing and, in particularly to an optimization method, apparatus, and system for a whole process of molecular-level oil refinery processing and a storage medium.

Background

Due to the large number of molecular species in crude oil, the refining and production process of oil products is very complicated. In order to maximize the utilization of crude oil resources, the most important point is to realize the overall optimal configuration of crude oil molecules in a whole process of oil refinery processing, so as to maximize the overall benefits. The overall optimization technology of a whole process of oil refinery processing has always been one of the research hotspots and difficulties. Li J. et al. (2016) "Data-driven mathematical modeling and global optimization framework for entire petrochemical planning operations", AICHE JOURNAL, vol. 62, no. 9, pages 3020-3040 discloses a modeling and global optimization-based planning formulation, which predicts product yields and properties for all of the production units within a highly integrated refinery-petrochemical complex. Khor C. S. et al. (2016) "Petroleum refinery optimization", OPTIMIZATION AND ENGINEERING, vol. 18, no. 4, pages 943-989 is a review article on the role of optimization methods in refineries for wide-ranging multiscale applications and activities including the traditional planning linear programming. Gueddar T. et al. (2011) "Disaggregation-aggregation based model reduction for refinery-wide optimization", COMPUTERS & CHEMICAL ENGINEERING, vol. 35, no. 9, pages 1838-1856 describes artificial neural network-based reduced nonlinear refinery optimization models developed by generating and using input-output data from a process simulator. Chernysheva E. A. et al. (2020) "Enhancing the Efficiency of Refinery Crude Oil Distillation Process by Optimized Preliminary Feedstock Blending (Review)", PETROLEUM CHEMISTRY, vol. 60, no. 1, pages 1-15 is a review article on the optimization of crude oil refining processes through optimal blending of components. Ren Y. et al. (2018) "Molecular reconstruction: Recent progress toward composition modeling of petroleum fractions", CHEMICAL ENGENEERING JOURNAL, vol. 357, pages 761-775 is a review article on molecular reconstruction models used in oil refinery modeling.

Summary

To address the problems of the related art, at least one embodiment of the present disclosure provides an optimization method, apparatus, and system for a whole process of molecular-level oil refinery processing and a storage medium.

In a first aspect, the present disclosure provides an optimization method for a whole process of molecular-level oil refinery processing, the optimization method including:

> acquiring molecular composition of crude oil;
> acquiring molecular composition of various fractions obtained by distillation of the crude oil according to physical properties of various single molecules in the molecular composition of the crude oil;
> respectively inputting, according to a preset feedstock ratio, the corresponding fractions into a product prediction model of a respective petroleum processing device as petroleum processing feedstocks, to obtain molecular composition of a corresponding predicted product and content of each single molecule in the predicted product;
> blending each of the predicted products which is used as a product blending feedstock according to a preset rule set, to obtain molecular composition of a plurality of mixed products and content of each single molecule in each of the mixed products;
> respectively calculating a product property of each of the mixed products according to the molecular composition of each of the mixed products and the content of each single molecule in each of the mixed products; and determining whether the product property of each of the mixed products meets any preset standard in a preset standard set;
> if the product property of each of the mixed products meets any preset standard in the preset standard set, acquiring a target parameter according to all mixed products and determining whether the target parameter meets a preset condition; and
> if the target parameter does not meet the preset condition, adjusting the preset feedstock ratio, a parameter in the product prediction model and a preset rule in the preset rule set, to re-obtain a plurality of mixed products until the product property of each of the mixed products meets any preset standard in the preset standard set and the target parameter meets the preset condition.

**In** the above technical solutions, if the target parameter meets the preset condition, it can be considered that the solution at this time is an optimal solution, and production and processing can be considered according to this solution. **In** particular, the preset feed ratio, the product prediction model, and the preset rule set may be outputted as a production and processing scheme.

Based on the above technical solutions, embodiments of the present disclosure may also make improvements as follows.

**In** combination with the first aspect, in a first embodiment of the first aspect, the optimization method further includes:

acquiring an input flow of petroleum processing feedstocks input to each of the petroleum processing devices;

determining whether each of the input flows meets a preset input flow range of the respective petroleum processing device; and

adjusting the preset feedstock ratio if any one of the input flows does not meet the preset input flow range of the respective petroleum processing device, and respectively re-inputting, according to the adjusted preset feedstock ratio, the corresponding fractions into the product prediction model of the respective petroleum processing device as petroleum processing feedstocks, until each of the input flows meets the preset input flow range of the respective petroleum processing device.

In the first embodiment of the first aspect, if each of the input flows meets the preset input flow range of the respective petroleum processing device, it is believed that a subsequent step may be carried out, namely, the step of obtaining molecular composition of a corresponding predicted product and content of each single molecule in the predicted product is performed.

In combination with the first aspect, in a second embodiment of the first aspect, the optimization method further includes:

acquiring molecular composition of the petroleum processing feedstocks input to each of the petroleum processing devices and content of each single molecule in the petroleum processing feedstocks;

calculating a physical property of each single molecule in the petroleum processing feedstocks, calculating a feedstock property of the petroleum processing feedstocks according to the physical property of each single molecule and the content of each single molecule in the petroleum processing feedstocks;

determining whether each of the feedstock properties meets a preset physical property restriction interval of the respective petroleum processing device; and

if any of the feedstock properties does not meet the preset physical property restriction interval of the respective petroleum processing device, adjusting the preset feedstock ratio, and respectively re-inputting, according to the adjusted preset feedstock ratio, the corresponding fractions into the product prediction model of the respective petroleum processing device as petroleum processing feedstocks, until each of the feedstock properties meets the preset physical property restriction interval of the respective petroleum processing device.

In the second embodiment of the first aspect, if each of the feedstock properties meets the preset physical property restriction interval of the respective petroleum processing device, it is believed that a subsequent step may be carried out, namely, the step of obtaining molecular composition of a corresponding predicted product and content of each single molecule in the predicted product is performed.

**In** combination with the first aspect, in a third embodiment of the first aspect, the acquiring a target parameter according to all mixed products and determining whether the target parameter meets a preset condition includes:

acquiring a product price of each of mixed products and a yield of each of mixed products;

calculating a product benefit of each of mixed products according to the yield of each of mixed products and the product price of each of mixed products;

accumulating the product benefit of each of mixed products to obtain a cumulative benefit;

acquiring a feedstock price of each group of the petroleum processing feedstocks and an operating cost of each of the petroleum processing devices;

subtracting feedstock prices of all petroleum processing feedstocks and operating costs of all petroleum processing devices from the cumulative benefit to obtain a comprehensive benefit;

serving the comprehensive benefit as the target parameter;

determining whether the comprehensive benefit reaches a maximum value;

determining that the target parameter meets the preset condition if the comprehensive benefit reaches the maximum value; and

determining that the target parameter does not meet the preset condition if the comprehensive benefit does not reach the maximum value.

**In** combination with the first aspect, in a fourth embodiment of the first aspect, the optimization method further includes:

if the product property of any mixed product does not meet any preset standard in the preset standard set, adjusting the preset rule in the preset rule set and blending each of the product blending feedstocks according to the adjusted preset rule set, to re-obtain a plurality of mixed products until the product property of each of the mixed products meets any preset standard in the preset standard set.

**In** combination with the first aspect, in a fifth embodiment of the first aspect, the respectively calculating a product property of each of the mixed products according to the molecular composition of each of the mixed products and the content of each single molecule in each of the mixed products includes:

acquiring molecular composition (i.e., first molecular composition) of each group of the product blending feedstocks and content (i.e., first component content) of each single molecule in each group of the product blending feedstocks;
based on the preset rule set, obtaining molecular composition (i.e., second molecular composition) of each of mixed products and content (i.e., second component content) of each single molecule in each of mixed products according to the first molecular composition of each group of the product blending feedstock and the first component content of each single molecule in each group of the product blending feedstocks;
calculating a physical property of each single molecule in each of the mixed products according to the number of groups of each group contained in each single molecule in each of the mixed products and a contribution value of each group to the physical property; and
calculating a product property of each of the mixed products according to the physical property and the second component content of each single molecule in each of the mixed products.

**In** combination with the second embodiment or the fifth embodiment of the first aspect, in a sixth embodiment of the first aspect, calculation of the physical property of each single molecule includes:

for each single molecule, acquiring the number of groups of each group constituting the single molecule and a contribution value of each group to the physical property; and
inputting the number of groups of each group constituting the single molecule and the contribution value of each group to the physical property into a pre-trained property calculation model, to acquire the physical property of the single molecule outputted by the pre-trained property calculation model.

**In** combination with the sixth embodiment of the first aspect, in a seventh embodiment of the first aspect, before the inputting the number of groups of each group constituting the single molecule and the contribution value of each group to the physical property into a pre-trained property calculation model, the optimization method further includes:

comparing the number of groups of each group constituting the single molecule with molecular information of a template single molecule with known physical properties pre-stored in a database, the molecular information including the number of groups of each group constituting the template single molecule;
determining whether there is a same template single molecule as the single molecule;
if there is a same template single molecule as the single molecule, outputting the physical properties of the template single molecule as a physical property of the single molecule; and
if there is not a same template single molecule as the single molecule, then performing the step of the inputting the number of groups of each group constituting the single molecule and the contribution value of each group to the physical property into a pre-trained property calculation model.

In combination with the first aspect, in an eighth embodiment of the first aspect, the acquiring molecular composition of various fractions obtained by distillation of the crude oil according to physical properties of various single molecules in the molecular composition of the crude oil includes:

acquiring each single molecule in the crude oil and the content of each single molecule;
calculating a boiling point of each single molecule, respectively; and
cutting the crude oil by distillation according to a preset fractional distillation range to obtain multiple fractions, and determining a single molecule and content of each single molecule contained in each of the fractions according to the boiling point and the content of each single molecule in the crude oil.

In combination with the eighth embodiment of the first aspect, in a ninth embodiment of the first aspect, the optimization method further includes:

for two fractions with adjacent distillation ranges, taking the fraction with a relatively high temperature in the distillation range as a first fraction, and taking the fraction with a relatively low temperature in the distillation range as a second fraction;

determining an overlapping interval of an overlapping distillation range of the first fraction and the second fraction; calculating content of distilled part into the first fraction of each single molecule in th e overlapping interval and calculating content of distilled part into the second fraction of ea ch single molecule in the overlapping interval according to the content of each single molecule and each single molecule corresponding to each boiling point of the overlapping interval; and

obtaining the content of each single molecule and each single molecule in each of the first fraction and the second fraction after the crude oil is cut by distillation according to the content of distilled part into the first fraction of each single molecule in the overlapping interval and the content of distilled part into the second fraction of each single molecule in the overlapping interval.

A minimum value and a maximum value of the overlapping interval of the overlapping distillation range of the first fraction and the second fraction are respectively determined by a separation index of the first fraction and the second fraction and the distillation cut temperature of the first fraction and the second fraction.

In this embodiment, the minimum value of the overlapping interval of the overlapping distillation range of the first fraction and the second fraction may be determined by: obtaining a difference value between the separation index of the first fraction and the second fraction, and determining the minimum value of the overlapping interval based on a product of the difference value and the distillation cut temperature of the first fraction and the second fraction.

For example, calculating the minimum value of the overlapping interval of the overlapping distillation range of the first fraction and the second fraction by the following formula: $T_{min} = T_{cut} \times (1 - SF)$.

In this embodiment, the maximum value of the overlapping interval of the overlapping distillation range of the first fraction and the second fraction may be determined by: obtaining a sum of the separation index of the first fraction and the second fraction, and determining the maximum value of the overlapping interval based on a product of the sum and the distillation cut temperature of the first fraction and the second fraction.

For example, calculating the maximum value of the overlapping interval of the overlapping distillation range of the first fraction and the second fraction by the following formula: $T_{max} = T_{cut} \times (1 + SF)$;

where, $T_{min}$ is the minimum value of the overlapping interval, $T_{max}$ is the maximum value of the overlapping interval, $T_{cut}$ is the distillation cut temperature of the first fraction and the second fraction, and *SF* is a separation index of the first fraction and the second fraction.

**In** combination with the ninth embodiment of the first aspect, in a tenth embodiment of the first aspect, the content of distilled part into the first fraction of each single molecule in the overlapping interval and the content of distilled part into the second fraction of each single molecule in the overlapping interval are determined by the following method:

for each single molecule whose boiling point is located in the overlapping interval, obtaining the difference between natural logarithms of the boiling point of the single molecule and the minimum value of the overlapping interval; determining the content of distilled part into the first fraction of the single molecule in the overlapping interval according to a product of the difference of natural logarithms and the content of the single molecule in the overlapping interval; and

determining the content of distilled part into the second fraction of the single molecule in the overlapping interval according to a difference between the content of the single molecule in the overlapping interval and the content of distilled part into the second fraction of the single molecule in the overlapping interval.

For example, the content of distilled part into the first fraction of each single molecule in the overlapping interval and the content of distilled part into the second fraction of each single molecule in the overlapping interval are calculated by the following equation:

$$C_h^i = \ln\ (\frac{T_i}{T_{min}}) \times C^i \quad ; C_l^i = C^i - C_h^i \ ;$$

where, $C_h^i$ is the content of distilled part into the first fraction of the i-th single molecule in all molecules with a boiling point located in the overlapping interval, which the i-th single molecule has the boiling point located in the overlapping interval, $C_l^i$ is the content of distilled part into the first fraction of the i-th single molecule in all molecules with a boiling point located in the overlapping interval, which the i-th single molecule has the boiling point located in the overlapping interval, $T_i$ is the

boiling point of the i-th single molecule, $T_{min}$ is the minimum value of the overlapping interval, and $C^i$ is the content of the i-th single molecule in all molecules with a boiling point located in the overlapping interval, which the i-th single molecule has the boiling point located in the overlapping interval.

In combination with the eighth embodiment of the first aspect, in an eleventh embodiment of the first aspect, the calculating a boiling point of each single molecule includes:

for each of the single molecule, acquiring the number of groups of each group constituting the single molecule and a contribution value of each group to the boiling point; and

inputting the number of groups of each group constituting the single molecule and the contribution value of each group to the boiling point into a pre-trained property calculation model, to acquire the boiling point of the single molecule outputted by the pre-trained property calculation model.

In combination with the eleventh embodiment of the first aspect, in a twelfth embodiment of the first aspect, before the inputting the number of groups of each group constituting the single molecule and the contribution value of each group to the boiling point into a pre-trained property calculation model, the optimization method further includes:

comparing the number of groups of each group constituting the single molecule with molecular information of a template single molecule with known boiling point pre-stored in a database, the molecular information including the number of groups of each group constituting the template single molecule;

determining whether there is a same template single molecule as the single molecule;

if there is a same template single molecule as the single molecule, outputting the boiling point of the template single molecule as a boiling point of the single molecule; and

if there is not a same template single molecule as the single molecule, then performing the step of the inputting the number of groups of each group constituting the single molecule and the contribution value of each group to the boiling point into a pre-trained property calculation model.

In combination with the six or tenth embodiment of the first aspect, in a tenth embodiment of the first aspect, a step of training the property calculation model includes:

constructing a property calculation model of a single molecule;

acquiring the number of groups of each group constituting a sample single molecule; wherein the physical property of the sample single molecule is known;

inputting the number of groups of each group constituting the sample single molecule into the property calculation model;

acquiring a predicted physical property of the sample single molecule outputted by the property calculation model;

if a deviation value between the predicted physical property and the physical property which is known is less than a preset deviation threshold, determining that the property calculation model converges, acquiring a contribution value corresponding to each group in the property calculation model which is converged, and storing the contribution value as a contribution value of the group to the physical property; and

if the deviation value between the predicted physical property and the physical property which is known is greater than or equal to the deviation threshold, adjusting a contribution value corresponding to each group in the property calculation model until the property calculation model converges.

In combination with the thirteenth embodiment of the first aspect, in a fourteenth embodiment of the first aspect,

$$f = a + \sum_i n_i \Delta f_i$$

the property calculation model is established as shown below:     ;

where, $f$ is the physical property of the single molecule, $n_i$ is the number of groups of the $i$-th group, $\Delta f_i$ is the contribution value of the $i$-th group to the physical property, and $a$ is an associated constant.

In combination with the thirteenth embodiment of the first aspect, in a fifteenth embodiment of the first aspect, the acquiring the number of groups of each group constituting a sample single molecule includes:

determining a primary group, the number of groups of the primary group, a multi-stage group, and the number of groups of the multi-stage group in all groups of the single molecule;

taking all groups constituting the single molecule as the primary group; and

taking various groups which coexist and contribute to a same physical property in common as the multi-stage group, and taking the number of the various groups as a level of the multi-stage group.

In combination with the fifteenth embodiment of the first aspect, in a sixteenth embodiment of the first aspect, the property calculation model is established as shown below:

$$f = a + \sum_i \mathrm{m}_{1i}\Delta f_{1i} + \sum_j m_{2j}\Delta f_{2j} \ldots\ldots + \sum_l m_{Nl}\Delta f_{Nl}\ ;$$

where, $f$ is the physical property of the single molecule, $m_{1i}$ is the number of groups of the $i$-th group in the primary group, $\Delta f_{1i}$ is the contribution value of the $i$-th group in the primary group to the physical property, $m_{2j}$ is the number of groups of the $j$-th group in a secondary group, $\Delta f_{2j}$ is the contribution value of the $j$-th group in the secondary group to the physical property, $m_{Nl}$ is the number of groups of the $l$-th group in an N-stage group, $\Delta f_{Nl}$ is the contribution value of the $l$-th group in the N-stage group to the physical property, $a$ is an associated constant, and $N$ is a positive integer greater than or equal to 2.

In combination with the eleventh embodiment of the first aspect, in a seventeenth embodiment of the first aspect, the acquiring the number of groups of each group constituting the single molecule includes:

determining a primary group, the number of groups of the primary group, a multi-stage group, and the number of groups of the multi-stage group in all groups of the single molecule;
taking all groups constituting the single molecule as the primary group; and
taking various groups which coexist and contribute to a same physical property in common as the multi-stage group, and taking the number of the various groups as a level of the multi-stage group.

In combination with the seventeenth embodiment of the first aspect, in an eighteenth embodiment of the first aspect, the inputting the number of groups of each group constituting the single molecule and the contribution value of each group to the physical property into a pre-trained property calculation model, to acquire the boiling point of the single molecule outputted by the pre-trained property calculation model includes:

calculating the boiling point of the single molecule according to the following the property calculation model:

$$T = \frac{SOL \times GROUP_1 + SOL \times GROUP_2 + \ldots\ldots + SOL \times GROUP_N}{(SOL \times Numh)^d + b} + c\ ;$$

where, $T$ is the boiling point of the single molecule, $SOL$ is a single molecule vector converted according to the number of groups of each group constituting the single molecule, $GROUP_{11}$ is a first contribution value vector converted according to a contribution value of the primary group to the boiling point, $GROUP_{12}$ is a second contribution value vector converted according to a contribution value of the secondary group to the boiling point, $GROUP_{1N}$ is an N-th contribution value vector converted according to a contribution value of the N-stage group to the boiling point, $Numh$ is the number of atoms other than the hydrogen atom in the single molecule, $d$ is a first preset constant, $b$ is a second preset constant, $c$ is a third preset constant, and $N$ is a positive integer greater than or equal to 2.

In combination with the eighteenth embodiment of the first aspect, in a nineteenth embodiment of the first aspect, the optimization method further includes:
converting the single molecule vector according to the number of groups of each group constituting the single molecule includes:

taking the number of species of groups as a dimension of the single molecule vector; and
taking the number of groups of each group as an element value of the corresponding dimension in the single molecule vector,
converting the first contribution value vector according to a contribution value of the primary group to the boiling point includes:

taking the number of types of primary groups as the dimension of the first contribution value vector; and
taking the contribution value of each primary group to the boiling point as an element value of the corresponding dimension in the first contribution value vector,

converting the second contribution value vector according to a contribution value of the secondary group to the boiling point includes:

taking the number of types of secondary groups as a dimension of the second contribution value vector; and taking the contribution value of each secondary group to the boiling point as an element value of the corresponding dimension in the second contribution value vector,
converting the N-th contribution value vector according to a contribution value of each N-stage group to the boiling point includes:

taking the number of types of N-stage groups as a dimension of the N-th contribution value vector; and taking the contribution value of each N-stage group to the boiling point as an element value of the corresponding dimension in the N-th contribution value vector.

In combination with the first aspect, in a twenty-second embodiment of the first aspect, the respectively inputting, according to a preset feedstock ratio, the corresponding fractions into a product prediction model of a respective petroleum processing device includes:

obtaining different amounts of each fraction according to the preset feedstock ratio, and respectively inputting each fraction into the product prediction model of the respective petroleum processing device,
the petroleum processing device includes a catalytic cracking unit, a delayed coking unit, a residue hydrotreating unit, a hydrocracking unit, a diesel hydro-upgrading unit, a diesel hydro-refining unit, a gasoline hydro-refining unit, a catalytic reforming unit and an alkylation unit.

In combination with the twentieth embodiment of the first aspect, in a twenty-first embodiment of the first aspect, a step of training the product prediction model includes:

establishing a product prediction model; wherein the product prediction model includes: a set of reaction rules including a plurality of reaction rules and a reaction rate algorithm;
acquiring sample feedstock information for a sample feedstock;
training the set of reaction rules by using the sample feedstock information, and fixing the set of reaction rules that has been trained; and
training the reaction rate algorithm by using the sample feedstock information, and fixing the reaction rate algorithm that has been trained, to obtain the product prediction model that has been trained.

In combination with the twenty-first embodiment of the first aspect, in a twenty-second embodiment of the first aspect, the sample feedstock information of the sample feedstock includes: molecular composition of the sample feedstock, molecular content of each molecule in the sample feedstock, molecular composition of an actual product corresponding to the sample feedstock, and actual content of each molecule in the actual product.

In combination with the twenty-second of the first aspect, in a twenty-third embodiment of the first aspect, the training the set of reaction rules by using the sample feedstock information includes:

processing the molecular composition of the sample feedstock according to a preset set of reaction rules, to obtain a reaction pathway corresponding to each molecule in the molecular composition of the sample feedstock;
obtaining first molecule composition of a device output product including the sample feedstock, an intermediate product, and a predicted product according to the reaction path corresponding to each molecule in the molecular composition of the sample feedstock; in the device output product, including: the sample feedstock, the intermediate product, and the predicted product;
calculating a first relative deviation according to the first molecular composition of the device output product and second molecular composition of the actual product;
if the first relative deviation meets a preset condition, fixing the set of reaction rules; and
if the first relative deviation does not meet the preset condition, adjusting a reaction rule in the set of reaction rules, and recalculating the first relative deviation according to the adjusted set of reaction rules until the first relative deviation meets the preset condition.

In combination with the twenty-third of the first aspect, in a twenty-fourth embodiment of the first aspect, the calculating a first relative deviation according to the first molecular composition of the device output product and second molecular composition of the actual product includes:

acquiring species of single molecules in the first molecule composition, to constitute a first set;
acquiring species of single molecules in the second molecule composition, to constitute a second set;
determining whether the second set is a subset of the first set;
if the second set is not a subset of the first set, obtaining a pre-stored relative deviation value that does not meet the preset condition as the first relative deviation; and
if the second set is a subset of the first set, calculating the first relative deviation by the following manner: determining a first relative deviation according to the proportion of the number of the predicted products that are not in the second set in the total number of predicted products.

For example, calculating the first relative deviation by the formula as follows:

$$x_1 = \frac{\mathrm{card}\big((M - M_1 - M_2) - M_3\big)}{\mathrm{card}\ (M - M_1 - M_2)}\ ;$$

where, $x_1$ is the first relative deviation, $M$ is the first set, $M_1$ is a set of species of single molecules in the molecular composition of the sample feedstock, $M_2$ is a set of species of single molecules in the molecular composition of the intermediate product, $M_3$ is the second set, and card represents the number of elements in the sets.

In combination with the twenty-second of the first aspect, in a twenty-fifth embodiment of the first aspect, the training the reaction rate algorithm by using the sample feedstock information includes:

calculating a reaction rate of a reaction path corresponding to each molecule in the molecular composition of the sample feedstock, respectively, according to the reaction rate algorithm;
obtaining predicted content of each molecule in a predicted product corresponding to the sample feedstock according to molecular content of each molecule in the sample feedstock and the reaction rate of the reaction path corresponding to the molecule;
calculating a second relative deviation according to the predicted content of each molecule in the predicted product and the actual content of each molecule in the actual product;
if the second relative deviation meets a preset condition, fixing the reaction rate algorithm; and
if the second relative deviation does not meet the preset condition, adjusting a parameter in the reaction rate algorithm, and recalculating the second relative deviation according to the adjusted reaction rate algorithm until the second relative deviation meets the preset condition.

In combination with the twenty-fifth of the first aspect, in a twenty-sixth embodiment of the first aspect, the calculating a reaction rate of a reaction path corresponding to each molecule in the molecular composition of the sample feedstock, respectively, according to the reaction rate algorithm includes:

calculating a reaction rate of each reaction path according to a reaction rate constant in the reaction rate algorithm; wherein the reaction rate constant is determined based on a transition state theoretical calculation method.

For example, determining the reaction rate constant according to a calculation formula as follows:

$$k = \frac{k_B E}{h} \exp(\frac{E\Delta S - \Delta E}{RE})\varphi \times P^\alpha\ ;$$

where, $k$ is the reaction rate constant, $k_B$ is the Boltzmann constant, $h$ is the Planck constant, $R$ is an ideal gas constant, $E$ is a temperature value of the environment at which the reaction path is located, exp is an exponential function with base of natural constant, $\Delta S$ is an entropy change before and after the reaction corresponding to the reaction rule corresponding to the reaction path, $\Delta E$ is a reaction energy barrier corresponding to the reaction rule corresponding to the reaction path, $\varphi$ is a catalyst activity factor, $P$ is a pressure value of the environment at which the reaction path is located, and $\alpha$ is a pressure influencing factor corresponding to the reaction rule corresponding to the reaction path.

The reaction rate of the reaction path is calculated according to the reaction rate constant.

In combination with the first aspect or in connection with the first, second, third, fourth, fifth, sixth, seventh, eighth, ninth, tenth, eleventh, twelfth, thirteenth, fourteenth, fifteenth, sixteenth, seventeenth, eighteenth, nineteenth, twentieth, twenty-first, twenty-second, twenty-third, twenty-fourth, twenty-fifth, twenty-sixth embodiments of the first aspect, in a twenty-seventh embodiment of the first aspect, each petroleum processing device corresponds to a set of reaction rules.

In a second aspect, the present disclosure provides an optimization apparatus for a whole process of molecular-level oil refinery processing, the optimization apparatus including:

an acquisition unit configured to acquire molecular composition of crude oil;

a first processing unit configured to acquire molecular composition of various fractions obtained by distillation of the crude oil according to physical properties of various single molecules in the molecular composition of the crude oil, and respectively input, according to a preset feedstock ratio, the corresponding fractions into a product prediction model of a respective petroleum processing device as petroleum processing feedstocks, to obtain molecular composition of a corresponding predicted product and content of each single molecule in the predicted product;

a second processing unit configured to blend each of the predicted products which is used as a product blending feedstock according to a preset rule set, to obtain molecular composition of a plurality of mixed products and content of each single molecule in each of the mixed products;

a third processing unit configured to respectively calculate a product property of each of the mixed products according to the molecular composition of each of the mixed products and the content of each single molecule in each of the mixed products; and determine whether the product property of each of the mixed products meets any preset standard in a preset standard set; and

a fourth processing unit configured to, if the product property of each of the mixed products meets any preset standard in the preset standard set, acquire a target parameter according to all mixed products and determine whether the target parameter meets a preset condition, and, if the target parameter does not meet the preset condition, adjust the preset feedstock ratio, a parameter in the product prediction model and a preset rule in the preset rule set, to re-obtain a plurality of mixed products until the product property of each of the mixed products meets any preset standard in the preset standard set and the target parameter meets the preset condition.

In combination with the second aspect, in a first embodiment of the second aspect, the optimization apparatus further includes:

a flow control unit configured to acquire an input flow of petroleum processing feedstocks input to each of the petroleum processing devices, determine whether each of the input flows meets a preset input flow range of the respective petroleum processing device; and adjust the preset feedstock ratio if any one of the input flows does not meet the preset input flow range of the respective petroleum processing device, and respectively re-input, according to the adjusted preset feedstock ratio, the corresponding fractions into the product prediction model of the respective petroleum processing device as petroleum processing feedstocks, until each of the input flows meets the preset input flow range of the respective petroleum processing device.

In combination with the second aspect, in a second embodiment of the second aspect, the optimization apparatus further includes:

an in-feed property control unit configured to acquire molecular composition of the petroleum processing feedstocks inputted to each of the petroleum processing devices and content of each single molecule in the petroleum processing feedstocks, calculate a physical property of each single molecule in the petroleum processing feedstocks, calculate a feedstock property of the petroleum processing feedstocks according to the physical property of each single molecule and the content of each single molecule in the petroleum processing feedstocks, determine whether each of the feedstock properties meets a preset physical property restriction interval of the respective petroleum processing device; and, if any of the feedstock properties does not meet the preset physical property restriction interval of the respective petroleum processing device, adjust the preset feedstock ratio and respectively re-input, according to the adjusted preset feedstock ratio, the corresponding fractions into the product prediction model of the respective petroleum processing device as petroleum processing feedstocks, until each of the feedstock properties meets the preset physical property restriction interval of the respective petroleum processing device.

In combination with the second aspect, in a third embodiment of the second aspect, the fourth processing unit is, in particular, configured to acquire a product price of each of mixed products and a yield of each of mixed products, calculate a product benefit of each of mixed products according to the yield of each of mixed products and the product price of each of mixed products, accumulate the product benefit of each of mixed products to obtain a cumulative benefit, acquire a feedstock price of each group of the petroleum processing feedstocks and an operating cost of each of the petroleum processing devices, subtract feedstock prices of all petroleum processing feedstocks and operating costs of all petroleum processing devices from the cumulative benefit to obtain a comprehensive benefit, serve the comprehensive benefit as the target parameter, determine whether the comprehensive benefit reaches a maximum value, determine that the target parameter meets the preset condition if the comprehensive benefit reaches the maximum value; and determine that the target parameter does not meet the preset condition if the comprehensive benefit does not reach the maximum value.

In combination with the second aspect, in a fourth embodiment of the second aspect, the optimization apparatus further includes:

a product property control unit configured to, if the product property of any mixed product does not meet any preset standard in the preset standard set, adjust the preset rule in the preset rule set and blend each of the product blending feedstocks according to the adjusted preset rule set, to re-obtain a plurality of mixed products until the product property of each of the mixed products meets any preset standard in the preset standard set.

In combination with the second aspect, in a fifth embodiment of the second aspect, the third processing unit is, in particular, configured to acquire first molecular composition of each group of the product blending feedstocks and first component content of each single molecule in each group of the product blending feedstocks, based on the preset rule set, obtain second molecular composition of each of mixed products and second component content of each single molecule in each of mixed products according to the first molecular composition of each group of the product blending feedstock and the first component content of each single molecule in each group of the product blending feedstocks, calculate a physical property of each single molecule in each of the mixed products according to the number of groups of each group contained in each single molecule in each of the mixed products and a contribution value of each group to the physical property; and calculate a product property of each of the mixed products according to the physical property and the second component content of each single molecule in each of the mixed products.

In combination with the fifth embodiment of the second aspect, in a sixth embodiment of the second aspect, the third processing unit is, in particular, configured to, for each single molecule, acquire the number of groups of each group constituting the single molecule and a contribution value of each group to the physical property; and input the number of groups of each group constituting the single molecule and the contribution value of each group to the physical property into a pre-trained property calculation model, to acquire the physical property of the single molecule outputted by the property calculation model.

In combination with the sixth embodiment of the second aspect, in a seventh embodiment of the second aspect, the optimization apparatus further includes:

a single molecule property template matching unit configured to compare the number of groups of each group constituting the single molecule with molecular information of a template single molecule with known physical properties pre-stored in a database, the molecular information including the number of groups of each group constituting the template single molecule, determine whether there is a same template single molecule as the single molecule, if there is a same template single molecule as the single molecule, output the physical properties of the template single molecule as a physical property of the single molecule; and if there is not a same template single molecule as the single molecule, then perform, by the third processing unit, the step of the inputting the number of groups of each group constituting the single molecule and the contribution value of each group to the physical property into a pre-trained property calculation model.

In combination with the second aspect, in an eighth embodiment of the second aspect, the first processing unit is, in particular, configured to acquire each single molecule in the crude oil and the content of each single molecule, calculate a boiling point of each single molecule, respectively, cut the crude oil by distillation according to a preset fractional distillation range to obtain multiple fractions, and determine a single molecule and content of the single molecule contained in each of the fractions according to the boiling point and the content of each single molecule in the crude oil.

In combination with the eighth embodiment of the second aspect, in a ninth embodiment of the second aspect, the first processing unit is further configured to, for two fractions with adjacent distillation ranges, take the fraction with a relatively high temperature in the distillation range as a first fraction, and take the fraction with a relatively low temperature in the distillation range as a second fraction;

calculate a minimum value of an overlapping interval of an overlapping distillation range of the first fraction and the second fraction by the following formula:

$$T_{\min} = T_{\text{cut}} \times (1 - SF)$$
;

and
calculate a maximum value of the overlapping interval of the overlapping distillation range of the first fraction and the second fraction by the following formula:

$$T_{\max} = T_{\text{cut}} \times (1 + SF)$$
;

where, $T_{\min}$ is the minimum value of the overlapping interval, $T_{\max}$ is the maximum value of the overlapping interval, $T_{\text{cut}}$ is the distillation cut temperature of the first fraction and the second fraction, and $SF$ is a separation index of the first fraction and the second fraction.

In combination with the ninth embodiment of the second aspect, in a tenth embodiment of the second aspect, the first processing unit is further configured to calculate content of distilled part into the first fraction of each single molecule in the overlapping int erval and calculate content of distilled part into the second fraction of each single molecule in the overlapping interval according to the content of each single molecule and each single molecule corresponding to each boiling point of the overlapping interval, and obtain the content of each single molecule and each single molecule in each of the first fraction and the second fraction after the crude oil is cut by distillation according to the content of distilled part into the first fraction of each single molecule in the overlapping interval and the content of distilled part into the second fraction of each single molecule in the overlapping interval,

wherein the content of distilled part into the first fraction of each single molecule in the overlapping interval and the content of distilled part into the second fraction of each single molecule in the overlapping interval are calculated by the following equation:

$$C_h^i = \ln \left( \frac{T_i}{T_{\min}} \right) \times C^i \; ; \; C_l^i = C^i - C_h^i \; ;$$

where, $C_h^i$ is the content of distilled part into the first fraction of the i-th single molecule in all molecules with a boiling point located in the overlapping interval, which the i-th single molecule has the boiling point located in the overlapping interval, $C_l^i$ is the content of distilled part into the first fraction of the i-th single molecule in all molecules with a boiling point located in the overlapping interval, which the i-th single molecule has the boiling point located in the overlapping interval, $T_i$ is the boiling point of the i-th single molecule, $T_{\min}$ is the minimum value of the overlapping interval, and $C^i$ is the content of the i-th single molecule in all molecules with a boiling point located in the overlapping interval, which the i-th single molecule the boiling point located in the overlapping interval.

In combination with the eighth embodiment of the second aspect, in an eleventh embodiment of the second aspect, the first processing unit is, in particular, configured to, for each of the single molecule, acquire the number of groups of each group constituting the single molecule and a contribution value of each group to the boiling point; and input the number of groups of each group constituting the single molecule and the contribution value of each group to the boiling point into a pre-trained property calculation model, to acquire the boiling point of the single molecule outputted by the pre-trained property calculation model.

In combination with the eleventh embodiment of the second aspect, in a twelfth embodiment of the second aspect, the optimization apparatus further includes:
a single-molecule boiling point template matching unit configured to compare the number of groups of each group constituting the single molecule with molecular information of a template single molecule with known boiling point pre-stored in a database, the molecular information including the number of groups of each group constituting the template single molecule, determine whether there is a same template single molecule as the single molecule, if there is a same template single molecule as the single molecule, output the boiling point of the template single molecule as a boiling point of the single molecule; and if there is not a same template single molecule as the single molecule, then perform, by the first processing unit, the step of the inputting the number of groups of each group constituting the single molecule and the contribution value of each group to the boiling point into a pre-trained property calculation model.

In combination with the sixth or eleventh embodiment of the second aspect, in a thirteenth embodiment of the second aspect, the optimization apparatus further includes:
a model training unit configured to construct a property calculation model of a single molecule, acquire the number of groups of each group constituting a sample single molecule; wherein the physical property of the sample single molecule is known, input the number of groups of each group constituting the sample single molecule into the property calculation model, acquire a predicted physical property of the sample single molecule outputted by the property calculation model, if a deviation value between the predicted physical property and the physical property which is known is less than a preset deviation threshold, determine that the property calculation model converges, acquire a contribution value corresponding to each group in the property calculation model which is converged, and store the contribution value as a contribution value of the group to the physical property; and, if the deviation value between the predicted physical property and the physical property which is known is greater than or equal to the deviation threshold, adjust a contribution value corresponding to each group in the property calculation model until the property calculation model converges.

In combination with the thirteenth embodiment of the second aspect, in a fourteenth embodiment of the second aspect, the model training unit is configured to establish the property calculation model as shown below:

$$f = a + \sum_i n_i \Delta f_i \; ;$$

where, $f$ is the physical property of the single molecule, $n_i$ is the number of groups of the $i$-th group, $\Delta f_i$ is the contribution value of the $i$-th group to the physical property, and $a$ is an associated constant.

In combination with the fourteenth embodiment of the second aspect, in a fifteenth embodiment of the second

# EP 4 089 681 B1

aspect, the model training unit is, in particular, configured to determine a primary group, the number of groups of the primary group, a multi-stage group, and the number of groups of the multi-stage group in all groups of the single molecule, take all groups constituting the single molecule as the primary group, take various groups which coexist and contribute to a same physical property in common as the multi-stage group, and take the number of the various groups as a level of the multi-stage group.

In combination with the fifteenth embodiment of the second aspect, in a sixth embodiment of the second aspect, the model training unit is configured to establish the property calculation model as shown below:

$$f = a + \sum_i \mathrm{m}_{1i} \Delta f_{1i} + \sum_j m_{2j} \Delta f_{2j} \ldots\ldots + \sum_l m_{Nl} \Delta f_{Nl}$$ ;

where, $f$ is the physical property of the single molecule, $m_{1i}$ is the number of groups of the $i$-th group in the primary group, $\Delta f_{1i}$ is the contribution value of the $i$-th group in the primary group to the physical property, $m_{2j}$ is the number of groups of the $j$-th group in a secondary group, $\Delta f_{2j}$ is the contribution value of the $j$-th group in the secondary group to the physical property, $m_{Nl}$ is the number of groups of the $l$-th group in an N-stage group, $\Delta f_{Nl}$ is the contribution value of the $l$-th group in the N-stage group to the physical property, $a$ is an associated constant, and $N$ is a positive integer greater than or equal to 2.

In combination with the eleventh embodiment of the second aspect, in a seventeenth embodiment of the second aspect, the first processing unit is, in particular, configured to determine a primary group, the number of groups of the primary group, a multi-stage group, and the number of groups of the multi-stage group in all groups of the single molecule, take all groups constituting the single molecule as the primary group, take various groups which coexist and contribute to a same physical property in common as the multi-stage group, and take the number of the various groups as a level of the multi-stage group.

In combination with the seventeenth embodiment of the second aspect, in an eighteenth embodiment of the second aspect, the first processing unit is, in particular, configured to calculate the boiling point of the single molecule according to the following the property calculation model:

$$T = \frac{SOL \times GROUP_1 + SOL \times GROUP_2 + \ldots\ldots + SOL \times GROUP_N}{(SOL \times Numh)^d + b} + c$$ ;

where, $T$ is the boiling point of the single molecule, $SOL$ is a single molecule vector converted according to the number of groups of each group constituting the single molecule, $GROUP_{11}$ is a first contribution value vector converted according to a contribution value of the primary group to the boiling point, $GROUP_{12}$ is a second contribution value vector converted according to a contribution value of the secondary group to the boiling point, $GROUP_{1N}$ is an N-th contribution value vector converted according to a contribution value of the N-stage group to the boiling point, $Numh$ is the number of atoms other than the hydrogen atom in the single molecule, $d$ is a first preset constant, $b$ is a second preset constant, $c$ is a third preset constant, and $N$ is a positive integer greater than or equal to 2.

In combination with the eighteenth embodiment of the second aspect, in a nineteenth embodiment of the second aspect, the first processing unit is, in particular, configured to take the number of species of groups as a dimension of the single molecule vector, and take the number of groups of each group as an element value of the corresponding dimension in the single molecule vector;

the first processing unit is, in particular, configured to take the number of types of primary groups as a dimension of the first contribution value vector, and take the contribution value of each primary group to the boiling point as an element value of the corresponding dimension in the first contribution value vector;
the first processing unit is, in particular, configured to take the number of types of secondary groups as a dimension of the second contribution value vector, and take the contribution value of each secondary group to the boiling point as an element value of the corresponding dimension in the second contribution value vector; and
the first processing unit is, in particular, configured to take the number of types of N-stage groups as a dimension of the N-th contribution value vector and take the contribution value of each N-stage group to the boiling point as an element value of the corresponding dimension in the N-th contribution value vector.

In combination with the second aspect, in a twentieth embodiment of the second aspect, the first processing unit is, in particular, configured to obtain different amounts of each fraction according to the preset feedstock ratio, and respectively input each fraction into the product prediction model of the respective petroleum processing device, and the petroleum processing device includes a catalytic cracking unit, a delayed coking unit, a residue hydrotreating unit, a hydrocracking unit, a diesel hydro-upgrading unit, a diesel hydro-refining unit, a gasoline hydro-refining unit, a catalytic

14

reforming unit and an alkylation unit.

In combination with the twentieth embodiment of the second aspect, in a twenty-first embodiment of the second aspect, the optimization apparatus further includes:

a model training unit configured to establish a product prediction model, acquire sample feedstock information for a sample feedstock, train the set of reaction rules by using the sample feedstock information, fix the set of reaction rules that has been trained; and train the reaction rate algorithm by using the sample feedstock information, and fix the reaction rate algorithm that has been trained, to obtain the product prediction model that has been trained; wherein the product prediction model includes: a set of reaction rules including a plurality of reaction rules and a reaction rate algorithm.

In combination with the twenty-first embodiment of the second aspect, in a twenty-second embodiment of the second aspect, the sample feedstock information of the sample feedstock includes: molecular composition of the sample feedstock, molecular content of each molecule in the sample feedstock, molecular composition of an actual product corresponding to the sample feedstock, and actual content of each molecule in the actual product.

In combination with the twenty-second embodiment of the second aspect, in a twenty-third embodiment of the second aspect, the model training unit is, in particular, configured to process the molecular composition of the sample feedstock according to a preset set of reaction rules, to obtain a reaction pathway corresponding to each molecule in the molecular composition of the sample feedstock, obtain first molecule composition of a device output product including the sample feedstock, an intermediate product, and a predicted product according to the reaction path corresponding to each molecule in the molecular composition of the sample feedstock, in the device output product, including: the sample feedstock, the intermediate product, and the predicted product, calculate a first relative deviation according to the first molecular composition of the device output product and second molecular composition of the actual product, if the first relative deviation meets a preset condition, fix the set of reaction rules, and, if the first relative deviation does not meet the preset condition, adjust a reaction rule in the set of reaction rules, and recalculate the first relative deviation according to the adjusted set of reaction rules until the first relative deviation meets the preset condition.

In combination with the twenty-third embodiment of the second aspect, in a twenty-fourth embodiment of the second aspect, the model training unit is, in particular, configured to acquire species of single molecules in the first molecule composition, to constitute a first set, acquire species of single molecules in the second molecule composition, to constitute a second set, determine whether the second set is a subset of the first set, if the second set is not a subset of the first set, obtain a pre-stored relative deviation value that does not meet the preset condition as the first relative deviation, and, if the second set is a subset of the first set, calculate the first relative deviation by a calculating formula as follows:

$$x_1 = \frac{\mathrm{card}\big((M - M_1 - M_2) - M_3\big)}{\mathrm{card}\ (M - M_1 - M_2)}\ ;$$

where, $x_1$ is the first relative deviation, $M$ is the first set, $M_1$ is a set of species of single molecules in the molecular composition of the sample feedstock, $M_2$ is a set of species of single molecules in the molecular composition of the intermediate product, $M_3$ is the second set, and card represents the number of elements in the sets.

In combination with the twenty-third embodiment of the second aspect, in a twenty-fifth embodiment of the second aspect, the model training unit is, in particular, configured to calculate a reaction rate of a reaction path corresponding to each molecule in the molecular composition of the sample feedstock, respectively, according to the reaction rate algorithm, obtain predicted content of each molecule in the predicted product corresponding to the sample feedstock according to molecular content of each molecule in the sample feedstock and the reaction rate of the reaction path corresponding to the molecule, calculate a second relative deviation according to the predicted content of each molecule in the predicted product and the actual content of each molecule in the actual product, if the second relative deviation meets a preset condition, fix the reaction rate algorithm, and, if the second relative deviation does not meet the preset condition, adjust a parameter in the reaction rate algorithm, and recalculate the second relative deviation according to the adjusted reaction rate algorithm until the second relative deviation meets the preset condition.

In combination with the twenty-fifth embodiment of the second aspect, in a twenty-sixth embodiment of the second aspect, the model training unit is, in particular, configured to calculate a reaction rate of each reaction path according to a reaction rate constant in the reaction rate algorithm;

wherein the reaction rate constant is determined according to a calculation formula as follows:

$$k = \frac{k_B E}{h} \exp(\frac{E\Delta S - \Delta E}{RE})\varphi \times P^\alpha\ ;$$

where, $k$ is the reaction rate constant, $k_B$ is the Boltzmann constant, $h$ is the Planck constant, $R$ is an ideal gas constant, $E$ is a temperature value of the environment at which the reaction path is located, exp is an exponential function with base of natural constant, $\Delta S$ is an entropy change before and after the reaction corresponding to the reaction rule corresponding to the reaction path, $\Delta E$ is a reaction energy barrier corresponding to the reaction rule corresponding to

the reaction path, $\varphi$ is a catalyst activity factor, $P$ is a pressure value of the environment at which the reaction path is located, and $\alpha$ is a pressure influencing factor corresponding to the reaction rule corresponding to the reaction path.

In combination with the twentieth, twenty-first, twenty-second, twenty-third, twenty-fourth, twenty-fifth, or twenty-sixth embodiments of the second aspect, in a twenty-seventh embodiment of the second aspect, each petroleum processing device corresponds to a set of reaction rules.

In a third aspect, the embodiments of the present disclosure provide an optimization system for a whole process of molecular-level oil refinery processing including a processor, a communication interface, a memory, and a communication bus, wherein the processor, the communication interface, and the memory are in communication with each other via the communication bus;

the memory is configured to store a computer program; and
the processor is configured to carry out the optimization method according to any embodiment of the first aspects when executing the program stored in the memory.

In a fourth aspect, the embodiments of the present disclosure provide a computer-readable storage medium, the computer-readable storage medium has stored therein one or more programs, the one or more programs being executable by one or more processors to implement the optimization method according to any embodiment of the first aspects.

The above-described technical solutions provided by embodiments of the present disclosure have the following advantages over the related art: in the embodiments of the present disclosure, a plurality of mixed products are obtained by acquiring molecular composition of crude oil, obtaining the molecular composition of different fractions of crude oil after distillation, obtaining the molecular composition and the content of each single molecule of the predicted products of different fractions processed by the product prediction model of the respective petroleum processing device, and blending the predicted products; when the physical properties of any mixed product do not meet any preset standard, or when a target parameter of the mixed products does not meet a preset condition, the proportion of different fractions introduced into the respective petroleum processing device is adjusted, an operating parameter in a product prediction model is adjusted, a mixing rule for mixing the predicted products is adjusted, and the mixed products are re-obtained, until the physical properties of all mixed products meet any preset standard and the target parameter of all mixed products meets the preset condition; the final predicted products are predicted by adjusting the proportion of fractions for secondary processing, the mixed products are obtained by blending according to the preset mixing rules, so as to ensure that the physical properties of the finally mixed products meet the preset standard, and the target parameter of the mixed products meets the preset conditions; and the production efficiency is improved by simulating and optimizing the production process.

Brief Description of the Drawings

FIG. 1 is a schematic flow diagram of an optimization method for a whole process of molecular-level oil refinery processing according to an embodiment of the present disclosure;
FIG. 2 is a schematic flow diagram of an optimization method for a whole process of molecular-level oil refinery processing according to another embodiment of the present disclosure;
FIG. 3 is a schematic flow diagram (one) of an optimization method for a whole process of molecular-level oil refinery processing according to still another embodiment of the present disclosure;
FIG. 4 is a schematic flow diagram (two) of an optimization method for a whole process of molecular-level oil refinery processing according to yet another embodiment of the present disclosure;
FIG. 5 is a schematic flow diagram (three) of an optimization method for a whole process of molecular-level oil refinery processing according to yet another embodiment of the present disclosure;
FIG. 6 is a schematic flow diagram (four) of an optimization method for a whole process of molecular-level oil refinery processing according to yet another embodiment of the present disclosure;
FIG. 7 is a schematic flow diagram (five) of an optimization method for a whole process of molecular-level oil refinery processing according to yet another embodiment of the present disclosure;
FIG. 8 is a schematic flow diagram (six) of an optimization method for a whole process of molecular-level oil refinery processing according to yet another embodiment of the present disclosure;
FIG. 9 is a schematic flow diagram (seven) of an optimization method for a whole process of molecular-level oil refinery processing according to yet another embodiment of the present disclosure;
FIG. 10 is a schematic flow diagram (eight) of an optimization method for a whole process of molecular-level oil refinery processing according to yet another embodiment of the present disclosure;

FIG. 11 is a schematic flow diagram (nine) of an optimization method for a whole process of molecular-level oil refinery processing according to yet another embodiment of the present disclosure;

FIG. 12 is a schematic flow diagram (ten) of an optimization method for a whole process of molecular-level oil refinery processing according to yet another embodiment of the present disclosure;

FIG. 13 is a schematic flow diagram (eleven) of an optimization method for a whole process of molecular-level oil refinery processing according to yet another embodiment of the present disclosure;

FIG. 14 is a schematic structural diagram of an optimization apparatus for a whole process of molecular-level oil refinery processing according to yet another embodiment of the present disclosure;

FIG. 15 is a schematic structural diagram of an optimization system for a whole process of molecular-level oil refinery processing according to yet another embodiment of the present disclosure; and

FIG. 16 is schematic structural diagram of an optimization system for a whole process of molecular-level oil refinery processing according to yet another embodiment of the present disclosure.

## Detailed Description

In order to make the objects, technical solutions, and advantages of the embodiments of the disclosure more fully apparent, it will be clear, fully described, and fully described in connection with the accompanying drawings, which are incorporated in and constitute a part of this specification, which illustrate some, but not all embodiments of the disclosure. Based on the embodiments in this disclosure, those of ordinary skill in the art, with no creative effort, are within the scope of the disclosure.

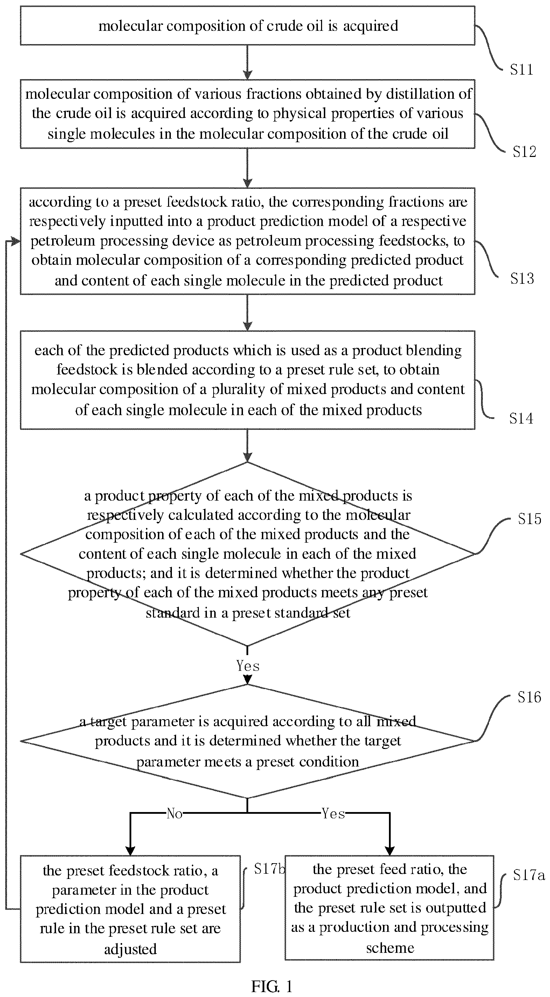

As shown in FIG. 1, an embodiment of the present disclosure provides an optimization method for a whole process of molecular-level oil refinery processing. Referring to FIG. 1, the optimization method includes the following steps:

S11, molecular composition of crude oil is acquired.

In this embodiment, there are many species of molecules in crude oil, different single molecules have different boiling points, and they need to be separated by distillation at different temperatures. Generally, a single molecule with a larger molecular weight in crude oil has a higher boiling point and is more difficult to separate. In the process of crude oil separation, the distillation range is divided according to the type of distilled oil and boiling points of molecules, each distillation range corresponding to type of distilled oil, to complete the separation of crude oil. In this step, single molecules in crude oil and content corresponding to each single molecule are acquired.

In this embodiment, the molecular composition of the petroleum processing feedstocks may be determined by one or more of a comprehensive two-dimensional gas chromatography method, a quaternary rod gas chromatography-mass spectrometer detection method, a gas chromatography/field ionization-time-of-flight mass spectrometry detection method, a gas chromatography method, a near-infrared spectroscopy method, a nuclear magnetic resonance spectroscopy method, a Raman spectroscopy method, a Fourier transform ion cyclotron resonance mass spectrometry method, an electrostatic field rail trap mass spectrometry method, and an ion mobility mass spectrometry method. In addition, the molecular composition of the petroleum processing feedstocks may also be determined in other ways, such as ASTM D2425, SH/T 0606, and ASTM D8144-18.

The molecular detection method described above may detect the structure of the molecule and thereby obtaining the species of the molecule. However, due to the large number of molecular species in crude oil, although the crude oil can no longer be detected when the crude oil is reused after the crude oil is detected once, the workload of detecting each single molecule is large and time-consuming. Therefore, in this scheme, single molecules may also be constructed based on the structure-oriented lumped molecular characterization method. The structure-oriented lumped molecular characterization method namely is the SOL molecular characterization method, which uses 24 structural increment fragments to characterize the basic structure of complex hydrocarbon molecules. Any single petroleum molecule may be represented by a specific set of structural increment fragments. The SOL molecular characterization method is lumped at the molecular scale, reducing the number of molecules in the actual system from millions to thousands, greatly reducing the complexity of the simulation. This characterization method may represent not only alkanes, cycloalkanes, up to complex aromatic structures containing 50-60 carbon atoms, but also olefins or cycloolefins as intermediate products or secondary reaction products, and also consider sulfur, nitrogen, oxygen and other heteroatom compounds.

In this embodiment, the molecular composition of crude oil is information of various molecules (single molecules) in crude oil, such as single molecules contained in the feedstock, a species of a single molecule, a volume and content of each single molecule.

S12, molecular composition of various fractions obtained by distillation of the crude oil is acquired according to physical properties of various single molecules in the molecular composition of the crude oil.

In this embodiment, the boiling point of each single molecule in the crude oil can be calculated separately, the fractional distillation range can be determined based on the boiling point and content of each single molecule, and the crude oil can be distilled and cut according to the fractional distillation range to obtain multiple fractions. **In** this step, since the crude oil is distilled based on the physical property of a single molecule, the molecular composition of each fraction obtained after crude oil distillation can be known.

S 13, according to a preset feedstock ratio, the corresponding fractions are respectively inputted into a product prediction model of a respective petroleum processing device as petroleum processing feedstocks, to obtain molecular composition of a corresponding predicted product and content of each single molecule in the predicted product.

In this embodiment, the corresponding fractions are used as petroleum processing feedstocks for secondary processing, wherein the preset feedstock ratio namely is the ratio of each fraction input into different petroleum processing devices, respectively. Through the product prediction model of each petroleum processing device, combined with the molecular composition of the fraction input to the petroleum processing device, the molecular composition in the predicted product and the content of each single molecule in the predicted product are obtained.

In this embodiment, the fractions obtained after distillation of crude oil include light fractions and heavy fractions. Among them, light fractions, such as naphtha, do not need secondary processing, while heavy fractions generally require different secondary processing, so that heavy fractions are converted into light oil products to improve the properties of oil products. In this solution, the corresponding fractions are input into the petroleum processing device for processing according to the preset feedstock ratio. The preset feedstock ratio includes: the type and amount of the fractions input to the petroleum processing device, and the fraction that does not require secondary processing is no longer preset.

In this embodiment, the product prediction model has been trained and optimized. Through the product prediction model, it is possible to adjust the reaction conditions in the petroleum processing device, such as pressure, temperature and space velocity after the petroleum processing feedstocks are input into the petroleum processing device, so as to suppress the progress of certain reactions or improve the progress of certain reactions to control the formation of products. In this step, the product situation under a certain set condition may be obtained.

The petroleum processing device includes a catalytic cracking unit, a delayed coking unit, a residue hydrotreating unit, a hydrocracking unit, a diesel hydro-upgrading unit, a diesel hydro-refining unit, a gasoline hydro-refining unit, a catalytic reforming unit and an alkylation unit.

S14, each of the predicted products which is used as a product blending feedstock is blended according to a preset rule set, to obtain molecular composition of a plurality of mixed products and content of each single molecule in each of the mixed products.

In this embodiment, the predicted products inputted by each petroleum processing device are blended as a product blending feedstock, wherein each set of preset rules in the preset rule set includes the type and amount of the predicted product used. Corresponding mixed products are obtained by mixing the predicted products outputted by different petroleum processing devices, wherein the mixed products include but are not limited to gasoline products such as automotive oil, lubricating oil, hydraulic oil, gear oil, cutting oil and so on for vehicles. The production planning may be completed by blending the product blending feedstocks so that the obtained mixed products meet the national standards of the corresponding products.

In this embodiment, according to the molecular composition of the predicted product and the content of each single molecule in the predicted product, combined with the preset rule set, the molecular composition of the different mixed products and the content of each single molecule in each of the mixed products are obtained.

S15, a product property of each of the mixed products is respectively calculated according to the molecular composition of each of the mixed products and the content of each single molecule in each of the mixed products; and it is determined whether the product property of each of the mixed products meets any preset standard in a preset standard set.

In this embodiment, the product property of each of mixed products is calculated separately. The various single molecules included in each of mixed products are determined, that is, the molecular composition of the mixed product is determined by determining, the physical product property of each single molecule in the mixed product is calculated separately, then the physical property of the mixed gasoline product is calculated according to the physical property and content of each single molecule in the mixed gasoline product. The physical property of single molecule includes, but not limited to, a density, a boiling point, a density, and an octane number. For example, the physical property of single molecule may also include viscosity, solubility parameters, cetane number, degree of unsaturation, etc.

In this embodiment, the preset standard in a preset standard set may be gasoline product standards such as vehicle gasoline standards, lubricating oil standards, hydraulic oil standards, gear oil standards, and cutting oil standards. If the mixed product meets any item in the preset standard set, it means that the mixed product can be sold; furthermore, since different mixed products are blended at the same time, the mixed products obtained by blending at the same time should meet any standard in the preset standard set, so that the preset rule set used for blending is a qualified set, avoiding the situation where mixed products cannot generate value.

The method for establishing the preset standard set may include the following steps: obtaining the standards of vehicle oil products of different brands; and using the standards of each brand of vehicle oil products as preset standards to form the preset standard set. By obtaining the standards of vehicle oil products of different brands and forming the preset standard set, the blended gasoline products are all vehicle oil products.

S16, if the product property of each of the mixed products meets any preset standard in the preset standard set, a target parameter is acquired according to all mixed products and it is determined whether the target parameter meets a preset condition.

In this embodiment, if the product property of each of mixed products meets any preset standard in the preset standard set, it means that each of the mixed products blended at this time is an eligible product. The relevant target parameters are acquired according to the mixed products, and it is determined whether the target parameters meet the preset conditions. The target parameters may be the economic benefits of the product, the content of substances in the product that will cause harm to the environment, and the proportion of products, which meet a preset standard, in all mixed products to all of the mixed products. In this step, the ultimate goal of the refinery's refining is to pursue benefits. A gross profit value may be calculated according to the price of each mixed product and the amount of the mixed product, and the gross profit value may be used to confirm whether the final benefit has reached the maximum, so as to confirm whether the target parameters meet the preset conditions, among which, confirming whether the final benefit has reached the maximum may be calculated by random algorithm. Meanwhile, with the gradual strengthening of people's environmental protection awareness, the content of substances that will cause harm to the environment in the mixed product will also affect the sales of the mixed product, even if the calculated benefit value is large, which cannot be sold at the sales end and cannot be converted into benefits. Therefore, in order to increase the competitiveness of oil products, it is possible to limit the content of substances that are harmful to the environment in mixed products. Moreover, when different mixed products are sold, the market will have different demand. For example, the price of No. 98 motor gasoline is higher than the price of No. 95 motor gasoline, but the consumption of No. 95 motor gasoline is larger, and if the refinery produces a large amount of No. 98 motor gasoline, the market will take longer to digest it, resulting in a backlog of No. 98 motor gasoline inventory, resulting in more labor and other costs, resulting in the final benefits are not as good as that of No. 95 motor gasoline. Therefore, in this step, the proportion of the production.

volume of mixed products that meet a certain preset standard in all mixed products may be calculated to avoid product backlog.

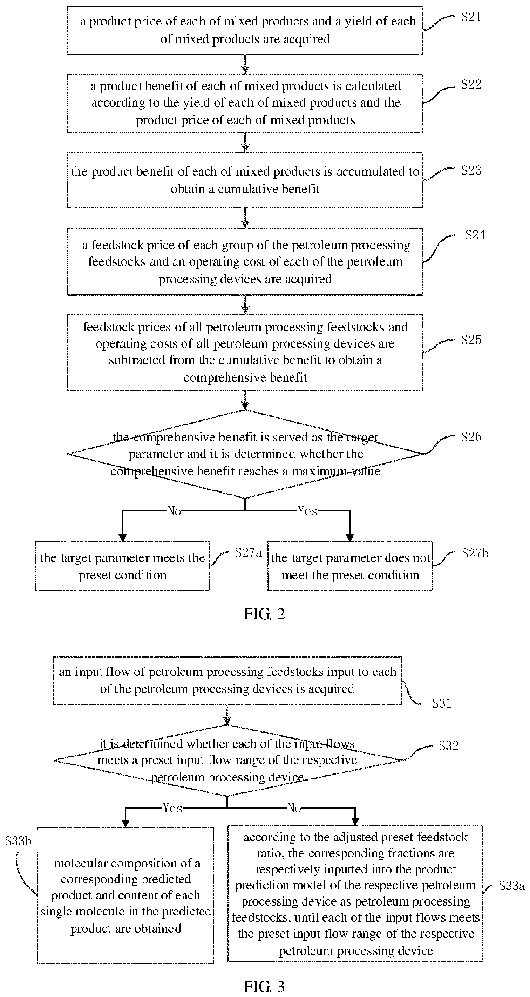

For example, the determining whether the target parameter meets a preset condition includes the following steps (see FIG. 2):

S21, a product price of each of mixed products and a yield of each of mixed products are acquired.

S22, a product benefit of each of mixed products is calculated according to the yield of each of mixed products and the product price of each of mixed products.

S23, the product benefit of each of mixed products is accumulated to obtain a cumulative benefit.

S24, a feedstock price of each group of the petroleum processing feedstocks and an operating cost of each of the petroleum processing devices are acquired.

S25, feedstock prices of all petroleum processing feedstocks and operating costs of all petroleum processing devices are subtracted from the cumulative benefit to obtain a comprehensive benefit.

In this embodiment, the corresponding comprehensive benefit may be obtained by subtracting the operating cost of each petroleum processing device and the feedstock cost of each group of petroleum processing feedstocks from the cumulative benefit of the mixed product, wherein the operating cost of the petroleum processing device includes: device loss cost and labor cost.

S26, the comprehensive benefit is served as the target parameter and it is determined whether the comprehensive benefit reaches a maximum value.

S27a, it is determined that the target parameter meets the preset condition if the comprehensive benefit reaches the maximum value.

S27b, it is determined that the target parameter does not meet the preset condition if the comprehensive benefit does not reach the maximum value.

In this embodiment, the comprehensive benefit is taken as the target parameter to ensure the production benefit, which may be determined whether the comprehensive benefit reaches the maximum value through a global optimization algorithm of random search with multiple starting points.

S17a, if the target parameter meets a preset condition, the preset feed ratio, the product prediction model, and the preset rule set is outputted as a production and processing scheme.

In this embodiment, when the target parameter also meets the corresponding preset condition, it means that the overall production process has met the production requirements at this time, and sustainable production may be carried out. At this time, the preset feedstock ratios for different fractions input into different petroleum processing devices in the output scheme, the product prediction model used to calculate the molecular composition of the predicted product produced by each petroleum processing device and the content of each single molecule, and the preset rule set for blending predicted products output from petroleum processing devices are taken as a production and processing scheme. In the actual production process, the production and processing scheme is used for production, and the whole process optimization for oil refining is realized at the molecular level.

S17b, if the target parameter does not meet the preset condition, the preset feedstock ratio, a parameter in the product prediction model and a preset rule in the preset rule set are adjusted, to re-obtain a plurality of mixed products until the product property of each of the mixed products meets any preset standard in the preset standard set and the target parameter meets the preset condition.

In this embodiment, when the target parameter does not meet the preset condition, it means that the economic benefits of the final blended mixed product may not reach the maximum value, or that the amount of substances with environmental impact in the mixed product exceeds the set value, or that the proportion of mixed products that meet a preset standard in all mixed products does not reach the set value. At this time, by adjusting the preset feedstock ratio, the operation parameter in the product prediction model and the preset rule in the preset rule set, a plurality of mixed products in another situation may be obtained, until the product properties of each of mixed products output in this scheme meets any preset standard in the preset standard set and the target parameter meets the preset condition, that is, the whole process optimization for refineries is completed.

In this embodiment, the optimization method further includes the following steps:

if the product property of any the mixed product does not meet any preset standard in the preset standard set, the preset rule in the preset rule set is adjusted and each of the product blending feedstocks is blended according to the adjusted preset rule set, to re-obtain a plurality of mixed products until the product property of each of the mixed products meets any preset standard in the preset standard set.

In this embodiment, if the product property of any mixed product does not meet any preset standard in the preset standard set, for example, the octane number of the adjusted No. 95 gasoline does not meet the standard of No. 95 gasoline, then the blending is a failure blending, and the products obtained by blending cannot enter the market. At this time, the preset rule in the preset rule set is adjusted and the product blending feedstocks are re-blended until the mixed product meets any preset standard in the preset standard set.

As shown in FIG. 3, in a specific embodiment, the optimization method further includes the following steps: S31, an input flow of petroleum processing feedstocks input to each of the petroleum processing devices is acquired.

In this embodiment, according to the preset feedstock ratio and the petroleum processing feedstocks respectively input to the petroleum processing device, the amount respectively inputted to each petroleum processing device per unit time may be obtained, that is, the input flow for the petroleum processing device may be obtained.

S32, it is determined whether each of the input flows meets a preset input flow range of the respective petroleum processing device.

In this embodiment, each group of petroleum processing devices has a corresponding processing capacity, to avoid the situation where the processing time of the feedstocks in the petroleum processing device is too short and the feedstocks do not react completely due to the input of the feedstocks exceeding the processing capacity of the petroleum processing unit, and the worse situation may cause damage to the petroleum processing device. In this embodiment, a preset input flow range is set, and the maximum value of the range can be between 80% and 95% of the maximum processing capacity of the petroleum processing device, and thus by limiting the amount of feedstocks entering the petroleum processing device, damage to the petroleum processing device is avoided.

S33a, the preset feedstock ratio is adjusted if any one of the input flows does not meet the preset input flow range of the respective petroleum processing device, and, according to the adjusted preset feedstock ratio, the corresponding fractions are respectively inputted into the product prediction model of the respective petroleum processing device as petroleum processing feedstocks, until each of the input flows meets the preset input flow range of the respective petroleum processing device.

In this embodiment, when the feedstock input flow of any of the petroleum processing devices is greater than the preset input flow range, the preset feedstock ratio is adjusted and the amount of petroleum processing feedstocks input to the petroleum processing device is re-planed, such that the input flow of the feedstocks of each petroleum processing device meets the preset input flow rate range of the respective petroleum processing device.

S33b, if each of the input flow meets the preset input flow rate range of the respective petroleum processing device, the step of obtaining molecular composition of a corresponding predicted product and content of each single molecule in the predicted product is performed.

In this embodiment, when the input flow of the feedstocks meets the preset input flow rate range of the respective petroleum processing device, the subsequent steps of the scheme are directly performed.

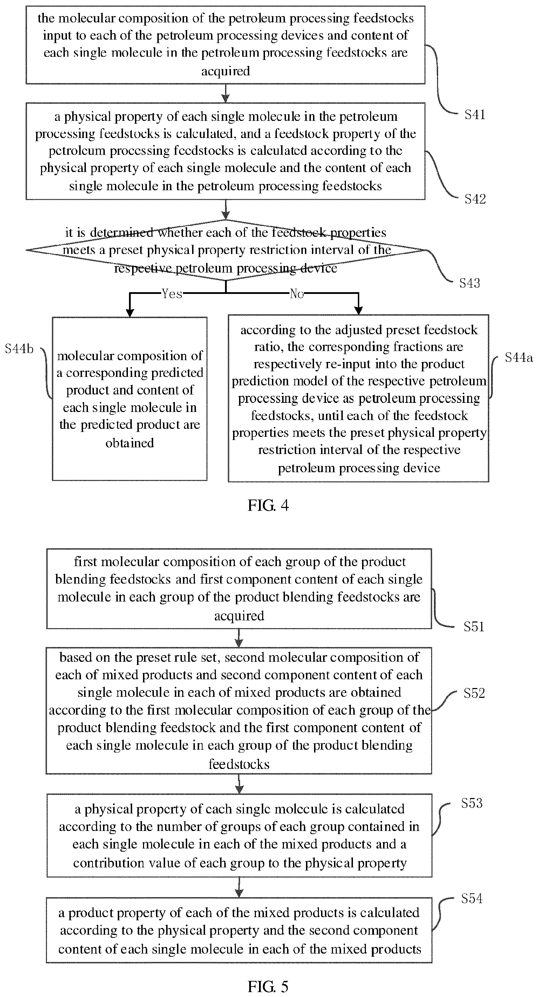

As shown in FIG. 4, in a specific embodiment, the optimization method further includes the following steps: S41, the molecular composition of the petroleum processing feedstocks inputted to each of the petroleum processing devices and content of each single molecule in the petroleum processing feedstocks are acquired.

In this embodiment, according to the preset feedstock ratio, and the molecular composition and content of each fraction product, the molecular composition of the petroleum processing feedstock respectively input to each petroleum processing device and the content of each single molecule in the petroleum processing feedstock are obtained.

S42, a physical property of each single molecule in the petroleum processing feedstocks is calculated, and a feedstock property of the petroleum processing feedstocks is calculated according to the physical property of each single molecule and the content of each single molecule in the petroleum processing feedstocks.

In this embodiment, the physical properties of each single molecule in the petroleum processing feedstocks input to each group of petroleum processing devices are calculated respectively, and the feedstock properties of the petroleum processing feedstocks are calculated according to the physical properties of each single molecule and the content of each single molecule in the petroleum processing feedstocks.

The physical properties of a single molecule may be calculated by the methods of calculating the physical properties of a single molecule in other embodiments, and the feedstock properties of petroleum processing feedstocks may be calculated by calculating the physical properties of a mixture in other embodiments.

S43, it is determined whether each of the feedstock properties meets a preset physical property restriction interval of the respective petroleum processing device.

S44a, if any of the feedstock properties does not meet the preset physical property restriction interval of the respective petroleum processing device, the preset feedstock ratio is adjusted and, according to the adjusted preset feedstock ratio, the corresponding fractions are respectively re-input into the product prediction model of the respective petroleum processing device as petroleum processing feedstocks, until each of the feedstock properties meets the preset physical property restriction interval of the respective petroleum processing device.

In this embodiment, different petroleum processing devices have different requirements on the physical properties of the incoming feedstocks to ensure the service life of the petroleum processing device. In this solution, the physical properties of the petroleum processing feedstocks input to the petroleum processing device are confirmed, and it is determined whether the physical properties of the feedstocks meet the preset physical property restriction interval of the respective petroleum processing device, so as to ensure the normal use of the petroleum processing device. If any petroleum processing feedstock does not meet the respective petroleum processing device, the preset feedstock ratio is adjusted again, and the feedstock properties of the petroleum processing feedstocks input to the petroleum processing device are adjusted, until the feedstock properties of the petroleum processing feedstocks meet the usage restrictions of the respective petroleum processing device. Since the petroleum restrictions of different petroleum processing devices are different, each petroleum processing device corresponds to a preset physical property restriction interval.

S44b, if each of the petroleum processing feedstock properties meets the preset physical property restriction interval of the respective petroleum processing device, the step of obtaining molecular composition of a corresponding predicted product and content of each single molecule in the predicted product is performed.

In this embodiment, the subsequent steps of the scheme are performed if the feedstock properties of the petroleum processing feedstocks meet the preset physical property restriction condition.

A further description of calculating the product properties of each of mixed products, as shown in FIG. 5, FIG. 5 is a flowchart of steps for calculating the physical properties of a mixed product according to an embodiment of the present disclosure.

S51, first molecular composition of each group of the product blending feedstocks and first component content of each single molecule in each group of the product blending feedstocks are acquired.

Since the product blending feedstocks are the predicted product of each group of petroleum processing devices, the first molecular composition of the product blending feedstocks and the first component content of each single molecule may be obtained based on the predicted product.

S52, based on the preset rule set, second molecular composition of each of mixed products and second component content of each single molecule in each of mixed products are obtained according to the first molecular composition of each group of the product blending feedstock and the first component content of each single molecule in each group of the product blending feedstocks.

In this embodiment, the preset rules in the preset rule set set the type and quantity of the required product blending feedstocks, therefore, according to the molecular composition and the first component content of each single molecule in the product blending feedstocks, the second molecular composition of the mixed product and the second component content of each single molecule may be obtained.

S53, a physical property of each single molecule is calculated according to the number of groups of each group contained in each single molecule in each of the mixed products and a contribution value of each group to the physical property.

In this embodiment, for each single molecule, the number of groups of each group constituting the single molecule and a contribution value of the each group to the physical property are acquired, and the number of groups of each group constituting the single molecule and the contribution value of each group to the physical property are inputted into a pre-trained property calculation model, to acquire the physical property of the single molecule outputted by the pre-trained property calculation model.

S54, a product property of each of the mixed products is calculated according to the physical property and the second component content of each single molecule in each of the mixed products.

The properties of the mixed gasoline product include: Research Octane Number, Motor Octane Number, Reid vapor pressure, Enn's distillation range, density, benzene volume fraction, aromatics volume fraction, olefin volume fraction, oxygen mass fraction, and sulfur quality fraction.

Five manners to calculate the physical properties of a mixed product are provided below, but those skilled in the art should be appreciated that the following several manners are only used to illustrate the present embodiments and are not intended to limit the present embodiments.

Method one, when a product property of the mixed product is the density, the density of the mixed product is calculated according to the following calculation formula:

$$density = \sum (D_i \times x_{i-volume}),$$

where, *density* is the density of the mixed product, $D_i$ is the density of the *i*-th single molecule, and $x_{i\_volume}$ is second component content of the *i*-th single molecule.

Method two, when a product property of the mixed product is the cloud point, calculating the product property of the mixed product includes:

calculating a cloud point contribution value of each single molecule according to the density and the boiling point of each single molecule; and
calculating the cloud point of the mixed product according to cloud point contribution values and content of all of the single molecules in the mixed product.

Method three, when a product property of the mixed product is the pour point, calculating the product property of the mixed product includes:

calculating a pour point contribution value of each of the single molecule according to the density and molecular weight of each single molecule; and
calculating the pour point of the mixed product according to pour point contribution values and content of all of the single molecules in the mixed product.

Method four, when a product property of the mixed product is the aniline point, calculating the product property of the mixed product includes:

calculating an aniline point contribution value of the single molecule according to the density and the boiling point of the single molecule; and
calculating the aniline point of the mixed product according to the aniline point contribution values and content of all of the single molecules in the mixed product.

Method five, when a product property of the mixed product is the octane number, a calculation method includes:

acquiring the octane number and content of each single molecule in the mixed products; and
calculating the octane number of the mixed products according to calculation formula as follows:

$$\begin{aligned}
ON = (&\sum_{i=HISQFG} \upsilon_i \beta_i ON_i + C_H \sum_{i=H} \upsilon_i \beta_i ON_i + C_I \sum_{i=I} \upsilon_i \beta_i ON_i + C_S \sum_{i=S} \upsilon_i \beta_i ON_i \\
&+ C_Q \sum_{i=Q} \upsilon_i \beta_i ON_i + C_F \sum_{i=F} \upsilon_i \beta_i ON_i + C_G \sum_{i=G} \upsilon_i \beta_i ON_i) \div \\
(&\sum_{i=HISQFG} \upsilon_i \beta_i + C_H (\sum_{i=H} \upsilon_i \beta_i - \sum_{i=H} \upsilon_i) + C_I (\sum_{i=I} \upsilon_i \beta_i - \sum_{i=I} \upsilon_i) + C_S (\sum_{i=S} \upsilon_i \beta_i - \sum_{i=S} \upsilon_i) \\
&+ C_Q (\sum_{i=Q} \upsilon_i \beta_i - \sum_{i=Q} \upsilon_i) + C_F (\sum_{i=F} \upsilon_i \beta_i - \sum_{i=F} \upsilon_i) + C_G (\sum_{i=G} \upsilon_i \beta_i - \sum_{i=G} \upsilon_i)),
\end{aligned}$$

$$C_H = \frac{k_{HI}^{(a)} \upsilon_I + k_{HS}^{(a)} \upsilon_S + k_{HQ}^{(a)} \upsilon_Q + k_{HF}^{(a)} \upsilon_F + k_{HG}^{(a)} \upsilon_G}{1 + k_{HI}^{(b)} \upsilon_I + k_{HS}^{(b)} \upsilon_S + k_{HQ}^{(b)} \upsilon_Q + k_{HF}^{(b)} \upsilon_F + k_{HG}^{(b)} \upsilon_G};$$

$$C_{\mathrm{I}} = \frac{k_{\mathrm{HI}}^{(a)} \upsilon_{\mathrm{H}} + k_{\mathrm{IS}}^{(a)} \upsilon_{\mathrm{S}} + k_{\mathrm{IQ}}^{(a)} \upsilon_{\mathrm{Q}} + k_{\mathrm{IF}}^{(a)} \upsilon_{\mathrm{F}} + k_{\mathrm{IG}}^{(a)} \upsilon_{\mathrm{G}}}{1 + k_{\mathrm{HI}}^{(b)} \upsilon_{\mathrm{H}} + k_{\mathrm{IS}}^{(b)} \upsilon_{\mathrm{S}} + k_{\mathrm{IQ}}^{(b)} \upsilon_{\mathrm{Q}} + k_{\mathrm{IF}}^{(b)} \upsilon_{\mathrm{F}} + k_{\mathrm{IG}}^{(b)} \upsilon_{\mathrm{G}}};$$

$$C_{\mathrm{S}} = \frac{k_{\mathrm{HS}}^{(a)} \upsilon_{\mathrm{H}} + k_{\mathrm{IS}}^{(a)} \upsilon_{\mathrm{I}} + k_{SQ}^{(a)} \upsilon_{\mathrm{Q}} + k_{SF}^{(a)} \upsilon_{\mathrm{F}} + k_{\mathrm{SG}}^{(a)} \upsilon_{\mathrm{G}}}{1 + k_{\mathrm{HS}}^{(b)} \upsilon_{\mathrm{H}} + k_{\mathrm{IS}}^{(b)} \upsilon_{\mathrm{I}} + k_{\mathrm{SQ}}^{(b)} \upsilon_{\mathrm{Q}} + k_{\mathrm{SF}}^{(b)} \upsilon_{\mathrm{F}} + k_{\mathrm{SG}}^{(b)} \upsilon_{\mathrm{G}}};$$

$$C_{\mathrm{Q}} = \frac{k_{HQ}^{(a)} \upsilon_{\mathrm{H}} + k_{\mathrm{IQ}}^{(a)} \upsilon_{\mathrm{I}} + k_{\mathrm{SQ}}^{(a)} \upsilon_{S} + k_{QF}^{(a)} \upsilon_{\mathrm{F}} + k_{QG}^{(a)} \upsilon_{\mathrm{G}}}{1 + k_{HQ}^{(b)} \upsilon_{\mathrm{H}} + k_{\mathrm{QI}}^{(b)} \upsilon_{\mathrm{I}} + k_{\mathrm{SQ}}^{(b)} \upsilon_{\mathrm{S}} + k_{\mathrm{QF}}^{(b)} \upsilon_{\mathrm{F}} + k_{\mathrm{QG}}^{(b)} \upsilon_{\mathrm{G}}};$$

$$C_{\mathrm{F}} = \frac{k_{\mathrm{HF}}^{(a)} \upsilon_{\mathrm{H}} + k_{\mathrm{IF}}^{(a)} \upsilon_{\mathrm{I}} + k_{\mathrm{SF}}^{(a)} \upsilon_{\mathrm{S}} + k_{\mathrm{QF}}^{(a)} \upsilon_{Q} + k_{\mathrm{FG}}^{(a)} \upsilon_{\mathrm{G}}}{1 + k_{\mathrm{HF}}^{(b)} \upsilon_{\mathrm{H}} + k_{\mathrm{IF}}^{(b)} \upsilon_{\mathrm{I}} + k_{\mathrm{SF}}^{(b)} \upsilon_{\mathrm{S}} + k_{\mathrm{QF}}^{(b)} \upsilon_{\mathrm{Q}} + k_{\mathrm{FG}}^{(b)} \upsilon_{\mathrm{G}}};$$

$$C_{\mathrm{G}} = \frac{k_{HG}^{(a)} \upsilon_{\mathrm{H}} + k_{\mathrm{IG}}^{(a)} \upsilon_{\mathrm{I}} + k_{\mathrm{SG}}^{(a)} \upsilon_{\mathrm{S}} + k_{\mathrm{QG}}^{(a)} \upsilon_{\mathrm{Q}} + k_{\mathrm{FG}}^{(a)} \upsilon_{\mathrm{F}}}{1 + k_{HG}^{(b)} \upsilon_{\mathrm{H}} + k_{\mathrm{IG}}^{(b)} \upsilon_{\mathrm{I}} + k_{\mathrm{SG}}^{(b)} \upsilon_{\mathrm{S}} + k_{\mathrm{QG}}^{(b)} \upsilon_{\mathrm{Q}} + k_{FG}^{(b)} \upsilon_{\mathrm{F}}};$$

where, the ON is the octane number of the mixed product, HISQFG is a molecular collection, H is a molecular set of n-alkanes, I is a molecular set of isoalkanes, S is a molecular set of cycloalkanes, Q is a molecular set of olefins, F is a molecular set of aromatic hydrocarbons, G is a molecular set of oxygenated compounds, $\upsilon_i$ is content of each molecule in the mixed product, $\upsilon_{\mathrm{H}}, \upsilon_{\mathrm{I}}, \upsilon_{\mathrm{S}}, \upsilon_{\mathrm{Q}}, \upsilon_{\mathrm{F}}$ and $\upsilon_{\mathrm{G}}$ are total content of n-alkanes, total content of isoalkanes, total content of cycloalkanes, total content of olefins, total content of aromatic hydrocarbons, and total content of a compound of oxygenated compounds in the mixed product, respectively, $\beta_i$ is a regression parameter of each molecule in the mixed product, $ON_i$ is an octane number of each molecule in the mixed product, $C_{\mathrm{H}}$ is an interaction coefficient of n-alkanes with other molecules, $C_{\mathrm{I}}$ is an interaction coefficient of isoalkanes with other molecules; $C_{\mathrm{S}}$ is an interaction coefficient of cycloalkanes with other molecules; $C_{\mathrm{Q}}$ is an interaction coefficient of olefins with other molecules, $C_{\mathrm{F}}$ is an interaction coefficient of aromatic hydrocarbons with other molecules, $C_{\mathrm{G}}$ is an interaction coefficient of oxygenated compounds with other molecules, $k_{\mathrm{HI}}^{(a)}$ is a first constant coefficient between n-alkanes and isoalkanes, $k_{\mathrm{HS}}^{(a)}$ is a first constant coefficient between n-alkanes and cycloalkanes, $k_{\mathrm{HQ}}^{(a)}$ is a first constant coefficient between n-alkanes and olefins, $k_{\mathrm{HF}}^{(a)}$ is a first constant coefficient between n-alkanes and aromatic hydrocarbons, $k_{HG}^{(a)}$ is a first constant coefficient between n-alkanes and oxygenated compounds, $k_{\mathrm{IS}}^{(a)}$ is a first constant coefficient between isoalkanes and cycloalkanes, $k_{\mathrm{IQ}}^{(a)}$ is a first constant coefficient between isoalkanes and olefins, $k_{\mathrm{IF}}^{(a)}$ is a first constant coefficient between isoalkanes and aromatic hydrocarbons, $k_{\mathrm{IG}}^{(a)}$ is a first constant coefficient between isoalkanes and oxygenated compounds, $k_{\mathrm{SQ}}^{(a)}$ is a first constant coefficient between cycloalkanes and olefins, $k_{\mathrm{SF}}^{(a)}$ is a first constant coefficient between cycloalkanes and aromatic hydrocarbons, $k_{\mathrm{SG}}^{(a)}$ is a first constant coefficient between cycloalkanes and oxygenated compounds, $k_{\mathrm{QF}}^{(a)}$ is a first constant coefficient between olefins and aromatic hydrocarbons, $k_{\mathrm{QG}}^{(a)}$ is a second constant coefficient between olefins and oxygenated compounds, $k_{\mathrm{FG}}^{(a)}$ is a first constant coefficient between aromatic hydrocarbons and oxygenated compounds, $k_{\mathrm{HI}}^{(b)}$ is a second

constant coefficient between n-alkanes and isoalkanes, $k_{HS}^{(b)}$ is a second constant coefficient between n-alkanes and cycloalkanes, $k_{HQ}^{(b)}$ is a second constant coefficient between n-alkanes and olefins, $k_{HF}^{(b)}$ is a second constant coefficient between n-alkanes and aromatic hydrocarbons, $k_{HG}^{(b)}$ is a second constant coefficient between n-alkanes and oxygenated compounds, $k_{IS}^{(b)}$ is a second constant coefficient between isoalkanes and cycloalkanes, $k_{IQ}^{(b)}$ is a second constant coefficient between isoalkanes and olefins, $k_{IF}^{(b)}$ is a second constant coefficient between isoalkanes and aromatic hydrocarbons, $k_{IG}^{(b)}$ is a second constant coefficient between isoalkanes and oxygenated compounds, $k_{SQ}^{(b)}$ is a second constant coefficient between cycloalkanes and olefins, $k_{SF}^{(b)}$ is a second constant coefficient between cycloalkanes and aromatic hydrocarbons, $k_{SG}^{(b)}$ is a second constant coefficient between cycloalkanes and oxygenated compound, $k_{QF}^{(b)}$ is a second constant coefficient between olefins and aromatic hydrocarbons, $k_{QG}^{(b)}$ is a second constant coefficient between olefins and oxygenated compound, and $k_{FG}^{(b)}$ is a second constant coefficient between aromatic hydrocarbons and oxygenated compound; wherein the octane number includes: a research octane number and a motor octane number.

In this embodiment, calculating the physical property of each single molecule includes the following steps: for each single molecule, acquiring the number of groups of each group constituting the single molecule and a contribution value of each group to the physical property; and inputting the number of groups of each group constituting the single molecule and the contribution value of each group to the physical property into a pre-trained property calculation model, to acquire the physical property of the single molecule outputted by the pre-trained property calculation model.

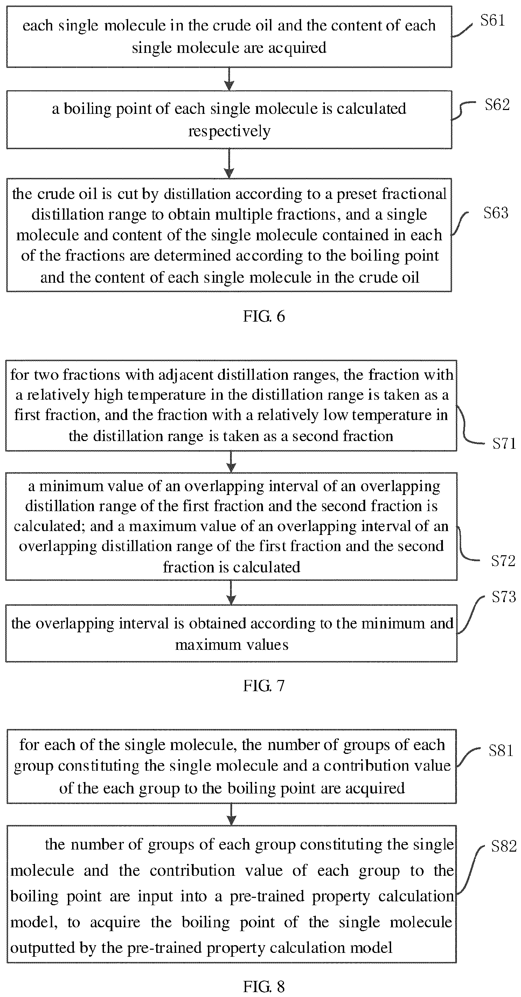

The acquiring molecular composition of various fractions obtained by distillation of the crude oil according to physical properties of the various single molecules in the molecular composition of the crude oil is further described below, as shown in FIG. 6, FIG. 6 is a flow chart of the steps for obtaining molecular compositions of different fractions according to an embodiment of the present disclosure.

S61, each single molecule in the crude oil and the content of each single molecule are acquired.

In this embodiment, there are many species of molecules in crude oil, different single molecules have different boiling points, and they need to be separated by distillation at different temperatures. Generally, a single molecule with a larger molecular weight in crude oil has a higher boiling point and is more difficult to separate. In the process of crude oil separation, the distillation range is divided according to the type of distilled oil and boiling points of molecules, each distillation range corresponding to an oil product, to complete the separation of crude oil. In this step, single molecules in crude oil and content corresponding to each single molecule are acquired.

In this embodiment, the molecular composition of the crude oil may be determined by one or more of a comprehensive two-dimensional gas chromatography method, a quaternary rod gas chromatography-mass spectrometer detection method, a gas chromatography/field ionization-time-of-flight mass spectrometry detection method, a gas chromatography method, a near-infrared spectroscopy method, a nuclear magnetic resonance spectroscopy method, a Raman spectroscopy method, a Fourier transform ion cyclotron resonance mass spectrometry method, an electrostatic field rail trap mass spectrometry method, and an ion mobility mass spectrometry method. In addition, the molecular composition of the crude oil may also be determined in other ways, such as ASTM D2425, SH/T 0606, and ASTM D8144-18.

The molecular detection method described above may detect the structure of the molecule and thereby obtaining the species of the molecule. However, due to the large number of molecular species in crude oil, although the crude oil can no longer be detected when the crude oil is reused after the crude oil is detected once, the workload of detecting each single molecule is large and time-consuming. Therefore, in this scheme, single molecules may also be constructed based on the structure-oriented lumped molecular characterization method. The structure-oriented lumped molecular characterization method namely is the SOL molecular characterization method, which uses 24 structural increment fragments to characterize the basic structure of complex hydrocarbon molecules. Any single petroleum molecule may be represented by a specific set of structural increment fragments. The SOL molecular characterization method is lumped at the molecular scale, reducing the number of molecules in the actual system from millions to thousands, greatly reducing the complexity of the simulation. This characterization method may represent not only alkanes, cycloalkanes, up to complex aromatic structures containing 50-60 carbon atoms, but also olefins or cycloolefins as intermediate products or secondary reaction products, and also consider sulfur, nitrogen, oxygen and other heteroatom compounds.

S62, a boiling point of each single molecule is calculated respectively.

In this embodiment, calculate the boiling point of each single molecule respectively by acquiring the number of groups of each group constituting the single molecule and a contribution value of each group to the physical property, and inputting the number of groups of each group constituting the single molecule and the contribution value of each group to the physical property into a pre-trained property calculation model, to acquire the physical property of the single molecule outputted by the pre-trained property calculation model; wherein, the groups that constitute a single molecule are the 24 structural increment fragments of the SOL-based molecular characterization method in the above embodiment.

S63, the crude oil is cut by distillation according to a preset fractional distillation range to obtain multiple fractions, and a single molecule and content of the single molecule contained in each of the fractions are determined according to the boiling point and the content of each single molecule in the crude oil.

In this embodiment, crude oil is cut according to the preset fractional distillation range to obtain respectively each fraction of crude oil distillation.

As shown in FIG. 7, in a specific embodiment, the optimization method for a whole process further includes the following steps:

S71, for two fractions with adjacent distillation ranges, the fraction with a relatively high temperature in the distillation range is taken as a first fraction, and the fraction with a relatively low temperature in the distillation range is taken as a second fraction.

In this embodiment, for any two fractions with adjacent distillation ranges, during the distillation process, at the distillation cut temperature of the fractions, not only the fractions with lower boiling points is distilled out, but another fractions with boiling points higher than the distillation cut temperature is distilled out to a certain amount. For example, the boiling point of water is 100 degrees Celsius, but at temperatures below 100 degrees Celsius, the water also evaporates. In this solution, it may be obtained by calculation: when crude oil is distilled, at what temperature, the molecules in the first fraction will appear in the second fraction; at what temperature, the molecules in the second fraction will not appear in the first fraction, thereby achieving molecular-level control of molecular species in fractions.

S72, a minimum value of an overlapping interval of an overlapping distillation range of the first fraction and the second fraction is calculated by the following formula:

$$T_{\min} = T_{\text{cut}} \times (1 - SF)$$ ,

a maximum value of the overlapping interval of the overlapping distillation range of the first fraction and the second fraction is calculated by the following formula:

$$T_{\max} = T_{\text{cut}} \times (1 + SF)$$ ,

where, $T_{\min}$ is the minimum value of the overlapping interval, $T_{\max}$ is the maximum value of the overlapping interval, $T_{\text{cut}}$ is the distillation cut temperature of the first fraction and the second fraction, and *SF* is a separation index of the first fraction and the second fraction.

S73, the overlapping interval is obtained according to the minimum and maximum values.

In this embodiment, the overlapping intervals of the adjacent two fractions are calculated. For example, the distillation range of the first fraction is 100-150 °C, the distillation range of the second fraction is 50 to 100 °C, °C being the temperature unit, the distillation partition temperature at this time is 100 °C. Although the distillation range of the first fraction is 100-150 °C, for example, when the distillation temperature is 70 °C, in the process of distilling to obtain the second fraction, part of the first fraction is distilled out and is doped in the second fraction; the first fraction has less the amount of distillation at lower temperatures, and as the temperature increases, more of the first fraction is distilled into the second fraction. In this scheme, recording is made by identifying when a preset amount of the second fraction is present in the first fraction. In this embodiment, the separation index of the first fraction and the second fraction may be calculated from the mixing of adjacent fractions recorded in the previous distillation process. Specifically, in the past distillation process, the temperature at which a preset amount of the first fraction appears in the second fraction and the temperature at which the preset amount of the second fraction no longer appears in the first fraction are recorded, based on the distillation cut temperature of the first fraction and the second fraction, a preliminary separation index is calculated, a large number of preliminary separation index calculation results are obtained, and the separation index at this distillation cut temperature is obtained by averaging them.

In this embodiment, the steps of entering the content of each single molecule into different fractions in the overlapping interval includes:

content of distilled part into the first fraction of each single molecule in the overlapping interval and content of distilled part into the second fraction of each single molecule in the overlapping interval are calculated according to the content of each single molecule and each single molecule corresponding to each boiling point of the overlapping interval; wherein the content of distilled part into the first fraction of each single molecule in the overlapping interval and the content of distilled part into the second fraction of each single molecule in the overlapping interval are calculated by the following equation:

$$C_h^i = \ln\ (\frac{T_i}{T_{\min}}) \times C^i \ , C_l^i = C^i - C_h^i;$$

where, $C_h^i$ is the content of distilled part into the first fraction of the i-th single molecule in all molecules with a boiling point located in the overlapping interval, which the i-th single molecule has the boiling point located in the overlapping interval, $C_l^i$ is the content of distilled part into the first fraction of the i-th single molecule in all molecules with a boiling point located in the overlapping interval, which the i-th single molecule has the boiling point located in the overlapping interval, $T_i$ is the boiling point of the i-th single molecule, $T_{\min}$ is the minimum value of the overlapping interval, and $C^i$ is the content of the i-th single molecule in all molecules with a boiling point located in the overlapping interval, which the i-th single molecule has the boiling point located in the overlapping interval; and
the content of each single molecule and each single molecule in each of the first fraction and the second fraction after the crude oil is cut by distillation are obtained according to he content of distilled part into the first fraction of each single molecule in the overlapping interval and the content of distilled part into the second fraction of each single molecule in the overlapping interval.

In this embodiment, after the overlapping interval is determined, the amount of each single molecule in the overlapping interval entering the adjacent two groups of fractions is calculated, and the content of various molecules in different fractions is determined by building a model, thereby improving the accuracy of the subsequent refining.

The calculation of boiling point of the single molecule is further described below.

As shown in FIG. 8, the steps of calculating the boiling point of each single molecule includes:
S81, for each of the single molecule, the number of groups of each group constituting the single molecule and a contribution value of the each group to the boiling point are acquired.

In this embodiment, single molecules may also be constructed based on the structure-oriented lumped molecular characterization method. The structure-oriented lumped molecular characterization method namely is the SOL molecular characterization method, which uses 24 structural increment fragments to characterize the basic structure of complex hydrocarbon molecules. Any single petroleum molecule may be represented by a specific set of structural increment fragments. The SOL method is lumped at the molecular scale, reducing the number of molecules in the actual system from millions to thousands, greatly reducing the complexity of the simulation. This characterization method may represent not only alkanes, cycloalkanes, up to complex aromatic structures containing 50-60 carbon atoms, but also olefins or cycloolefins as intermediate products or secondary reaction products, and also consider sulfur, nitrogen, oxygen and other heteroatom compounds. The molecular structure may be determined by one or more of a Raman spectroscopy, a quaternary rod gas chromatography-mass spectrometer detection method, a gas chromatography/field ionization-time-of-flight mass spectrometry detection method, a gas chromatography method, a near-infrared spectroscopy method, a nuclear magnetic resonance spectroscopy method, a Fourier transform ion cyclotron resonance mass spectrometry method, an electrostatic field rail trap mass spectrometry method and an ion mobility mass spectrometry method, and the single molecule was then constructed by structure-directed lumped molecular characterization method. In this step, the number of groups of each group constituting the single molecule and a contribution value of each group to the physical property are acquired; since the physical properties of the molecule are determined by the structure of the molecule, in this scheme, a single molecule is constructed by groups, and the number of groups of each group and the contribution value of each group to the physical properties are acquired.

In this embodiment, the groups included in each single molecule are determined based on the SOL molecular characterization method; in each of the single molecule, the number of groups of each group of the single molecule and a contribution value of each group to the physical property in the single molecule are determined. Since the number of physical properties of a single molecule is multiple, it is necessary to determine the contribution value of each group in the single molecule to each physical property.

S82, the number of groups of each group constituting the single molecule and the contribution value of each group to the boiling point are input into a pre-trained property calculation model, to acquire the boiling point of the single molecule outputted by the pre-trained property calculation model.

In this embodiment, a plurality of physical properties of the single molecule outputted by the pre-trained property calculation model by inputting the number of groups of each group and the contribution value of each group to the physical property into the pre-trained property calculation model.

The steps of the training property calculation model are described further below.

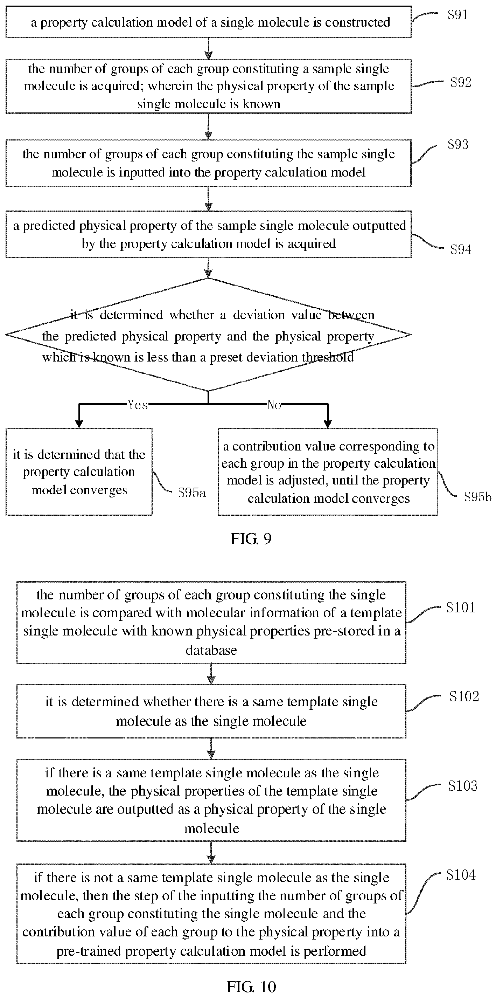

As shown in FIG. 9, the steps of training the property calculation model include:

S91, a property calculation model of a single molecule is constructed.

In this embodiment, in the property calculation model, the contribution value of each group to the physical property is included. The contribution value is an adjustable value, and the contribution value is the initial value during the first training. Further, in the property calculation model, a contribution value of each group to each physical property is included.

S92, the number of groups of each group constituting a sample single molecule is acquired; wherein the physical property of the sample single molecule is known.

In this embodiment, a training sample set is preset. A plurality of sample single molecule information is included in the training sample set. The sample single molecule information includes, but not limited to, the number of groups of each group constituting a sample single molecule, as well as the physical property of the sample single molecule.

S93, the number of groups of each group constituting the sample single molecule is inputted into the property calculation model.

S94, a predicted physical property of the sample single molecule outputted by the property calculation model is acquired.

S95a, if a deviation value between the predicted physical property and the physical property which is known is less than a preset deviation threshold, it is determined that the property calculation model converges, a contribution value corresponding to each group is acquired in the property calculation model which is converged, and the contribution value is stored as a contribution value of the group to the physical property.

Since the physical property of the single molecule may be various, the contribution value of each group to each physical property may be obtained from the converged property calculation model.

For each group, the contribution value of the group to each physical property is stored, so that when the physical property of the single molecule is subsequently calculated, the contribution value of each group in the single molecule to the physical property that needs to be known may be obtained, and the number of groups of each group in the single molecule and the contribution value of each group to the physical properties that need to be known are used as the input of the property calculation model. The property calculation model takes the number of groups of each group in the single molecule as the model variable, and the contribution value of each group to the physical property that needs to be known as the model parameter (replace the adjustable contribution value of each group in the property calculation model to the physical property), and calculate the physical properties that need to be known.

S95b, if the deviation value between the predicted physical property and the physical property which is known is greater than or equal to the deviation threshold, a contribution value corresponding to each group in the property calculation model is adjusted, until the property calculation model converges.

In this embodiment, if there are multiple physical properties of the sample single molecule, the predicted physical property of the sample single molecule outputted by the property calculation model will also be multiple. At this time, the deviation value between each predicted physical property and the corresponding physical property which is known is calculated, it is determined if the deviation values between all the predicted physical property and the corresponding physical property which is known are less than the preset deviation threshold, and if so, it is determined that the property calculation model is converged, and the contribution value of each group corresponding to the physical property is acquired according to the property calculation model which is converged. The contribution value of each group to different physical properties may be obtained through the above scheme.

Two property calculation models that may be used for different physical properties are given below. It should be appreciated by those skilled in the art that the following two property calculation models are only illustrative of the present embodiments and are not intended to limit the present embodiments.

Model one, a property calculation model as follows is established:

$$f = a + \sum n_i \Delta f_i$$

where, $f$ is the physical property of the single molecule, $n_i$ is the number of groups of the $i$-th group in the single molecule, $\Delta f_i$ is the contribution value of the $i$-th group in the single molecule to the physical property, and $a$ is an associated constant.

For example: for the boiling point, in the SOL-based molecular characterization method, 24 groups are present as a primary group; among the 24 groups, one or more of N6, N5, N4, N3, me, AA, NN, RN, NO, RO, and KO also contribute to the boiling point, while for different physical properties, the contribution value of the group to the physical property is inconsistent, but the contribution value of the same group in different molecules to the same physical property is consistent. Based on this scheme, the above-mentioned property calculation model is established in this embodiment, and by training the established property calculation model, it makes the property calculation model convergent, that is, the contribution value of each group in the model to the physical property is trained, and the contribution value of each group to the physical property is finally obtained.

In this embodiment, for a group that constitutes a single molecule, the group may be further divided into multi-stage groups. Further, a primary group and a multi-stage group are determined in all groups of a single molecule; wherein all groups constituting the single molecule are taken as the primary group, and various groups which coexist and contribute to the same physical property in common are taken as the multi-stage group, and the number of the various groups is taken as a level of the multi-stage group; it can be used as a multi-stage group according to the simultaneous existence of multiple groups that will act together on the same physical property. Specifically, for example, when N6 and N4 groups exist separately in different molecules, they will have a certain impact on the physical properties, and when they exist in one molecule at the same time, on the basis of the original contribution to the physical properties, they make the contribution value of the physical properties fluctuated to a certain extent. The method of dividing the above-mentioned multi-stage groups may also be divided according to the chemical bond force between the groups according to the preset bond force interval. For different physical properties, the various chemical bond forces have different effects, which can be divided according to the impact of molecular stability on physical properties.

Model two: based on the divided multi-stage group, a property calculation model may be established as follows:

$$f = a + \sum_i \mathrm{m}_{1i} \Delta f_{1i} + \sum_j m_{2j} \Delta f_{2j} \dotsc + \sum_l m_{Nl} \Delta f_{Nl} \; ;$$

where, $f$ is the physical property of the single molecule, $\mathrm{m}_{1i}$ is the number of groups of the $i$-th group in the primary group, $\Delta f_{1i}$ is the contribution value of the $i$-th group in the primary group to the physical property, $m_{2j}$ is the number of groups of the $j$-th group in a secondary group, $\Delta f_{2j}$ is the contribution value of the $j$-th group in the secondary group to the physical property, $m_{Nl}$ is the number of groups of the $l$-th group in an N-stage group, $\Delta f_{Nl}$ is the contribution value of the $l$-th group in the N-stage group to the physical property, $a$ is an associated constant, and N is a positive integer greater than or equal to 2.

In addition to the general property calculation model described above, a property calculation model may be established for each property, respectively, depending on the type of property.

For example, the boiling point of a single molecule is calculated according to a following property calculation model as follows:

$$T = \frac{SOL \times GROUP_1 + SOL \times GROUP_2 + \dotsc + SOL \times GROUP_N}{(SOL \times Numh)^d + b} + c \; ;$$

where, $T$ is the boiling point of the single molecule, $SOL$ is a single molecule vector converted according to the number of groups of each group constituting the single molecule, $GROUP_{11}$ is a first contribution value vector converted according to a contribution value of the primary group to the boiling point, $GROUP_{12}$ is a second contribution value vector converted according to a contribution value of the secondary group to the boiling point, $GROUP_{1N}$ is an N-th contribution value vector converted according to a contribution value of the N-stage group to the boiling point, $Numh$ is the number of atoms other than the hydrogen atom in the single molecule, $d$ is a first preset constant, $b$ is a second preset constant, $c$ is a third preset constant, and $N$ is a positive integer greater than or equal to 2.

Converting the single molecule vector according to the number of groups of each group constituting the single molecule, includes: taking the number of species of all groups constituting a single molecule as the dimension of the single molecule vector; and taking the number of groups of each group as an element value of the corresponding dimension in the single molecule vector.

Converting the first contribution value vector according to a contribution value of each primary group of the single molecule to the boiling point includes: taking the number of types of primary groups as the dimension of the first contribution value vector; and taking the contribution value of each primary group to the boiling point as an element value of the corresponding dimension in the first contribution value vector. Converting the second contribution value vector according to a contribution value of each secondary group of the single molecule to the boiling point includes: taking the number of types of secondary groups as the dimension of the second contribution value vector; and taking the contribution value of each secondary group to the boiling point as an element value of the corresponding dimension in the second contribution value vector. By analogy, converting the N-th contribution value vector according to a contribution value of each N-stage group of the single molecule to the boiling point includes: taking the number of types of N-stage groups as the dimension of the N-th contribution value vector; and taking the contribution value of each N-stage group to the boiling point as an element value of the corresponding dimension in the N-th contribution value vector.

As another example, the density of the single molecule is calculated according to a property calculation model as follows:

$$D = \frac{SOL \times GROUP_{21}}{(SOL \times GROUP_{22} + \ldots\ldots + SOL \times GROUP_{2N}) \times e};$$

where, $D$ is the density of the single molecule, $SOL$ is a single molecule vector converted according to the number of groups of each group constituting the single molecule, $GROUP_{21}$ is an N+1-th contribution value vector converted according to a contribution value of the primary group to the density, $GROUP_{22}$ is an N+2-th contribution value vector converted according to a contribution value of the secondary group to the density, $GROUP_{2N}$ is a 2N-th contribution value vector converted according to a contribution value of the N-stage group to the density, $e$ is the fourth preset constant; and $N$ is a positive integer greater than or equal to 2.

Converting the single molecule vector according to the number of groups of each group constituting the single molecule, includes: taking the number of species of all groups constituting a single molecule as a dimension of the single molecule vector; and taking the number of groups of each group as an element value of the corresponding dimension in the single molecule vector.

Converting the N+1-th contribution value vector according to a contribution value of each primary group of the single molecule to the density includes: taking the number of types of primary groups as a dimension of the N+1-th contribution value vector; and taking the contribution value of each primary group to the density as an element value of the corresponding dimension in the N+1-th contribution value vector. Converting the N+2-th contribution value vector converted according to a contribution value of each secondary group of the single molecule to the density includes: taking the number of types of secondary groups as a dimension of the N+2-th contribution value vector; and taking the contribution value of each secondary group to the density as an element value of the corresponding dimension in the N+2-th contribution value vector. By analogy, converting the 2N-th contribution value vector converted according to a contribution value of each N-stage group of the single molecule to the density includes: taking the number of types of N-stage groups as a dimension of the 2N contribution value vector; and taking the contribution value of each N-stage group to the density as an element value of the corresponding dimension in the 2N contribution value vector.

For example, the octane number of the single molecule is calculated according to a property calculation model as follows:

$$X = SOL \times GROUP_{31} + SOL \times GROUP_{32} + \ldots\ldots + SOL \times GROUP_{3N} + h;$$

where, $X$ is the octane number of the single molecule, $SOL$ is a single molecule vector converted according to the number of groups of each group constituting the single molecule, $GROUP_{31}$ is a 2N+1-th contribution value vector converted according to a contribution value of the primary group to the octane number, $GROUR_{32}$ is a 2N+2-th contribution value vector converted according to a contribution value of the secondary group to the octane number, $GROUP_{3N}$ is a 3N-th contribution value vector converted according to a contribution value of the N-stage group to the octane number; $N$ is a positive integer greater than or equal to 2; and $h$ is the fifth preset constant.

Converting the single molecule vector according to the number of groups of each group constituting the single molecule, includes: taking the number of species of all groups constituting a single molecule as a dimension of the single molecule vector; and taking the number of groups of each group as an element value of the corresponding dimension in the single molecule vector.

Converting the 2N+1-th contribution value vector according to a contribution value of each primary group of the single molecule to the octane number includes: taking the number of types of primary groups as a dimension of the 2N+1-th contribution value vector; and taking the contribution value of each primary group to the octane number as an element value of the corresponding dimension in the 2N+1-th contribution value vector. Converting the 2N+2-th contribution value vector converted according to a contribution value of each secondary group of the single molecule to the octane number includes: taking the number of types of secondary groups as a dimension of the 2N+2-th contribution value vector; and taking the contribution value of each secondary group to the octane number as an element value of the corresponding dimension in the 2N+2-th contribution value vector. By analogy, converting the 3N contribution value vector converted according to a contribution value of each N-stage group of the single molecule to the octane number includes: taking the number of types of N-stage groups as a dimension of the 3N contribution value vector; and taking the contribution value of each N-stage group to the octane number as an element value of the corresponding dimension in the 3N contribution value vector.

After the physical property of the corresponding single molecule is obtained by calculation in the above steps, the single molecule is taken as a template single molecule, and the number of groups and corresponding physical properties of each group constituting a single molecule are stored into the database.

As shown in FIG. 10, before the step S82, the method of calculation further includes:

S101, the number of groups of each group constituting the single molecule is compared with molecular information of a template single molecule with known physical properties pre-stored in a database, the molecular information including the number of groups of each group constituting the template single molecule.

S102, it is determined whether there is a same template single molecule as the single molecule.

S103, if there is a same template single molecule as the single molecule, the physical properties of the template single molecule are outputted as a physical property of the single molecule.

S104, if there is not a same template single molecule as the single molecule, then the step of the inputting the number of groups of each group constituting the single molecule and the contribution value of each group to the physical property into a pre-trained property calculation model is performed.

In this solution, after the number of groups of each group that constitutes a single molecule is obtained, by comparing the number of corresponding groups, it is confirmed whether the structure and physical properties of this type of single molecule have been stored in the database, and after the appearance of the template single molecule consistent with the single molecule is confirmed, the physical properties of the single molecule are directly outputted, thereby improving the calculation efficiency of single molecule physical properties and reducing the amount of calculation.

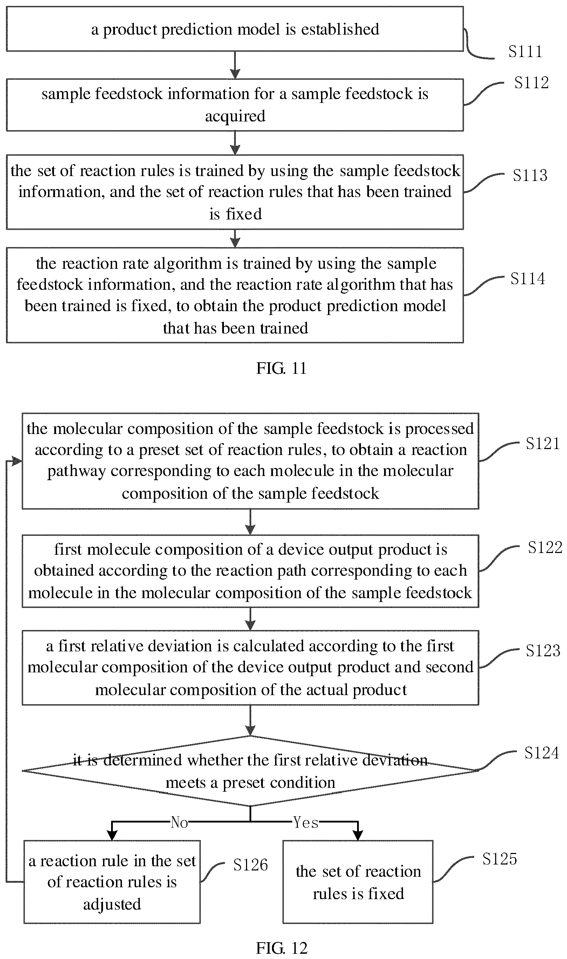

The steps of training the product prediction model are described further below. As shown in FIG. 11, FIG. 11 is a flow chart of steps of training a product prediction model according to an embodiment of the present disclosure.

S111, a product prediction model is established; wherein the product prediction model includes: a set of reaction rules including a plurality of reaction rules and a reaction rate algorithm.

The product prediction model is correspondingly established according to the type of petroleum processing devices.

The product prediction model corresponding to the petroleum processing device includes: a set of reaction rules and a reaction rate algorithm corresponding to the petroleum processing device. The set of reaction rules includes: a plurality of reaction rules corresponding to the petroleum processing devices.

S112, sample feedstock information for a sample feedstock is acquired.

The sample feedstock information of the sample feedstock includes: molecular composition of the sample feedstock, molecular content of each molecule in the sample feedstock, molecular composition of an actual product corresponding to the sample feedstock, and actual content of each molecule in the actual product. The actual product refers to the product obtained after the sample feedstock is processed by the petroleum processing device.

S113, the set of reaction rules is trained by using the sample feedstock information, and the set of reaction rules that has been trained is fixed.

A way to train a set of reaction rules is given below. It should be appreciated by those skilled in the art that the way is only illustrative of the present embodiments and is not intended to limit the present embodiments.

As shown in FIG. 12, FIG. 12 is a flowchart diagram of steps of training a set of reaction rules according to an embodiment of the present disclosure.

S121, the molecular composition of the sample feedstock is processed according to a preset set of reaction rules, to obtain a reaction pathway corresponding to each molecule in the molecular composition of the sample feedstock.

When the reaction pathway is firstly calculated, the molecular composition of the sample feedstock is processed in a set of preset reaction rules, to obtain a reaction pathway corresponding to each molecule in the molecular composition of the sample feedstock.

Each molecule in the sample feedstock is reacted according to reaction rules in a set of reaction rules, to obtain a reaction path corresponding to each molecule.

S122, first molecule composition of a device output product is obtained according to the reaction path corresponding to each molecule in the molecular composition of the sample feedstock.

**In** the device output product, the sample feedstock, the intermediate product, and the predicted product are included.

S123, a first relative deviation is calculated according to the first molecular composition of the device output product and second molecular composition of the actual product.

This step specifically includes: acquiring species of single molecules in the first molecule composition, to constitute a first set; acquiring species of single molecules in the second molecule composition, to constitute a second set; determining whether the second set is a subset of the first set; if the second set is not a subset of the first set, obtaining a pre-stored relative deviation value that does not meet the preset condition as the first relative deviation; and if the second set is a subset of the first set, calculating the first relative deviation by a calculating formula as follows:

$$x_1 = \frac{\text{card}\big((M - M_1 - M_2) - M_3\big)}{\text{card}\ (M - M_1 - M_2)} \ ;$$

where, $x_1$ is the first relative deviation, $M$ is the first set, $M_1$ is a set of species of single molecules in the molecular composition of the sample feedstock, $M_2$ is a set of species of single molecules in the molecular composition of the intermediate product, $M_3$ is the second set, and card is the number of elements in the sets.

S124, it is determined whether the first relative deviation meets a preset condition; if yes, S125 is executed; if no, S126 is executed.

Step S125, if the first relative deviation meets a preset condition, the set of reaction rules is fixed.

Step S126, if the first relative deviation does not meet the preset condition, a reaction rule in the set of reaction rules is adjusted and go to step S121, and the first relative deviation is recalculated according to the adjusted set of reaction rules until the first relative deviation meets the preset condition.

S114, the reaction rate algorithm is trained by using the sample feedstock information, and the reaction rate algorithm that has been trained is fixed, to obtain the product prediction model that has been trained.

A way to train the reaction rate algorithm is given below. Those skilled in the art should be appreciated that this way is only used to illustrate the present embodiments and is not intended to limit the present embodiments.

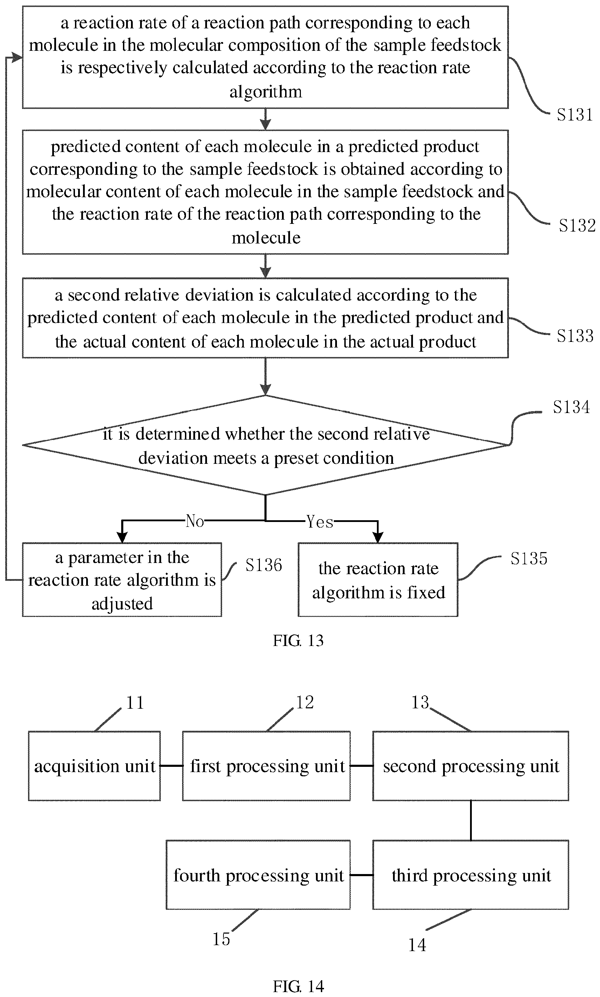

As shown in FIG. 13, FIG. 13 is a flowchart of steps of training a reaction rate algorithm according to an embodiment of the present disclosure.

S131, a reaction rate of a reaction path corresponding to each molecule in the molecular composition of the sample feedstock is respectively calculated according to the reaction rate algorithm.

Specifically, a reaction rate of each reaction path is calculated according to a reaction rate constant in the reaction rate algorithm.

The reaction rate constant is determined according to a calculation formula as follows:

$$k = \frac{k_B E}{h} \exp(\frac{E\Delta S - \Delta E}{RE})\varphi \times P^{\alpha} \ ;$$

where, $k$ is the reaction rate constant, $k_B$ is the Boltzmann constant, $h$ is the Planck constant, $R$ is an ideal gas constant, $E$ is a temperature value of the environment at which the reaction path is located, exp is an exponential function with base of natural constant, $\Delta S$ is an entropy change before and after the reaction corresponding to the reaction rule corresponding to the reaction path, $\Delta E$ is a reaction energy barrier corresponding to the reaction rule corresponding to the reaction path, $\varphi$ is a catalyst activity factor, $P$ is a pressure value of the environment at which the reaction path is located, and $\alpha$ is a pressure influencing factor corresponding to the reaction rule corresponding to the reaction path.

Specifically, the reaction rate of the reaction path is obtained according to the reaction rate constant and the reaction concentration corresponding to the reaction path. For example, under the condition that the reaction rate constant has been determined, the larger the space velocity, the shorter the contact time between the feedstocks and the catalyst, the shorter the reaction time of the feedstocks, the higher the concentration of the reactant in the feedstocks, and the higher the reaction rate of the reaction path; on the contrary, the smaller the space velocity, the longer the contact time between the feedstocks and the catalyst, the longer the reaction time of the feedstocks, the lower the concentration of reactants in the feedstocks, and the lower the reaction rate of this reaction path.

S132, predicted content of each molecule in a predicted product corresponding to the sample feedstock is obtained according to molecular content of each molecule in the sample feedstock and the reaction rate of the reaction path corresponding to the molecule.

In this embodiment, the reaction rate corresponding to each reaction path is calculated by the reaction rate calculation method in the product prediction model, in combination with the single molecule content of each single molecule in the feedstock, the predicted content of each single molecule in the predicted product. For example, for the single molecule A in the feedstock, it is assumed that the single molecule A corresponds to three reaction paths, and the reaction rates corresponding to the three reaction paths are known; as the reaction proceeds, the concentration of the single molecule A decreases, and the reaction rates corresponding to the three reaction paths will decrease in proportion to the decrease in concentration, and thus single molecule A will generate products in proportion to the reaction rates of the three paths. According to the above steps, the product obtained by the reaction of each molecule may be obtained, and the predicted product may be obtained. When the single molecule content of each single molecule in the catalytic reforming feedstock is known, the content of each single molecule in the predicted product may be obtained.

S133, a second relative deviation is calculated according to the predicted content of each molecule in the predicted product and the actual content of each molecule in the actual product.

In this embodiment, calculating the second relative deviation is, for example:
The second relative deviation = (actual content - predicted content) ÷ actual content.

S134, it is determined whether the second relative deviation meets a preset condition; if yes, S135 is executed; if no, S136 is executed.

Step S135, if the second relative deviation meets a preset condition, the reaction rate algorithm is fixed.

Step S136, if the second relative deviation does not meet the preset condition, a parameter in the reaction rate algorithm is adjusted and go the step S131, and the second relative deviation is recalculated according to the adjusted reaction rate algorithm until the second relative deviation meets the preset condition.

As shown in FIG. 14, the embodiments of the present disclosure provide an optimization apparatus for a whole process of molecular-level oil refinery processing, the optimization apparatus including: an acquisition unit 11, a first processing unit 12, a second processing unit 13, a third processing unit 14, and a fourth processing unit 15.

In this embodiment, the acquisition unit 11 is configured to acquire molecular composition of crude oil.

In this embodiment, the first processing unit 12 is configured to acquire molecular composition of various fractions obtained by distillation of the crude oil according to physical properties of various single molecules in the molecular composition of the crude oil, and respectively input, according to a preset feedstock ratio, the corresponding fractions into a product prediction model of a respective petroleum processing device as petroleum processing feedstocks, to obtain molecular composition of a corresponding predicted product and content of each single molecule in the predicted product.

In this embodiment, the second processing unit 13 is configured to blend each of the predicted products which is used as a product blending feedstock according to a preset rule set, to obtain molecular composition of a plurality of mixed products and content of each single molecule in each of the mixed products.

In this embodiment, the third processing unit 14 is configured to respectively calculate a product property of each of the mixed products according to the molecular composition of each of the mixed products and the content of each single molecule in each of the mixed products; and determine whether the product property of each of the mixed products meets any preset standard in a preset standard set.

In this embodiment, the fourth processing unit 15 is configured to, if the product property of each of the mixed products meets any preset standard in the preset standard set, acquire a target parameter according to all mixed products and determine whether the target parameter meets a preset condition, and, if the target parameter does not meet the preset condition, adjust the preset feedstock ratio, a parameter in the product prediction model and a preset rule in the preset rule set, to re-obtain a plurality of mixed products until the product property of each of the mixed products meets any preset standard in the preset standard set and the target parameter meets the preset condition.

In this embodiment, the optimization apparatus further includes:
a flow control unit configured to acquire an input flow of petroleum processing feedstocks input to each of the petroleum processing devices, determine whether each of the input flows meets a preset input flow range of the respective petroleum processing device; and adjust the preset feedstock ratio if any one of the input flows does not meet the preset input flow range of the respective petroleum processing device, and respectively re-input, according to the adjusted preset feedstock ratio, the corresponding fractions into the product prediction model of the respective petroleum processing device as petroleum processing feedstocks, until each of the input flows meets the preset input flow range of the respective petroleum processing device.

In this embodiment, the optimization apparatus further includes:
an in-feed property control unit configured to acquire the molecular composition of the petroleum processing feedstocks inputted to each of the petroleum processing devices and content of each single molecule in the petroleum processing feedstocks, calculate a physical property of each single molecule in the petroleum processing feedstocks, calculate a feedstock property of the petroleum processing feedstocks according to the physical property of each single molecule and the content of each single molecule in the petroleum processing feedstocks, determine whether each of the feedstock properties meets a preset physical property restriction interval of the respective petroleum processing device; and, if any of the feedstock properties does not meet the preset physical property restriction interval of the respective petroleum processing device, adjust the preset feedstock ratio and respectively re-input, according to the adjusted preset feedstock ratio, the corresponding fractions into the product prediction model of the respective petroleum processing device as petroleum processing feedstocks, until each of the feedstock properties meets the preset physical property restriction interval of the respective petroleum processing device.

In this embodiment, the fourth processing unit 15 is , in particular, configured to acquire a product price of each of mixed products and a yield of each of mixed products, calculate a product benefit of each of mixed products according to the yield of each of mixed products and the product price of each of mixed products, accumulate the product benefit of each of mixed products to obtain a cumulative benefit, acquire a feedstock price of each group of the petroleum processing feedstocks and an operating cost of each of the petroleum processing devices, subtract feedstock prices of all petroleum processing feedstocks and operating costs of all petroleum processing devices from the cumulative benefit to obtain a comprehensive benefit, serve the comprehensive benefit as the target parameter, determine whether the comprehensive benefit reaches a maximum value, determine that the target parameter meets the preset condition if the comprehensive benefit reaches the maximum value; and determine that the target parameter does not meet the preset condition if the comprehensive benefit does not reach the maximum value.

In this embodiment, the optimization apparatus further includes:

a product property control unit configured to, if the product property of any mixed product does not meet any preset standard in the preset standard set, adjust the preset rule in the preset rule set and blend each of the product blending feedstocks according to the adjusted preset rule set, to re-obtain a plurality of mixed products until the product property of each of the mixed products meets any preset standard in the preset standard set.

In this embodiment, the third processing unit 14 is, in particular, configured to acquire first molecular composition of each group of the product blending feedstocks and first component content of each single molecule in each group of the product blending feedstocks, based on the preset rule set, obtain second molecular composition of each of mixed products and second component content of each single molecule in each of mixed products according to the first molecular composition of each group of the product blending feedstock and the first component content of each single molecule in each group of the product blending feedstocks, calculate a physical property of each single molecule in each of the mixed products according to the number of groups of each group contained in each single molecule in each of the mixed products and a contribution value of each group to the physical property; and calculate a product property of each of the mixed products according to the physical property and the second component content of each single molecule in each of the mixed products.

In this embodiment, the third processing unit 14 is, in particular, configured to, for each single molecule, acquire the number of groups of each group constituting the single molecule and a contribution value of each group to the physical property; and input the number of groups of each group constituting the single molecule and the contribution value of each group to the physical property into a pre-trained property calculation model, to acquire the physical property of the single molecule outputted by the property calculation model.

In this embodiment, the optimization apparatus further includes: a single molecule property template matching unit.

The single molecule property template matching unit is configured to compare the number of groups of each group constituting the single molecule with molecular information of a template single molecule with known physical properties pre-stored in a database, the molecular information including the number of groups of each group constituting the template single molecule, determine whether there is a same template single molecule as the single molecule, if there is a same template single molecule as the single molecule, output the physical properties of the template single molecule as a physical property of the single molecule; and if there is not a same template single molecule as the single molecule, then perform, by the third processing unit 14, the step of the inputting the number of groups of each group constituting the single molecule and the contribution value of each group to the physical property into a pre-trained property calculation model.

In this embodiment, the first processing unit 12 is, in particular, configured to acquire each single molecule in the crude oil and the content of each single molecule, calculate a boiling point of each single molecule, respectively, cut the crude oil by distillation according to a preset fractional distillation range to obtain multiple fractions, and determine a single molecule and content of the single molecule contained in each of the fractions according to the boiling point and the content of each single molecule in the crude oil.

In this embodiment, the first processing unit 12 is further configured to, for two fractions with adjacent distillation ranges, take the fraction with a relatively high temperature in the distillation range as a first fraction, and take the fraction with a relatively low temperature in the distillation range as a second fraction;

calculate a minimum value of an overlapping interval of an overlapping distillation range of the first fraction and the second fraction by the following formula:

$$T_{\min} = T_{\text{cut}} \times (1 - SF)$$;

calculate a maximum value of the overlapping interval of the overlapping distillation range of the first fraction and the second fraction by the following formula:

$$T_{\max} = T_{\text{cut}} \times (1 + SF)$$;

where, $T_{\min}$ is the minimum value of the overlapping interval, $T_{\max}$ is the maximum value of the overlapping interval, $T_{\text{cut}}$ is the distillation cut temperature of the first fraction and the second fraction, and $SF$ is a separation index of the first fraction and the second fraction.

In this embodiment, the first processing unit 12 is further configured to calculate content of distilled part into the first fraction of each single molecule in the overlapping int erval and calculate content of distilled part into the second fraction of each single molecule in the overlapping interval according to the content of each single molecule and each single molecule corresponding to each boiling point of the overlapping interval, and obtain the content of each single molecule and each single molecule in each of the first fraction and the second fraction after the crude oil is cut by distillation according to the content of distilled part into the first fraction of each single molecule in the overlapping interval and the content of distilled part into the second fraction of each single molecule in the overlapping interval;

wherein the content of distilled part into the first fraction of each single molecule in the overlapping interval and the content of distilled part into the second fraction of each single molecule in the overlapping interval are calculated by the following equation:

$$C_h^i = \ln \left( \frac{T_i}{T_{\min}} \right) \times C^i \; , \; C_l^i = C^i - C_h^i \; ;$$

where, $C_h^i$ is the content of distilled part into the first fraction of the i-th single molecule in all molecules with a boiling point located in the overlapping interval, which the i-th single molecule has the boiling point located in the overlapping interval, $C_l^i$ is the content of distilled part into the first fraction of the i-th single molecule in all molecules with a boiling point located in the overlapping interval, which the i-th single molecule has the boiling point located in the overlapping interval, $T_i$ is the boiling point of the i-th single molecule, $T_{\min}$ is the minimum value of the overlapping interval, and $C^i$ is the content of the i-th single molecule in all molecules with a boiling point located in the overlapping interval, which the i-th single molecule the boiling point located in the overlapping interval.

In this embodiment, the first processing unit 12 is, in particular, configured to, for each of the single molecule, acquire the number of groups of each group constituting the single molecule and a contribution value of each group to the physical property; and input the number of groups of each group constituting the single molecule and the contribution value of each group to the boiling point into a pre-trained property calculation model, to acquire the boiling point of the single molecule outputted by pre-trained the property calculation model.

In this embodiment, the optimization apparatus further includes: a single-molecule boiling point template matching unit.

The single-molecule boiling point template matching unit is configured to compare the number of groups of each group constituting the single molecule with molecular information of a template single molecule with known boiling point pre-stored in a database, the molecular information including the number of groups of each group constituting the template single molecule, determine whether there is a same template single molecule as the single molecule, if there is a same template single molecule as the single molecule, output the boiling point of the template single molecule as a boiling point of the single molecule; and if there is not a same template single molecule as the single molecule, then perform, by the first processing unit 12, the step of the inputting the number of groups of each group constituting the single molecule and the contribution value of each group to the boiling point into a pre-trained property calculation model.

In this embodiment, the optimization apparatus further includes: a model training unit.

The model training unit is configured to construct a property calculation model of a single molecule, acquire the number of groups of each group constituting a sample single molecule; wherein the physical property of the sample single molecule is known, input the number of groups of each group constituting the sample single molecule into the property calculation model, acquire a predicted physical property of the sample single molecule outputted by the property calculation model, if a deviation value between the predicted physical property and the physical property which is known is less than a preset deviation threshold, determine that the property calculation model converges, acquiring a contribution value for each group to the physical property in the property calculation model which is converged, and storing a contribution value for the group to the physical property; and, if the deviation value between the predicted physical property and the physical property which is known is greater than or equal to the deviation threshold, adjust a contribution value corresponding to each group in the property calculation model until the property calculation model converges.

In this embodiment, the model training unit is configured to establish the property calculation model as shown below:

$$f = a + \sum_i n_i \Delta f_i$$

where, $f$ is the physical property of the single molecule, $n_i$ is the number of groups of the $i$-th group, $\Delta f_i$ is the contribution value of the $i$-th group to the physical property, and $\alpha$ is an associated constant.

In the present embodiment, the model training unit is, in particular, configured to determine a primary group, the number of groups of the primary group, a multi-stage group, and the number of groups of the multi-stage group in all groups of the single molecule, take all groups constituting the single molecule as the primary group, take various groups which coexist and contribute to a same physical property in common as the multi-stage group, and take the number of the various groups as a level of the multi-stage group.

In this embodiment, the model training unit is configured to establish the property calculation model as shown below:

$$f = a + \sum_i \mathrm{m}_{1i} \Delta f_{1i} + \sum_j m_{2j} \Delta f_{2j} \ldots\ldots + \sum_l m_{Nl} \Delta f_{Nl}$$

where, $f$ is the physical property of the single molecule, $\mathrm{m}_{1i}$ is the number of groups of the $i$-th group in the primary group, $\Delta f_{1i}$ is the contribution value of the $i$-th group in the primary group to the physical property, $m_{2j}$ is the number of groups of the $j$-th group in a secondary group, $\Delta f_{2j}$ is the contribution value of the $j$-th group in the secondary group to the physical property, $m_{Nl}$ is the number of groups of the $l$-th group in an N-stage group, $\Delta f_{Nl}$ is the contribution value of the $l$-th group in the N-stage group to the physical property, $\alpha$ is an associated constant, and N is a positive integer greater than or equal to 2.

In this embodiment, the first processing unit 12 is, in particular, configured to determine a primary group, the number of groups of the primary group, a multi-stage group, and the number of groups of the multi-stage group in all groups of the single molecule, take all groups constituting the single molecule as the primary group, take various groups which coexist and contribute to a same physical property in common as the multi-stage group, and take the number of the various groups as a level of the multi-stage group.

In this embodiment, the first processing unit 12 is, in particular, configured to calculate the boiling point of the single molecule according to the following the property calculation model:

$$T = \frac{SOL \times GROUP_1 + SOL \times GROUP_2 + \ldots\ldots + SOL \times GROUP_N}{(SOL \times \mathrm{Numh})^d + b} + c$$

where, $T$ is the boiling point of the single molecule, $SOL$ is a single molecule vector converted according to the number of groups of each group constituting the single molecule, $GROUP_{11}$ is a first contribution value vector converted according to a contribution value of the primary group to the boiling point, $GROUP_{12}$ is a second contribution value vector converted according to a contribution value of the secondary group to the boiling point, $GROUP_{1N}$ is an N-th contribution value vector converted according to a contribution value of the N-stage group to the boiling point, $Numh$ is the number of atoms other than the hydrogen atom in the single molecule, $d$ is a first preset constant, $b$ is a second preset constant, $c$ is a third preset constant, and $N$ is a positive integer greater than or equal to 2.

In this embodiment, the first processing unit 12 is, in particular, configured to take the number of species of groups as a dimension of the single molecule vector, and take the number of groups of each group as an element value of the corresponding dimension in the single molecule vector.

The first processing unit 12 is, in particular, configured to take the number of types of primary groups as a dimension of the first contribution value vector, and take the contribution value of each primary group to the boiling point as an element value of the corresponding dimension in the first contribution value vector;

The first processing unit 12 is, in particular, configured to take the number of types of secondary groups as a dimension of the second contribution value vector, and take the contribution value of each secondary group to the boiling point as an element value of the corresponding dimension in the second contribution value vector.

The first processing unit 12 is, in particular, configured to take the number of types of N-stage groups as a dimension of the N-th contribution value vector and take the contribution value of each N-stage group to the boiling point as an element value of the corresponding dimension in the N-th contribution value vector.

In this embodiment, the first processing unit 12 is, in particular, configured to obtain different amounts of each fraction according to the preset feedstock ratio, and respectively input each fraction into the product prediction model of the respective petroleum processing device, and the petroleum processing device includes a catalytic cracking unit, a delayed coking unit, a residue hydrotreating unit, a hydrocracking unit, a diesel hydro-upgrading unit, a diesel hydro-refining unit, a gasoline hydro-refining unit, a catalytic reforming unit and an alkylation unit.

In this embodiment, the optimization apparatus further includes:
a model training unit configured to establish a product prediction model, acquire sample feedstock information for a sample feedstock, train the set of reaction rules by using the sample feedstock information, fix the set of reaction rules that has been trained; and train the reaction rate algorithm by using the sample feedstock information, and fix the reaction rate algorithm that has been trained, to obtain the product prediction model that has been trained; wherein the product prediction model includes: a set of reaction rules including a plurality of reaction rules and a reaction rate algorithm.

In this embodiment, the sample feedstock information of the sample feedstock includes: molecular composition of the sample feedstock, molecular content of each molecule in the sample feedstock, molecular composition of an actual product corresponding to the sample feedstock, and actual content of each molecule in the actual product.

In this embodiment, the model training unit is, in particular, configured to process the molecular composition of the sample feedstock according to a preset set of reaction rules, to obtain a reaction pathway corresponding to each molecule in the molecular composition of the sample feedstock, obtain first molecule composition of a device output product including the sample feedstock, an intermediate product, and a predicted product according to the reaction path corresponding to each molecule in the molecular composition of the sample feedstock, in the device output product, including: the sample feedstock, the intermediate product, and the predicted product, calculate a first relative deviation according to the first molecular composition of the device output product and second molecular composition of the actual product, if the first relative deviation meets a preset condition, fix the set of reaction rules, and, if the first relative deviation does not meet the preset condition, adjust a reaction rule in the set of reaction rules, and recalculate the first relative deviation according to the adjusted set of reaction rules until the first relative deviation meets the preset condition.

In this embodiment, the model training unit is, in particular, configured to acquire species of single molecules in the first molecule composition, to constitute a first set, acquire species of single molecules in the second molecule composition, to constitute a second set, determine whether the second set is a subset of the first set, if the second set is not a subset of the first set, obtain a pre-stored relative deviation value that does not meet the preset condition as the first relative deviation, and, if the second set is a subset of the first set, calculate the first relative deviation by a calculating

$$x_1 = \frac{\mathrm{card}\big((M - M_1 - M_2) - M_3\big)}{\mathrm{card}\ (M - M_1 - M_2)}$$

formula as follows: ;

where, $x_1$ is the first relative deviation, $M$ is the first set, $M_1$ is a set of species of single molecules in the molecular composition of the sample feedstock, $M_2$ is a set of species of single molecules in the molecular composition of the intermediate product, $M_3$ is the second set, and card represents the number of elements in the sets.

In this embodiment, the model training unit is, in particular, configured to calculate a reaction rate of a reaction path corresponding to each molecule in the molecular composition of the sample feedstock, respectively, according to the reaction rate algorithm, obtain predicted content of each molecule in the predicted product corresponding to the sample feedstock according to molecular content of each molecule in the sample feedstock and the reaction rate of the reaction path corresponding to the molecule, calculate a second relative deviation according to the predicted content of each molecule in the predicted product and the actual content of each molecule in the actual product, if the second relative deviation meets a preset condition, fix the reaction rate algorithm, and, if the second relative deviation does not meet the preset condition, adjust a parameter in the reaction rate algorithm, and recalculate the second relative deviation according to the adjusted reaction rate algorithm until the second relative deviation meets the preset condition.

In this embodiment, the model training unit is, in particular, configured to calculate a reaction rate of each reaction path according to a reaction rate constant in the reaction rate algorithm;

wherein the reaction rate constant is determined according to a calculation formula as follows:

$$k = \frac{k_B E}{h} \exp(\frac{E\Delta S - \Delta E}{RE})\varphi \times P^\alpha$$

;

where, $k$ is the reaction rate constant, $k_B$ is the Boltzmann constant, $h$ is the Planck constant, $R$ is an ideal gas constant, $E$ is a temperature value of the environment at which the reaction path is located, exp is an exponential function with base of natural constant, $\Delta S$ is an entropy change before and after the reaction corresponding to the reaction rule corresponding to the reaction path, $\Delta E$ is a reaction energy barrier corresponding to the reaction rule corresponding to the reaction path, $\varphi$ is a catalyst activity factor, $P$ is a pressure value of the environment at which the reaction path is located, and $\alpha$ is a pressure influencing factor corresponding to the reaction rule corresponding to the reaction path.

In this embodiment, each petroleum processing device corresponds to a set of reaction rules.

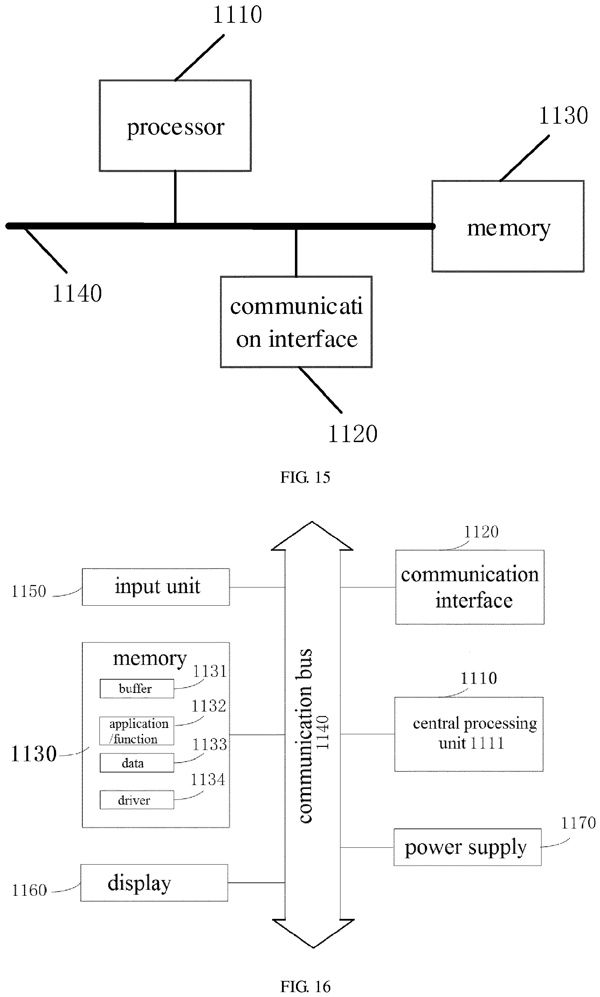

As shown in FIG. 15, the embodiments of the present disclosure provide an optimization system for a whole process of molecular-level oil refinery processing including a processor 1110, a communication interface 1120, a memory 1130, and a communications bus 1140, wherein the processor 1110, the communications interface 1120, and the memory 1130 are in communication with each other via the communications bus 1140;

the memory 1130 is configured to store a computer program;

the processor 1110 is configured to carry out the optimization method for a whole process of molecular-level oil refinery processing when executing the program stored on the memory 1130: acquiring molecular composition of crude oil; acquiring molecular composition of various fractions obtained by distillation of the crude oil according to physical properties of various single molecules in the molecular composition of the crude oil; respectively inputting, according to a preset feedstock ratio, the corresponding fractions into a product prediction model of a respective petroleum processing device as petroleum processing feedstocks, to obtain molecular composition of a corresponding predicted product and content of each single molecule in the predicted product; blending each of the predicted products which is used as a product blending feedstock according to a preset rule set, to obtain molecular composition of a plurality of mixed products and content of each single molecule in each of the mixed products; respectively calculating a product property of each of the mixed products according to the molecular composition of each of the mixed products and the content of each single molecule in each of the mixed products; and determining whether the product property of each of the mixed products meets any preset standard in a preset standard set; if the product property of each of the mixed products meets any preset standard in the preset standard set, acquiring a target parameter according to all mixed products and determining whether the target parameter meets a preset condition; and if the target parameter does not meet the preset condition, adjusting the preset feedstock ratio, a parameter in the product prediction model and a preset rule in the preset rule set, to re-obtain a plurality of mixed products until the product property of each of the mixed products meets any preset standard in the preset standard set and the target parameter meets the preset condition.

For the system provided by the embodiments of the present invention, the processor 1110 implements optimization by executing the program stored in the memory 1130.

The communication bus 1140 mentioned in the above electronic device may be a Peripheral Component Interconnect (PCI for short) bus or an Extended Industry Standard Architecture (EISA for short) bus or the like. The communication bus 1140 may be divided into an address bus, a data bus, a control bus, and the like. For ease of representation, only one thick line is shown in FIG. 15, but it does not mean that there is only one bus or one type of bus.

The communication interface 1120 is configured for communication between the above electronic device and other devices.

The memory 1130 may include Random Access Memory (RAM for short), or may include Non-volatile Memory, such as at least one disk storage. Alternatively, the memory may also be at least one storage device located away from the aforementioned processor.

The above-mentioned processor 1110 may be a general-purpose processor, including a Central Processing Unit (CPU for short), a Network Processor (NP for short), etc.; it may also be a Digital Signal Processing (DSP for short), Application Specific Integrated Circuit (ASIC for short), Field-Programmable Gate Array (FPGA for short) or other programmable logic devices, discrete gate or transistor logic devices, and discrete hardware components.

In a specific embodiment, a schematic block diagram of the optimization system for a whole process of molecular-level oil refinery processing is shown in FIG. 16, which further includes an input unit 1150, a display 1160, and a power supply 1170. The processor 1110 uses the central processing unit 1111 (when the central processing unit 1111 is used to execute the program stored in the memory 1130, it implements the steps of the gasoline blending method, which refers to the above content "the processor 1110, when executing the program stored on the memory 1130, performs the optimization method for a whole process of molecular-level oil refinery processing" and is not described herein redundantly).

The memory 1130 includes buffer memory 1131 (sometimes referred to as a buffer). The memory 140 may include an application/function store 1132 for storing application programs and functional programs or processes for performing the operation of the optimization system for a whole process of molecular-level oil refinery processing executed by the central processing unit 1111.

The memory 1130 may also include a data store 1133 for storing data, such as a product prediction model, a preset rule set, a preset criteria set, a preset input flow range, digital data, pictures, and/or any other data used by the optimization system for a whole process of molecular-level oil refinery processing; the driver store 1134 of the memory 1130 may include various drivers of the gasoline blending device.

The central processing unit 1111, also sometimes referred to as a controller or operating control, may include a microprocessor or other processor device and/or logic device. The central processing unit 1111 receives input and controls the operation of the various components of the optimization system for a whole process of molecular-level oil refinery processing.

The input unit 1150 provides input to the central processing unit 1111; the input unit 1150 is, for example, a key or a touch input device; the power supply 1170 is used to provide power to the optimization system for a whole process of molecular-level oil refinery processing; the display 1160 is used for display of display objects, such as images and text; the display, for example, may be an LCD display, but is not limited thereto.

The present disclosure provides a computer-readable storage medium, the computer-readable storage medium has stored therein one or more programs, one or more programs executable by one or more processors to implement an optimization method of any of the embodiments described above.

In any one of embodiments described above, it may be implemented in whole or in part by software, hardware, firmware, or any combination thereof. When implemented in software, the functions may be implemented in whole or in part in the form of a computer program product. The computer program product includes one or more computer instructions. When the computer program instructions are loaded and executed on a computer, the processes or functions of the embodiments in accordance with the present disclosure are generated in whole or in part. The computer may be a general purpose computer, a special purpose computer, a computer network, or other programmable apparatus. The computer instructions may be stored on or transmitted from one computer-readable storage medium to another computer-readable storage medium, e.g., the computer instructions may be transmitted from one website, computer, server, or data center to another website, computer, server, or data center via a wired (e.g., coaxial cable, fiber optic, digital subscriber line (DSL)), or wireless (e.g., infrared, radio, microwave). Computer readable storage media can be any available media that can be accessed by a computer or a data storage device that includes one or more servers, data centers, and the like, which can be integrated with one or more available media. The usable medium may be a magnetic medium, (e.g., a floppy disk, a hard disk, a magnetic tape), an optical medium (e.g., a DVD), or a semiconductor medium (e.g., a solid state drive (SSD)), among others.

**Claims**

1.  A computer-implemented method for obtaining a production and processing scheme of oil refinery processing, wherein the method comprises:

    acquiring molecular composition of crude oil;
    acquiring molecular composition of various fractions obtained by distillation of the crude oil according to physical properties of various single molecules in the molecular composition of the crude oil;
    respectively inputting, according to a preset feedstock ratio, the molecular composition of the corresponding fractions into a product prediction model of a respective petroleum processing device as petroleum processing feedstocks, to obtain molecular composition of a corresponding predicted product and content of each single molecule in the predicted product;
    blending each of the predicted products which is used as a product blending feedstock according to a preset rule set, to obtain molecular composition of a plurality of mixed products and content of each single molecule in each of the mixed products, wherein each set of preset rules in the preset rule set includes the type and amount of the predicted product used;
    respectively calculating a product property of each of the mixed products according to the molecular composition of each of the mixed products and the content of each single molecule in each of the mixed products; and determining whether the product property of each of the mixed products meets any preset standard in a preset standard set;
    if the product property of each of the mixed products meets any preset standard in the preset standard set, acquiring a target parameter according to all mixed products and determining whether the target parameter meets a preset condition; the target parameter represents: a content of substances in the product that will cause harm to the environment; or a proportion of products, which meet a preset standard, in all mixed products to all of the mixed products; and
    if the target parameter does not meet the preset condition, adjusting the preset feedstock ratio, an operating parameter in the product prediction model and a preset rule in the preset rule set, to re-obtain a plurality of mixed products until the product property of each of the mixed products meets any preset standard in the preset standard set and the target parameter meets the preset condition, the operating parameters in the product prediction model comprising pressure, temperature and space velocity;
    if the target parameter meets a preset condition, the preset feedstock ratio, the product prediction model, and the preset rule set are outputted as a production and processing scheme, and using the production and processing scheme to conduct oil refinery processing in an actual production process;
    wherein the acquiring molecular composition of various fractions obtained by distillation of the crude oil according to physical properties of various single molecules in the molecular composition of the crude oil comprises:

acquiring each single molecule in the crude oil and the content of each single molecule;

calculating a boiling point of each single molecule, respectively; and

cutting the crude oil by distillation according to a preset fractional distillation range to obtain multiple fractions, and determining a single molecule and content of each single molecule contained in each of the fractions according to the boiling point and the content of each single molecule in the crude oil;

the method further comprises:

for two fractions with adjacent distillation ranges, taking the fraction with a relatively high temperature in the distillation range as a first fraction, and taking the fraction with a relatively low temperature in the distillation range as a second fraction;

calculating a minimum value of an overlapping interval of an overlapping distillation range of the first fraction and the second fraction by the following formula:

$$T_{\min} = T_{\mathrm{cut}} \times (1 - SF);$$

and

calculating a maximum value of the overlapping interval of the overlapping distillation range of the first fraction and the second fraction by the following formula:

$$T_{\max} = T_{\mathrm{cut}} \times (1 + SF);$$

where, $T_{\min}$ is the minimum value of the overlapping interval, $T_{\max}$ is the maximum value of the overlapping interval, $T_{\mathrm{cut}}$ is the distillation cut temperature of the first fraction and the second fraction, and $SF$ is a separation index of the first fraction and the second fraction;

the method further comprises:

calculating content of distilled part into the first fraction of each single molecule in th e overlapping interval and calculating content of distilled part into the second fraction of ea ch single molecule in the overlapping interval according to the content of each single molecule and each single molecule corresponding to each boiling point of the overlapping interval;

wherein the content of distilled part into the first fraction of each single molecule in the overlapping interval and the content of distilled part into the second fraction of each single molecule in the overlapping interval are calculated by the following equation:

$$C_h^i = \ln\ (\frac{T_i}{T_{\min}}) \times C^i;$$

$$C_l^i = C^i - C_h^i;$$

where, $C_h^i$ is the content of distilled part into the first fraction of the i-th single molecule in all molecules with a boiling point located in the overlapping interval, which the i-th single molecule has the boiling point located in the overlapping interval, $C_l^i$ is the content of distilled part into the first fraction of the i-th single molecule in all molecules with a boiling point located in the overlapping interval, which the i-th single molecule has the boiling point located in the overlapping interval, $T_i$ is the boiling point of the i-th single molecule, $T_{\min}$ is the minimum value of the overlapping interval, and $C^i$ is the content of the i-th single molecule in all molecules with a boiling point located in the overlapping interval, which the i-th single molecule has the boiling point located in the overlapping interval; and

obtaining the content of each single molecule and each single molecule in each of the first fraction and the second fraction after the crude oil is cut by distillation according to the content of distilled part into the first fraction of each single molecule in the overlapping interval and the content of distilled part into the second fraction of each single molecule in the overlapping interval.

2. The method according to claim 1, wherein the method further comprises:

acquiring an input flow of petroleum processing feedstocks input to each of the petroleum processing devices; determining whether each of the input flows meets a preset input flow range of the respective petroleum processing device; and
adjusting the preset feedstock ratio if any one of the input flows does not meet the preset input flow range of the respective petroleum processing device, and respectively re-inputting, according to the adjusted preset feedstock ratio, the corresponding fractions into the product prediction model of the respective petroleum processing device as petroleum processing feedstocks, until each of the input flows meets the preset input flow range of the respective petroleum processing device.

3. The method according to claim 1, wherein the method further comprises:

acquiring molecular composition of the petroleum processing feedstocks inputted to each of the petroleum processing devices and content of each single molecule in the petroleum processing feedstocks;
calculating a physical property of each single molecule in the petroleum processing feedstocks, calculating a feedstock property of the petroleum processing feedstocks according to the physical property of each single molecule and the content of each single molecule in the petroleum processing feedstocks;
determining whether each of the feedstock properties meets a preset physical property restriction interval of the respective petroleum processing device; and
if any of the feedstock properties does not meet the preset physical property restriction interval of the respective petroleum processing device, adjusting the preset feedstock ratio, and respectively re-inputting, according to the adjusted preset feedstock ratio, the corresponding fractions into the product prediction model of the respective petroleum processing device as petroleum processing feedstocks, until each of the feedstock properties meets the preset physical property restriction interval of the respective petroleum processing device.

4. The method according to claim 1, wherein the method further comprises:
if the product property of any mixed product does not meet any preset standard in the preset standard set, adjusting the preset rule in the preset rule set and blending each of the product blending feedstocks according to the adjusted preset rule set, to re-obtain a plurality of mixed products until the product property of each of the mixed products meets any preset standard in the preset standard set.

5. The method according to claim 1, wherein the respectively calculating a product property of each of the mixed products according to the molecular composition of each of the mixed products and the content of each single molecule in each of the mixed products comprises:

acquiring first molecular composition of each set of the product blending feedstocks and first component content of each single molecule in each set of the product blending feedstocks;
based on the preset rule set, obtaining second molecular composition of each of mixed products and second component content of each single molecule in each of mixed products according to the first molecular composition of each set of the product blending feedstock and the first component content of each single molecule in each set of the product blending feedstocks;
calculating a physical property of each single molecule in each of the mixed products according to the number of groups of each group contained in each single molecule in each of the mixed products and a contribution value of each group to the physical property; and
calculating a product property of each of the mixed products according to the physical property and the second component content of each single molecule in each of the mixed products.

6. The method according to claim 5, wherein calculation of the physical property of each single molecule comprises:

for each single molecule, acquiring the number of groups of each group constituting the single molecule and a contribution value of each group to the physical property; and
inputting the number of groups of each group constituting the single molecule and the contribution value of each group to the physical property into a pre-trained property calculation model, to acquire the physical property of the single molecule outputted by the pre-trained property calculation model.

**7.** The method according to claim 6, wherein, before the inputting the number of groups of each group constituting the single molecule and the contribution value of each group to the physical property into a pre-trained property calculation model, the method further comprises:

comparing the number of groups of each group constituting the single molecule with molecular information of a template single molecule with known physical properties pre-stored in a database, the molecular information comprising the number of groups of each group constituting the template single molecule;
determining whether there is a same template single molecule as the single molecule;
if there is a same template single molecule as the single molecule, outputting the physical properties of the template single molecule as a physical property of the single molecule; and
if there is not a same template single molecule as the single molecule, then performing the step of the inputting the number of groups of each group constituting the single molecule and the contribution value of each group to the physical property into a pre-trained property calculation model.

**8.** The method according to claim 1, wherein the calculating a boiling point of each single molecule comprises:

for each of the single molecule, acquiring the number of groups of each group constituting the single molecule and a contribution value of each group to the boiling point; and
inputting the number of groups of each group constituting the single molecule and the contribution value of each group to the boiling point into a pre-trained property calculation model, to acquire the boiling point of the single molecule outputted by the pre-trained property calculation model.

**9.** The method according to claim 8, wherein, before the inputting the number of groups of each group constituting the single molecule and the contribution value of each group to the boiling point into a pre-trained property calculation model, the method further comprises:

comparing the number of groups of each group constituting the single molecule with molecular information of a template single molecule with known boiling point pre-stored in a database, the molecular information comprising the number of groups of each group constituting the template single molecule;
determining whether there is a same template single molecule as the single molecule;
if there is a same template single molecule as the single molecule, outputting the boiling point of the template single molecule as a boiling point of the single molecule; and
if there is not a same template single molecule as the single molecule, then performing the step of the inputting the number of groups of each group constituting the single molecule and the contribution value of each group to the boiling point into a pre-trained property calculation model.

**10.** The method according to claim 6 or 8, wherein a step of training the property calculation model comprises:

constructing a property calculation model of a single molecule;
acquiring the number of groups of each group constituting a sample single molecule; wherein the physical property of the sample single molecule is known;
inputting the number of groups of each group constituting the sample single molecule into the property calculation model;
acquiring a predicted physical property of the sample single molecule outputted by the property calculation model;
if a deviation value between the predicted physical property and the physical property which is known is less than a preset deviation threshold, determining that the property calculation model converges, acquiring a contribution value corresponding to each group in the property calculation model which is converged, and storing the contribution value as a contribution value of the group to the physical property; and
if the deviation value between the predicted physical property and the physical property which is known is greater than or equal to the deviation threshold, adjusting a contribution value corresponding to each group in the property calculation model until the property calculation model converges.

**11.** The method according to claim 10, wherein the property calculation model is established as shown below:

$$f = a + \sum_i n_i \Delta f_i$$

where, $f$ is the physical property of the single molecule, $n_i$ is the number of groups of the $I$-th group, $\Delta f_i$ is the contribution value of the i-th group to the physical property, and $a$ is an associated constant.

12. The method according to claim 10, wherein the acquiring the number of groups of each group constituting a sample single molecule comprises:

> determining a primary group, the number of groups of the primary group, a multi-stage group, and the number of groups of the multi-stage group in all groups of the single molecule;
> taking all groups constituting the single molecule as the primary group; and
> taking various groups which coexist and contribute to a same physical property in common as the multi-stage group, and taking the number of the various groups as a level of the multi-stage group.

13. The method according to claim 12, wherein,

the property calculation model is established as shown below:

$$f = a + \sum_i m_{1i}\Delta f_{1i} + \sum_j m_{2j}\Delta f_{2j} \ldots\ldots + \sum_l m_{Nl}\Delta f_{Nl}$$ ;

where, $f$ is the physical property of the single molecule, $m_{1i}$ is the number of groups of the $I$-th group in the primary group, $\Delta f_{1i}$ is the contribution value of the $i$-th group in the primary group to the physical property, $m_{2j}$ is the number of groups of the $j$-th group in a secondary group, $\Delta f_{2j}$ is the contribution value of the $j$-th group in the secondary group to the physical property, $m_{Nl}$ is the number of groups of the $I$-th group in an N-stage group, $\Delta f_{Nl}$ is the contribution value of the $I$-th group in the N-stage group to the physical property, $\alpha$ is an associated constant, and $N$ is a positive integer greater than or equal to 2.

14. The method according to claim 8, wherein the acquiring the number of groups of each group constituting the single molecule comprises:

> determining a primary group, the number of groups of the primary group, a multi-stage group, and the number of groups of the multi-stage group in all groups of the single molecule;
> taking all groups constituting the single molecule as the primary group; and
> taking various groups which coexist and contribute to a same physical property in common as the multi-stage group, and taking the number of the various groups as a level of the multi-stage group.

15. The method according to claim 14, wherein,
the inputting the number of groups of each group constituting the single molecule and the contribution value of each group to the boiling point into a pre-trained property calculation model, to acquire the boiling point of the single molecule outputted by the pre-trained property calculation model comprises:

calculating the boiling point of the single molecule according to the following the property calculation model:

$$T = \frac{SOL \times GROUP_1 + SOL \times GROUP_2 + \ldots\ldots + SOL \times GROUP_N}{(SOL \times Numh)^d + b} + c$$ ;

where, $T$ is the boiling point of the single molecule, $SOL$ is a single molecule vector converted according to the number of groups of each group constituting the single molecule, $GROUP_{11}$ is a first contribution value vector converted according to a contribution value of the primary group to the boiling point, $GROUPE_{12}$ is a second contribution value vector converted according to a contribution value of the secondary group to the boiling point, $GROUP_{1N}$ is an N-th contribution value vector converted according to a contribution value of the N-stage group to the boiling point, $Numh$ is the number of atoms other than the hydrogen atom in the single molecule, $d$ is a first preset constant, $b$ is a second preset constant, $c$ is a third preset constant, and $N$ is a positive integer greater than or equal to 2.

16. The method according to claim 15, wherein,
converting the single molecule vector according to the number of groups of each group constituting the single molecule comprises:

taking the number of species of groups as a dimension of the single molecule vector; and
taking the number of groups of each group as an element value of the corresponding dimension in the single molecule vector,
converting the first contribution value vector according to a contribution value of the primary group to the boiling point comprises:

taking the number of types of primary groups as a dimension of the first contribution value vector; and
taking the contribution value of each primary group to the boiling point as an element value of the corresponding dimension in the first contribution value vector,
converting the second contribution value vector according to a contribution value of the secondary group to the boiling point comprises:

taking the number of types of secondary groups as a dimension of the second contribution value vector; and
taking the contribution value of each secondary group to the boiling point as an element value of the corresponding dimension in the second contribution value vector,
converting the N-th contribution value vector according to a contribution value of each N-stage group to the boiling point comprises:

taking the number of types of N-stage groups as a dimension of the N-th contribution value vector; and
taking the contribution value of each N-stage group to the boiling point as an element value of the corresponding dimension in the N-th contribution value vector.

17. The method according to claim 1, wherein the respectively inputting, according to a preset feedstock ratio, the corresponding fractions into a product prediction model of a respective petroleum processing device comprises:

obtaining different amounts of each fraction according to the preset feedstock ratio, and respectively inputting each fraction into the product prediction model of the respective petroleum processing device,
the petroleum processing device comprises a catalytic cracking unit, a delayed coking unit, a residue hydrotreating unit, a hydrocracking unit, a diesel hydro-upgrading unit, a diesel hydro-refining unit, a gasoline hydro-refining unit, a catalytic reforming unit and an alkylation unit.

18. The method according to claim 17, wherein, a step of training the product prediction model comprises:

establishing a product prediction model; wherein the product prediction model comprises: a set of reaction rules comprising a plurality of reaction rules and a reaction rate algorithm;
acquiring sample feedstock information for a sample feedstock;
training the set of reaction rules by using the sample feedstock information, and fixing the set of reaction rules that has been trained; and
training the reaction rate algorithm by using the sample feedstock information, and fixing the reaction rate algorithm that has been trained, to obtain the product prediction model that has been trained.

19. The method according to claim 18, wherein the sample feedstock information of the sample feedstock comprises: molecular composition of the sample feedstock, molecular content of each molecule in the sample feedstock, molecular composition of an actual product corresponding to the sample feedstock, and actual content of each molecule in the actual product.

20. The method according to claim 19, wherein the training the set of reaction rules by using the sample feedstock information comprises:

processing the molecular composition of the sample feedstock according to a preset set of reaction rules, to obtain a reaction pathway corresponding to each molecule in the molecular composition of the sample feedstock;
obtaining first molecule composition of a device output product comprising the sample feedstock, an intermediate product, and a predicted product according to the reaction path corresponding to each molecule in the molecular composition of the sample feedstock; in the device output product, comprising: the sample feedstock, the intermediate product, and the predicted product;

calculating a first relative deviation according to the first molecular composition of the device output product and second molecular composition of the actual product;

if the first relative deviation meets a preset condition, fixing the set of reaction rules; and

if the first relative deviation does not meet the preset condition, adjusting a reaction rule in the set of reaction rules, and recalculating the first relative deviation according to the adjusted set of reaction rules until the first relative deviation meets the preset condition.

21. The method according to claim 20, wherein the calculating a first relative deviation according to the first molecular composition of the device output product and second molecular composition of the actual product comprises:

acquiring species of single molecules in the first molecule composition, to constitute a first set;

acquiring species of single molecules in the second molecule composition, to constitute a second set;

determining whether the second set is a subset of the first set;

if the second set is not a subset of the first set, obtaining a pre-stored relative deviation value that does not meet the preset condition as the first relative deviation; and

if the second set is a subset of the first set, calculating the first relative deviation by a calculating formula as follows:

$$x_1 = \frac{\text{card}\big((M - M_1 - M_2) - M_3\big)}{\text{card}\ (M - M_1 - M_2)}\ ;$$

where, $x_1$ is the first relative deviation, $M$ is the first set, $M_1$ is a set of species of single molecules in the molecular composition of the sample feedstock, $M_2$ is a set of species of single molecules in the molecular composition of the intermediate product, $M_3$ is the second set, and card represents the number of elements in the sets.

22. The method according to claim 19, wherein the training the reaction rate algorithm by using the sample feedstock information comprises:

calculating a reaction rate of a reaction path corresponding to each molecule in the molecular composition of the sample feedstock, respectively, according to the reaction rate algorithm;

obtaining predicted content of each molecule in a predicted product corresponding to the sample feedstock according to molecular content of each molecule in the sample feedstock and the reaction rate of the reaction path corresponding to the molecule;

calculating a second relative deviation according to the predicted content of each molecule in the predicted product and the actual content of each molecule in the actual product;

if the second relative deviation meets a preset condition, fixing the reaction rate algorithm; and

if the second relative deviation does not meet the preset condition, adjusting a parameter in the reaction rate algorithm, and recalculating the second relative deviation according to the adjusted reaction rate algorithm until the second relative deviation meets the preset condition.

23. The method according to claim 22, wherein the calculating a reaction rate of a reaction path corresponding to each molecule in the molecular composition of the sample feedstock, respectively, according to the reaction rate algorithm comprises:

calculating a reaction rate of each reaction path according to a reaction rate constant in the reaction rate algorithm;

wherein the reaction rate constant is determined according to a calculation formula as follows:

$$k = \frac{k_B E}{h} \exp(\frac{E\Delta S - \Delta E}{RE})\varphi \times P^\alpha\ ;$$

where, $k$ is the reaction rate constant, $k_B$ is the Boltzmann constant, $h$ is the Planck constant, $R$ is an ideal gas constant, $E$ is a temperature value of the environment at which the reaction path is located, exp is an exponential function with base of natural constant, $\Delta S$ is an entropy change before and after the reaction corresponding to the reaction rule corresponding to the reaction path, $\Delta E$ is a reaction energy barrier corresponding to the reaction rule corresponding to the reaction path, $\varphi$ is a catalyst activity factor, $P$ is a pressure value of the environment at which the reaction path is located, and $\alpha$ is a pressure influencing factor corresponding to the reaction rule corresponding to the reaction path.

24. The method according to any one of claims 17-23, wherein each petroleum processing device corresponds to a set of reaction rules.

25. An apparatus for obtaining a production and processing scheme of oil refinery and processing, wherein the apparatus comprising:

an acquisition unit configured to acquire molecular composition of crude oil;
a first processing unit configured to acquire molecular composition of various fractions obtained by distillation of the crude oil according to physical properties of various single molecules in the molecular composition of the crude oil, and serve the corresponding fractions as petroleum processing feedstocks, respectively input, according to a preset feedstock ratio, the molecular composition of the corresponding fractions into a product prediction model of a respective petroleum processing device, to obtain molecular composition of a corresponding predicted product and content of each single molecule in the predicted product;
a second processing unit configured to blend each of the predicted products which is used as a product blending feedstock according to a preset rule set, to obtain molecular composition of a plurality of mixed products and content of each single molecule in each of the mixed products, wherein each set of preset rules in the preset rule set includes the type and amount of the predicted product used;
a third processing unit configured to respectively calculate a product property of each of the mixed products according to the molecular composition of each of the mixed products and the content of each single molecule in each of the mixed products; and determine whether the product property of each of the mixed products meets any preset standard in a preset standard set; and
a fourth processing unit configured to, if the product property of each of the mixed products meets any preset standard in the preset standard set, acquire a target parameter according to all mixed products and determine whether the target parameter meets a preset condition, and, if the target parameter does not meet the preset condition, adjust the preset feedstock ratio, an operating parameter in the product prediction model and a preset rule in the preset rule set, to re-obtain a plurality of mixed products until the product property of each of the mixed products meets any preset standard in the preset standard set and the target parameter meets the preset condition;
if the target parameter meets a preset condition, output the preset feedstock ratio, the product prediction model, and the preset rule set as a production and processing scheme, and use the production and processing scheme to conduct oil refinery processing in an actual production process;
the target parameter is a content of substances in the product that will cause harm to the environment, or a proportion of products, which meet a preset standard, in all mixed products to all of the mixed products, the operating parameters in the product prediction model comprising pressure, temperature and space velocity;
the first processing unit is, in particular, configured to acquire each single molecule in the crude oil and the content of each single molecule, calculate a boiling point of each single molecule, respectively, cut the crude oil by distillation according to a preset fractional distillation range to obtain multiple fractions, and determine a single molecule and content of the single molecule contained in each of the fractions according to the boiling point and the content of each single molecule in the crude oil;
the first processing unit is further configured to, for two fractions with adjacent distillation ranges, take the fraction with a relatively high temperature in the distillation range as a first fraction, and take the fraction with a relatively low temperature in the distillation range as a second fraction;
calculate a minimum value of an overlapping interval of an overlapping distillation range of the first fraction and the second fraction by the following formula:

$$T_{\min} = T_{\text{cut}} \times (1 - SF)$$;

and
calculate a maximum value of the overlapping interval of the overlapping distillation range of the first fraction and the second fraction by the following formula:

$$T_{\max} = T_{\text{cut}} \times (1 + SF)$$;

where, $T_{min}$ is the minimum value of the overlapping interval, $T_{max}$ is the maximum value of the overlapping interval, $T_{cut}$ is the distillation cut temperature of the first fraction and the second fraction, and $SF$ is a separation index of the first fraction and the second fraction;

the first processing unit is further configured to calculate content of distilled part into the first fraction of each single molecule in the overlapping int erval and calculate content of distilled part into the second fraction of each single molecule in the overlapping interval according to the content of each single molecule and each single molecule corresponding to each boiling point of the overlapping interval, and obtain the content of each single molecule and each single molecule in each of the first fraction and the second fraction after the crude oil is cut by distillation according to the content of distilled part into the first fraction of each single molecule in the overlapping interval and the content of distilled part into the second fraction of each single molecule in the overlapping interval,

wherein the content of distilled part into the first fraction of each single molecule in the overlapping interval and the content of distilled part into the second fraction of each single molecule in the overlapping interval are calculated by the following equation:

$$C_h^i = \ln \ (\frac{T_i}{T_{\min}}) \times C^i$$
;

$$C_l^i = C^i - C_h^i$$
;

where, $C_h^i$ is the content of distilled part into the first fraction of the i-th single molecule in all molecules with a boiling point located in the overlapping interval, which the i-th single molecule has the boiling point located in the overlapping interval, $C_l^i$ is the content of distilled part into the first fraction of the i-th single molecule in all molecules with a boiling point located in the overlapping interval, which the i-th single molecule has the boiling point located in the overlapping interval, $T_i$ is the boiling point of the i-th single molecule, $T_{\min}$ is the minimum value of the overlapping interval, and $C^i$ is the content of the i-th single molecule in all molecules with a boiling point located in the overlapping interval, which the i-th single molecule has the boiling point located in the overlapping interval.

26. An system for obtaining a production and processing scheme of oil refinery comprising a processor, a communication interface, a memory, and a communication bus, wherein the processor, the communication interface, and the memory are in communication with each other via the communication bus;

the memory is configured to store a computer program; and
the processor is configured to carry out the method according to any one of claims 1-24 when executing the program stored in the memory.

27. A computer-readable storage medium, wherein the computer-readable storage medium has stored therein one or more programs, the one or more programs being executable by one or more processors to implement the method according to any one of claims 1-24.

**Patentansprüche**

1. Computerimplementiertes Verfahren zum Erhalten eines Produktions- und Verarbeitungsschemas der Erdölraffinationsverarbeitung, wobei das Verfahren Folgendes umfasst:

Erwerben von molekularer Zusammensetzung von Roherdöl;
Erwerben von molekularer Zusammensetzung von verschiedenen Fraktionen, die durch Destillation des Roherdöls erhalten werden, gemäß physikalischen Eigenschaften von verschiedenen einzelnen Molekülen in der molekularen Zusammensetzung des Roherdöls;
jeweils Eingeben, gemäß einem voreingestellten Ausgangsstoffverhältnis, der molekularen Zusammensetzung der entsprechenden Fraktionen in ein Produktvorhersagemodell einer jeweiligen Mineralölverarbeitungsvorrichtung als Mineralölverarbeitungsausgangsstoffe, um molekulare Zusammensetzung eines entsprechenden vorhergesagten Produkts und Gehalt jedes einzelnen Moleküls in dem vorhergesagten Produkt zu erhalten; Vermischen von jedem der vorhergesagten Produkte, das als ein Produktvermischungsausgangsstoff verwendet wird, gemäß einem voreingestellten Regelsatz, um molekulare Zusammensetzung einer Vielzahl von gemischten Produkten und Gehalt jedes einzelnen Moleküls in jedem der gemischten Produkte zu erhalten, wobei jeder Satz von voreingestellten Regeln in dem voreingestellten Regelsatz die Art und Menge des verwendeten vorhergesagten Produkts beinhaltet;

jeweils Berechnen einer Produkteigenschaft von jedem der gemischten Produkte gemäß der molekularen Zusammensetzung von jedem der gemischten Produkte und dem Gehalt jedes einzelnen Moleküls in jedem der gemischten Produkte; und Bestimmen, ob die Produkteigenschaft von jedem der gemischten Produkte einen beliebigen voreingestellten Standard in einem voreingestellten Standardsatz erfüllt;

wenn die Produkteigenschaft von jedem der gemischten Produkte einen voreingestellten Standard in dem voreingestellten Standardsatz erfüllt, Erwerben eines Zielparameters gemäß allen gemischten Produkten und Bestimmen, ob der Zielparameter eine voreingestellte Bedingung erfüllt; wobei der Zielparameter Folgendes repräsentiert: einen Gehalt an Substanzen in dem Produkt, welche die Umwelt schädigen; oder einen Anteil an Produkten, die einen voreingestellten Standard erfüllen, in allen gemischten Produkten zu allen der gemischten Produkte; und

wenn der Zielparameter die voreingestellte Bedingung nicht erfüllt, Anpassen des voreingestellten Ausgangsstoffverhältnisses, eines Betriebsparameters in dem Produktvorhersagemodell und einer voreingestellten Regel in dem voreingestellten Regelsatz, um eine Vielzahl von gemischten Produkten erneut zu erhalten, bis die Produkteigenschaft von jedem der gemischten Produkte einen beliebigen voreingestellten Standard in dem voreingestellten Standardsatz erfüllt und der Zielparameter die voreingestellte Bedingung erfüllt, wobei die Betriebsparameter in dem Produktvorhersagemodell Druck, Temperatur und Raumgeschwindigkeit umfassen;

wobei, wenn der Zielparameter eine voreingestellte Bedingung erfüllt, das voreingestellte Ausgangsstoffverhältnis, das Produktvorhersagemodell und der voreingestellte Regelsatz als ein Produktions- und Verarbeitungsschema ausgegeben werden, und Verwenden des Produktions- und Verarbeitungsschemas, um Erdölraffinationsverarbeitung in einem tatsächlichen Produktionsprozess durchzuführen;

wobei das Erwerben von molekularer Zusammensetzung von verschiedenen Fraktionen, die durch Destillation des Roherdöls gemäß physikalischen Eigenschaften von verschiedenen einzelnen Molekülen in der molekularen Zusammensetzung des Roherdöls erhalten werden, Folgendes umfasst:

Erwerben von jedem einzelnen Molekül in dem Roherdöl und dem Gehalt von jedem einzelnen Molekül; jeweils Berechnen eines Siedepunktes von jedem einzelnen Molekül; und

Schneiden des Roherdöls durch Destillation gemäß einem voreingestellten fraktionierten Destillationsbereich, um mehrere Fraktionen zu erhalten, und Bestimmen eines einzelnen Moleküls und Gehalts von jedem einzelnen Molekül, das in jeder der Fraktionen enthalten ist, gemäß dem Siedepunkt und dem Gehalt von jedem einzelnen Molekül in dem Roherdöl;

wobei das Verfahren ferner Folgendes umfasst:

für zwei Fraktionen mit benachbarten Destillationsbereichen, Nehmen der Fraktion mit einer relativ hohen Temperatur in dem Destillationsbereich als eine erste Fraktion und Nehmen der Fraktion mit einer relativ niedrigen Temperatur in dem Destillationsbereich als eine zweite Fraktion;

Berechnen eines Mindestwertes eines überlappenden Intervalls eines überlappenden Destillationsbereichs der ersten Fraktion und der zweiten Fraktion durch die folgende Formel:

$$T_{\min} = T_{\text{cut}} \times (1 - SF),$$

und

Berechnen eines Maximalwertes des überlappenden Intervalls des überlappenden Destillationsbereichs der ersten Fraktion und der zweiten Fraktion durch die folgende Formel:

$$T_{\max} = T_{\text{cut}} \times (1 + SF),$$

wobei $T_{\min}$ der Mindestwert des überlappenden Intervalls ist, $T_{\max}$ der Maximalwert des überlappenden Intervalls ist, $T_{\text{cut}}$ die Destillationsschnitttemperatur der ersten Fraktion und der zweiten Fraktion ist und $SF$ ein Trennungsindex der ersten Fraktion und der zweiten Fraktion ist;

wobei das Verfahren ferner Folgendes umfasst:

Berechnen von Gehalt von destilliertem Teil in die erste Fraktion von jedem einzelnen Molekül in dem überlappenden Intervall und Berechnen von Gehalt von destilliertem Teil in die zweite Fraktion von jedem einzelnen Molekül in dem überlappenden Intervall gemäß dem Gehalt von jedem einzelnen Molekül und jedem einzelnen Molekül entsprechend jedem Siedepunkt des überlappenden Intervalls;

wobei der Gehalt an destilliertem Teil in die erste Fraktion von jedem einzelnen Molekül in dem überlappenden Intervall und der Gehalt an destilliertem Teil in die zweite Fraktion von jedem einzelnen Molekül in dem überlappenden Intervall durch die folgende Gleichung berechnet werden:

$$C_h^i = \ln \left( \frac{T_i}{T_{\min}} \right) \times C^i ;$$

$$C_l^i = C^i - C_h^i ;$$

wobei $C_h^i$ der Gehalt an destilliertem Teil in die erste Fraktion des i-ten einzelnen Moleküls in allen Molekülen mit einem Siedepunkt ist, der sich in dem überlappenden Intervall befindet, wobei das i-te einzelne Molekül den sich in dem überlappenden Intervall befindlichen Siedepunkt aufweist, $C_l^i$ der Gehalt an destilliertem Teil in die erste Fraktion des i-ten einzelnen Moleküls in allen Molekülen ist, wobei sich ein Siedepunkt in dem überlappenden Intervall befindet, wobei das i-te einzelne Molekül den sich in dem überlappenden Intervall befindlichen Siedepunkt aufweist, $T_i$ der Siedepunkt des i-ten einzelnen Moleküls ist, $T_{\min}$ der Mindestwert des überlappenden Intervalls ist und $C^i$ der Gehalt des i-ten einzelnen Moleküls in allen Molekülen ist, wobei sich ein Siedepunkt in dem überlappenden Intervall befindet, wobei das i-te einzelne Molekül den sich in dem überlappenden Intervall befindlichen Siedepunkt aufweist; und

Erhalten des Gehalts von jedem einzelnen Molekül und jedem einzelnen Molekül in jeder von der ersten Fraktion und der zweiten Fraktion, nachdem das Roherdöl durch Destillation gemäß dem Gehalt an destilliertem Teil in die erste Fraktion von jedem einzelnen Molekül in dem überlappenden Intervall und dem Gehalt an destilliertem Teil in die zweite Fraktion von jedem einzelnen Molekül in dem überlappenden Intervall geschnitten ist.

2.  Verfahren nach Anspruch 1, wobei das Verfahren ferner Folgendes umfasst:

Erwerben eines Eingangsflusses von Mineralölverarbeitungsausgangsstoffen, die in jede der Mineralölverarbeitungsvorrichtungen eingegeben werden;

Bestimmen, ob jeder der Eingangsflüsse einen voreingestellten Eingangsflussbereich der jeweiligen Mineralölverarbeitungsvorrichtung erfüllt; und

Anpassen des voreingestellten Ausgangsstoffverhältnisses, wenn ein beliebiger der Eingangsflüsse den voreingestellten Eingangsflussbereich der jeweiligen Mineralölverarbeitungsvorrichtung nicht erfüllt, und jeweiliges erneutes Eingeben, gemäß dem angepassten voreingestellten Ausgangsstoffverhältnis, der entsprechenden Fraktionen in das Produktvorhersagemodell der jeweiligen Mineralölverarbeitungsvorrichtung als Mineralölverarbeitungsausgangsstoffe, bis jeder der Eingangsflüsse den voreingestellten Eingangsflussbereich der jeweiligen Mineralölverarbeitungsvorrichtung erfüllt.

3.  Verfahren nach Anspruch 1, wobei das Verfahren ferner Folgendes umfasst:

Erwerben von molekularer Zusammensetzung der Mineralölverarbeitungsausgangsstoffe, die in jede der Mineralölverarbeitungsvorrichtungen eingegeben werden, und Gehalt von jedem einzelnen Molekül in den Mineralölverarbeitungsausgangsstoffen;

Berechnen einer physikalischen Eigenschaft von jedem einzelnen Molekül in den Mineralölverarbeitungsausgangsstoffen, Berechnen einer Ausgangsstoffeigenschaft der Mineralölverarbeitungsausgangsstoffe gemäß der physikalischen Eigenschaft von jedem einzelnen Molekül und dem Gehalt von jedem einzelnen Molekül in den Mineralölverarbeitungsausgangsstoffen;

Bestimmen, ob jede der Ausgangsstoffeigenschaften ein voreingestelltes physikalisches Eigenschaftsbeschränkungsintervall der jeweiligen Mineralölverarbeitungsvorrichtung erfüllt; und

wenn beliebige der Ausgangsstoffeigenschaften das voreingestellte physikalische Eigenschaftsbeschränkungsintervall der jeweiligen Mineralölverarbeitungsvorrichtung nicht erfüllen, Anpassen des voreingestellten Ausgangsstoffverhältnisses, und jeweiliges erneutes Eingeben, gemäß dem angepassten voreingestellten Ausgangsstoffverhältnis, der entsprechenden Fraktionen in das Produktvorhersagemodell der jeweiligen Mineralölverarbeitungsvorrichtung als Mineralölverarbeitungsausgangsstoffe, bis jede der Ausgangsstoffeigenschaften das voreingestellte physikalische Eigenschaftsbeschränkungsintervall der jeweiligen Mineralölverarbeitungsvorrichtung erfüllt.

4. Verfahren nach Anspruch 1, wobei das Verfahren ferner Folgendes umfasst:
wenn die Produkteigenschaft von beliebigem gemischten Produkt keinen voreingestellten Standard in dem voreingestellten Standardsatz erfüllt, Anpassen der voreingestellten Regel in dem voreingestellten Regelsatz und Vermischen von jedem der Produktvermischungsausgangsstoffe gemäß dem angepassten voreingestellten Regelsatz, um eine Vielzahl von gemischten Produkten erneut zu erhalten, bis die Produkteigenschaft von jedem der gemischten Produkte einen beliebigen voreingestellten Standard in dem voreingestellten Standardsatz erfüllt.

5. Verfahren nach Anspruch 1, wobei das jeweilige Berechnen einer Produkteigenschaft von jedem der gemischten Produkte gemäß der molekularen Zusammensetzung von jedem der gemischten Produkte und dem Gehalt von jedem einzelnen Molekül in jedem der gemischten Produkte Folgendes umfasst:

Erwerben von erster molekularer Zusammensetzung von jedem Satz der Produktvermischungsausgangsstoffe und erstem Komponentengehalt von jedem einzelnen Molekül in jedem Satz der Produktvermischungsausgangsstoffe;
basierend auf dem voreingestellten Regelsatz, Erhalten von zweiter molekularer Zusammensetzung von jedem von gemischten Produkten und zweitem Komponentengehalt von jedem einzelnen Molekül in jedem von gemischten Produkten gemäß der ersten molekularen Zusammensetzung von jedem Satz des Produktvermischungsausgangsstoffes und dem ersten Komponentengehalt von jedem einzelnen Molekül in jedem Satz der Produktvermischungsausgangsstoffe;
Berechnen einer physikalischen Eigenschaft von jedem einzelnen Molekül in jedem der gemischten Produkte gemäß der Anzahl an Gruppen von jeder Gruppe, die in jedem einzelnen Molekül in jedem der gemischten Produkte enthalten ist, und einem Beitragswert von jeder Gruppe zu der physikalischen Eigenschaft; und
Berechnen einer Produkteigenschaft von jedem der gemischten Produkte gemäß der physikalischen Eigenschaft und dem zweiten Komponentengehalt von jedem einzelnen Molekül in jedem der gemischten Produkte.

6. Verfahren nach Anspruch 5, wobei Berechnung der physikalischen Eigenschaft von jedem einzelnen Molekül Folgendes umfasst:

für jedes einzelne Molekül, Erwerben der Anzahl an Gruppen von jeder Gruppe, die das einzelne Molekül darstellt, und eines Beitragswertes von jeder Gruppe zu der physikalischen Eigenschaft; und
Eingeben der Anzahl an Gruppen von jeder Gruppe, die das einzelne Molekül darstellt, und des Beitragswertes von jeder Gruppe zu der physikalischen Eigenschaft in ein vortrainiertes Eigenschaftsberechnungsmodell, um die physikalische Eigenschaft des einzelnen Moleküls zu erwerben, die durch das vortrainierte Eigenschaftsberechnungsmodell ausgegeben wird.

7. Verfahren nach Anspruch 6, wobei vor dem Eingeben der Anzahl an Gruppen von jeder Gruppe, die das einzelne Molekül darstellt, und des Beitragswertes von jeder Gruppe zu der physikalischen Eigenschaften in ein vortrainiertes Eigenschaftsberechnungsmodell das Verfahren ferner Folgendes umfasst:

Vergleichen der Anzahl an Gruppen von jeder Gruppe, die das einzelne Molekül darstellt, mit molekularen Informationen eines einzelnen Vorlagenmoleküls mit bekannten physikalischen Eigenschaften, die in einer Datenbank vorgespeichert sind, wobei die molekularen Informationen die Anzahl an Gruppen von jeder Gruppe, die das einzelne Vorlagenmolekül darstellt, umfassen;
Bestimmen, ob es ein gleiches einzelnes Vorlagenmolekül wie das einzelne Molekül gibt;
wenn es ein gleiches einzelnes Vorlagenmolekül wie das einzelne Molekül gibt, Ausgeben der physikalischen Eigenschaften des einzelnen Vorlagenmoleküls als eine physikalische Eigenschaft des einzelnen Moleküls; und
wenn es kein gleiches einzelnes Vorlagenmolekül wie das einzelne Molekül gibt, dann Durchführen des Schrittes des Eingebens der Anzahl an Gruppen von jeder Gruppe, die das einzelne Molekül darstellt, und des Beitragswertes von jeder Gruppe zu der physikalischen Eigenschaft in ein vortrainiertes Eigenschaftsberechnungsmodell.

8. Verfahren nach Anspruch 1, wobei das Berechnen eines Siedepunktes von jedem einzelnen Molekül Folgendes umfasst:

für jedes von dem einzelnen Molekül, Erwerben der Anzahl an Gruppen von jeder Gruppe, die das einzelne Molekül darstellt, und eines Beitragswertes von jeder Gruppe zu dem Siedepunkt; und
Eingeben der Anzahl an Gruppen von jeder Gruppe, die das einzelne Molekül darstellt, und des Beitragswertes von jeder Gruppe zu dem Siedepunkt in ein vortrainiertes Eigenschaftsberechnungsmodell, um den Siedepunkt des einzelnen Moleküls zu erwerben, der durch das vortrainierte Eigenschaftsberechnungsmodell ausgegeben wird.

9. Verfahren nach Anspruch 8, wobei vor dem Eingeben der Anzahl an Gruppen von jeder Gruppe, die das einzelne Molekül darstellt, und des Beitragswertes von jeder Gruppe zu dem Siedepunkt in ein vortrainiertes Eigenschaftsberechnungsmodell das Verfahren ferner Folgendes umfasst:

Vergleichen der Anzahl an Gruppen von jeder Gruppe, die das einzelne Molekül darstellt, mit molekularen Informationen eines einzelnen Vorlagenmoleküls mit bekanntem Siedepunkt, der in einer Datenbank vorgespeichert ist, wobei die molekularen Informationen die Anzahl an Gruppen von jeder Gruppe, die das einzelne Vorlagenmolekül darstellt, umfassen;
Bestimmen, ob es ein gleiches einzelnes Vorlagenmolekül wie das einzelne Molekül gibt;
wenn es ein gleiches einzelnes Vorlagenmolekül wie das einzelne Molekül gibt, Ausgeben des Siedepunktes des einzelnen Vorlagenmoleküls als einen Siedepunkt des einzelnen Moleküls; und
wenn es kein gleiches einzelnes Vorlagenmolekül wie das einzelne Molekül gibt, dann Durchführen des Schrittes des Eingebens der Anzahl an Gruppen von jeder Gruppe, die das einzelne Molekül darstellt, und des Beitragswertes von jeder Gruppe zu dem Siedepunkt in ein vortrainiertes Eigenschaftsberechnungsmodell.

10. Verfahren nach Anspruch 6 oder 8, wobei ein Schritt des Trainierens des Eigenschaftsberechnungsmodells Folgendes umfasst:

Konstruieren eines Eigenschaftsberechnungsmodells eines einzelnen Moleküls;
Erwerben der Anzahl an Gruppen von jeder Gruppe, die ein einzelnes Probenmolekül darstellt; wobei die physikalische Eigenschaft des einzelnen Probenmoleküls bekannt ist;
Eingeben der Anzahl an Gruppen von jeder Gruppe, die das einzelne Probenmolekül darstellt, in das Eigenschaftsberechnungsmodell;
Erwerben einer vorhergesagten physikalischen Eigenschaft des einzelnen Probenmoleküls, die durch das Eigenschaftsberechnungsmodell ausgegeben wird;
wenn ein Abweichungswert zwischen der vorhergesagten physikalischen Eigenschaft und der physikalischen Eigenschaft, die bekannt ist, weniger als ein voreingestellter Abweichungsschwellenwert ist, Bestimmen, dass das Eigenschaftsberechnungsmodell konvergiert, Erwerben eines Beitragswertes entsprechend jeder Gruppe in dem Eigenschaftsberechnungsmodell, das konvergiert ist, und Speichern des Beitragswertes als einen Beitragswert der Gruppe zu der physikalischen Eigenschaft; und
wenn der Abweichungswert zwischen der vorhergesagten physikalischen Eigenschaft und der physikalischen Eigenschaft, die bekannt ist, größer als der oder gleich dem Abweichungsschwellenwert ist, Anpassen eines Beitragswertes entsprechend jeder Gruppe in dem Eigenschaftsberechnungsmodell, bis das Eigenschaftsberechnungsmodell konvergiert.

11. Verfahren nach Anspruch 10, wobei das Eigenschaftsberechnungsmodell wie unten gezeigt erstellt ist:

$$f = a + \sum_i n_i \Delta f_i$$
;

wobei $f$ die physikalische Eigenschaft des einzelnen Moleküls ist, $n_i$ die Anzahl an Gruppen der $i$-ten Gruppe ist, $\Delta f_i$ der Beitragswert der $i$-ten Gruppe zu der physikalischen Eigenschaft ist und $\alpha$ eine assoziierte Konstante ist.

12. Verfahren nach Anspruch 10, wobei das Erwerben der Anzahl an Gruppen von jeder Gruppe, die ein einzelnes Probenmolekül darstellt, Folgendes umfasst:

Bestimmen einer primären Gruppe, der Anzahl an Gruppen der primären Gruppe, einer mehrstufigen Gruppe und der Anzahl an Gruppen der mehrstufigen Gruppe in allen Gruppen des einzelnen Moleküls;
Nehmen aller Gruppen, die das einzelne Molekül darstellen, als die primäre Gruppe; und
Nehmen von verschiedenen Gruppen, die koexistieren und zu einer gleichen physikalischen Eigenschaft beitragen, als die mehrstufige Gruppe und Nehmen der Anzahl der verschiedenen Gruppen als eine Ebene der mehrstufigen Gruppe.

**13.** Verfahren nach Anspruch 12, wobei

das Eigenschaftsberechnungsmodell wie unten gezeigt erstellt ist:

$$f = a + \sum_i \mathrm{m}_{1i} \Delta f_{1i} + \sum_j m_{2j} \Delta f_{2j} \dots\dots + \sum_l m_{Nl} \Delta f_{Nl} \; ;$$

wobei $f$ die physikalische Eigenschaft des einzelnen Moleküls ist, $\mathrm{m}_{1i}$ die Anzahl an Gruppen der $i$-ten Gruppe in der primären Gruppe ist, $\Delta f_{1i}$ der Beitragswert der $i$-ten Gruppe in der primären Gruppe zu der physikalischen Eigenschaft ist, $m_{2j}$ die Anzahl an Gruppen der $j$-ten Gruppe in einer sekundären Gruppe ist, $\Delta f_{2j}$ der Beitragswert der $j$-ten Gruppe in der sekundären Gruppe zu der physikalischen Eigenschaft ist, $m_{Nl}$ die Anzahl an Gruppen der $l$-ten Gruppe in einer N-stufigen Gruppe ist, $\Delta f_{Nl}$ der Beitragswert der $l$-ten Gruppe in der N-stufigen Gruppe zu der physikalischen Eigenschaft ist, $\alpha$ eine assoziierte Konstante ist und $N$ eine positive ganze Zahl größer als oder gleich 2 ist.

**14.** Verfahren nach Anspruch 8, wobei das Erwerben der Anzahl an Gruppen von jeder Gruppe, die das einzelne Molekül darstellt, Folgendes umfasst:

Bestimmen einer primären Gruppe, der Anzahl an Gruppen der primären Gruppe, einer mehrstufigen Gruppe und der Anzahl an Gruppen der mehrstufigen Gruppe in allen Gruppen des einzelnen Moleküls;
Nehmen aller Gruppen, die das einzelne Molekül darstellen, als die primäre Gruppe; und
Nehmen von verschiedenen Gruppen, die koexistieren und zu einer gleichen physikalischen Eigenschaft beitragen, als die mehrstufige Gruppe und Nehmen der Anzahl der verschiedenen Gruppen als eine Ebene der mehrstufigen Gruppe.

**15.** Verfahren nach Anspruch 14, wobei
das Eingeben der Anzahl an Gruppen von jeder Gruppe, die das einzelne Molekül darstellt, und des Beitragswertes von jeder Gruppe zu dem Siedepunkt in ein vortrainiertes Eigenschaftsberechnungsmodell, um den Siedepunkt des einzelnen Moleküls zu erwerben, der durch das vortrainierte Eigenschaftsberechnungsmodell ausgegeben wird, Folgendes umfasst:

Berechnen des Siedepunktes des einzelnen Moleküls gemäß dem folgenden Eigenschaftsberechnungsmodell:

$$T = \frac{SOL \times GRUPPE_1 + SOL \times GRUPPE_2 + \dots\dots + SOL \times GRUPPE_N}{(SOL \times N\mathrm{umh})^d + b} + c \; ;$$

wobei $T$ der Siedepunkt des einzelnen Moleküls ist, $SOL$ ein einzelner Molekülvektor ist, der gemäß der Anzahl an Gruppen von jeder Gruppe, die das einzelne Molekül darstellt, umgewandelt wird, $GRUPPE_{11}$ ein erster Beitragswertvektor ist, der gemäß einem Beitragswert der primären Gruppe zu dem Siedepunkt umgewandelt wird, $GRUPPE_{12}$ ein zweiter Beitragswertvektor ist, der gemäß einem Beitragswert der sekundären Gruppe zu dem Siedepunkt umgewandelt wird, $GRUPPE_{1N}$ ein N-ter Beitragswertvektor ist, der gemäß einem Beitragswert der N-stufigen Gruppe zu dem Siedepunkt umgewandelt wird, Numh die Anzahl an Atomen außer dem Wasserstoffatom in dem einzelnen Molekül ist, $d$ eine erste voreingestellte Konstante ist, $b$ eine zweite voreingestellte Konstante ist, c eine dritte voreingestellte Konstante ist und $N$ eine positive ganze Zahl größer als oder gleich 2 ist.

**16.** Verfahren nach Anspruch 15, wobei
das Umwandeln des einzelnen Molekülvektors gemäß der Anzahl an Gruppen von jeder Gruppe, die das einzelne Molekül darstellt, Folgendes umfasst:

Nehmen der Anzahl an Spezies an Gruppen als eine Dimension des einzelnen Molekülvektors; und
Nehmen der Anzahl an Gruppen von jeder Gruppe als einen Elementwert der entsprechenden Dimension in dem einzelnen Molekülvektor,
wobei das Umwandeln des ersten Beitragswertvektors gemäß einem Beitragswert der primären Gruppe zu dem Siedepunkt Folgendes umfasst:

Nehmen der Anzahl an Arten an primären Gruppen als eine Dimension des ersten Beitragswertvektors; und
Nehmen des Beitragswertes von jeder primären Gruppe zu dem Siedepunkt als einen Elementwert der entsprechenden Dimension in dem ersten Beitragswertvektor,
wobei das Umwandeln des zweiten Beitragswertvektors gemäß einem Beitragswert der sekundären Gruppe zu dem Siedepunkt Folgendes umfasst:

Nehmen der Anzahl an Arten an sekundären Gruppen als eine Dimension des zweiten Beitragswertvektors; und
Nehmen des Beitragswertes von jeder sekundären Gruppe zu dem Siedepunkt als einen Elementwert der entsprechenden Dimension in dem zweiten Beitragswertvektor,
wobei das Umwandeln des N-ten Beitragswertvektors gemäß einem Beitragswert von jeder N-stufigen Gruppe zu dem Siedepunkt Folgendes umfasst:

Nehmen der Anzahl an Arten an N-stufigen Gruppen als eine Dimension des N-ten Beitragswertvektors; und
Nehmen des Beitragswertes von jeder N-stufigen Gruppe zu dem Siedepunkt als einen Elementwert der entsprechenden Dimension in dem N-ten Beitragswertvektor.

17. Verfahren nach Anspruch 1, wobei das jeweilige Eingeben, gemäß einem voreingestellten Ausgangsstoffverhältnis, der entsprechenden Fraktionen in ein Produktvorhersagemodell einer jeweiligen Mineralölverarbeitungsvorrichtung Folgendes umfasst:

Erhalten von verschiedenen Mengen von jeder Fraktion gemäß dem voreingestellten Ausgangsstoffverhältnis, und jeweils Eingeben von jeder Fraktion in das Produktvorhersagemodell der jeweiligen Mineralölverarbeitungsvorrichtung,
wobei die Mineralölverarbeitungsvorrichtung eine katalytische Crackeinheit, eine verzögerte Kokereieinheit, eine Resthydrobehandlungseinheit, eine Hydrocrackeinheit, eine Dieselhydroaufbereitungseinheit, eine Dieselhydroraffinerieeinheit, eine Gashydroraffinerieeinheit, eine katalytische Umformeinheit und eine Alkylierungseinheit umfasst.

18. Verfahren nach Anspruch 17, wobei ein Schritt des Trainierens des Produktvorhersagemodells Folgendes umfasst:

Erstellen eines Produktvorhersagemodells; wobei das Produktvorhersagemodell Folgendes umfasst: einen Satz von Reaktionsregeln, der eine Vielzahl von Reaktionsregeln und einen Reaktionsratenalgorithmus umfasst;
Erwerben von Probenausgangsstoffinformationen für einen Probenausgangsstoff;
Trainieren des Satzes von Reaktionsregeln durch Verwenden der Probenausgangsstoffinformationen, und Festlegen des Satzes von Reaktionsregeln, der trainiert worden ist; und
Trainieren des Reaktionsratenalgorithmus durch Verwenden der Probenausgangsstoffinformationen, und Festlegen des Reaktionsratenalgorithmus, der trainiert worden ist, um das Produktvorhersagemodell zu erhalten, das trainiert worden ist.

19. Verfahren nach Anspruch 18, wobei die Probenausgangsstoffinformationen des Probenausgangsstoffes Folgendes umfassen: molekulare Zusammensetzung des Probenausgangsstoffes, molekularer Gehalt von jedem Molekül in dem Probenausgangsstoff, molekulare Zusammensetzung eines tatsächlichen Produkts entsprechend dem Probenausgangsstoff und tatsächlicher Gehalt von jedem Molekül in dem tatsächlichen Produkt.

20. Verfahren nach Anspruch 19, wobei das Trainieren des Satzes von Reaktionsregeln durch Verwenden der Probenausgangsstoffinformationen Folgendes umfasst:

Verarbeiten der molekularen Zusammensetzung des Probenausgangsstoffes gemäß einem voreingestellten Satz von Reaktionsregeln, um einen Reaktionsweg entsprechend jedem Molekül in der molekularen Zusammensetzung des Probenausgangsstoffes zu erhalten;

Erhalten von erster Molekülzusammensetzung eines Vorrichtungsausgangsprodukts, welches den Probenausgangsstoff, ein Zwischenprodukt und ein vorhergesagtes Produkt umfasst, gemäß dem Reaktionsweg entsprechend jedem Molekül in der molekularen Zusammensetzung des Probenausgangsstoffes; in dem Vorrichtungsausgangsprodukt, umfassend: den Probenausgangsstoff, das Zwischenprodukt und das vorhergesagte Produkt;

Berechnen einer ersten relativen Abweichung gemäß der ersten molekularen Zusammensetzung des Vorrichtungsausgangsprodukts und zweiten molekularen Zusammensetzung des tatsächlichen Produkts;

wenn die erste relative Abweichung eine voreingestellte Bedingung erfüllt, Festlegen des Satzes von Reaktionsregeln; und

wenn die erste relative Abweichung die voreingestellte Bedingung nicht erfüllt, Anpassen einer Reaktionsregel in dem Satz von Reaktionsregeln und erneutes Berechnen der ersten relativen Abweichung gemäß dem angepassten Satz von Reaktionsregeln, bis die erste relative Abweichung die voreingestellte Bedingung erfüllt.

21. Verfahren nach Anspruch 20, wobei das Berechnen einer ersten relativen Abweichung gemäß der ersten molekularen Zusammensetzung des Vorrichtungsausgangsprodukts und zweiten molekularen Zusammensetzung des tatsächlichen Produkts Folgendes umfasst:

Erwerben von Spezies von einzelnen Molekülen in der ersten Molekülzusammensetzung, um einen ersten Satz darzustellen;

Erwerben von Spezies von einzelnen Molekülen in der zweiten Molekülzusammensetzung, um einen zweiten Satz darzustellen;

Bestimmen, ob der zweite Satz ein Teilsatz des ersten Satzes ist;

wenn der zweite Satz kein Teilsatz des ersten Satzes ist, Erhalten eines vorgespeicherten relativen Abweichungswertes, der die voreingestellte Bedingung nicht erfüllt, als die erste relative Abweichung; und

wenn der zweite Satz ein Teilsatz des ersten Satzes ist, Berechnen der ersten relativen Abweichung durch eine Berechnungsformel wie folgt:

$$x_1 = \frac{\text{Karte}\left((M - M_1 - M_2) - M_3\right)}{\text{Karte}\left(M - M_1 - M_2\right)};$$

wobei $x_1$ die erste relative Abweichung ist, M der erste Satz ist, $M_1$ ein Satz von Spezies von einzelnen Molekülen in der molekularen Zusammensetzung des Probenausgangsstoffes ist, $M_2$ ein Satz von Spezies von einzelnen Molekülen in der molekularen Zusammensetzung des Zwischenproduktes ist, $M_3$ der zweite Satz ist und Karte die Anzahl an Elementen in den Sätzen repräsentiert.

22. Verfahren nach Anspruch 19, wobei das Trainieren des Reaktionsratenalgorithmus durch Verwenden der Probenausgangsstoffinformationen Folgendes umfasst:

jeweils Berechnen einer Reaktionsrate eines Reaktionsweges entsprechend jedem Molekül in der molekularen Zusammensetzung des Probenausgangsstoffes gemäß dem Reaktionsratenalgorithmus;

Erhalten von vorhergesagtem Gehalt von jedem Molekül in einem vorhergesagten Produkt entsprechend dem Probenausgangsstoff gemäß molekularem Gehalt von jedem Molekül in dem Probenausgangsstoff und der Reaktionsrate des Reaktionsweges entsprechend dem Molekül;

Berechnen einer zweiten relativen Abweichung gemäß dem vorhergesagten Gehalt von jedem Molekül in dem vorhergesagten Produkt und dem tatsächlichen Gehalt von jedem Molekül in dem tatsächlichen Produkt;

wenn die zweite relative Abweichung eine voreingestellte Bedingung erfüllt, Festlegen des Reaktionsratenalgorithmus; und

wenn die zweite relative Abweichung die voreingestellte Bedingung nicht erfüllt, Anpassen eines Parameters in dem Reaktionsratenalgorithmus und erneutes Berechnen der zweiten relativen Abweichung gemäß dem angepassten Reaktionsratenalgorithmus, bis die zweite relative Abweichung die voreingestellte Bedingung erfüllt.

23. Verfahren nach Anspruch 22, wobei das jeweilige Berechnen einer Reaktionsrate eines Reaktionsweges entsprechend jedem Molekül in der molekularen Zusammensetzung des Probenausgangsstoffes gemäß dem Reaktionsratenalgorithmus Folgendes umfasst:

Berechnen einer Reaktionsrate von jedem Reaktionsweg gemäß einer Reaktionsratenkonstante in dem Reaktionsratenalgorithmus;

wobei die Reaktionsratenkonstante gemäß einer Berechnungsformel wie folgt bestimmt wird:

$$k = \frac{k_B E}{h} \exp(\frac{E\Delta S - \Delta E}{RE})\varphi \times P^\alpha$$

wobei $k$ die Reaktionsratenkonstante ist, $k_B$ die Boltzmann-Konstante ist, $h$ die Planck-Konstante ist, $R$ eine ideale Gaskonstante ist, $E$ ein Temperaturwert der Umgebung ist, in der sich der Reaktionsweg befindet, exp eine Exponentialfunktion mit Basis von natürlicher Konstante ist, $\Delta S$ eine Entropieänderung vor und nach der Reaktion entsprechend der Reaktionsregel entsprechend dem Reaktionsweg ist, $\Delta E$ eine Reaktionsenergie-barriere entsprechend der Reaktionsregel entsprechend dem Reaktionsweg ist, $\varphi$ ein Katalysatoraktivitätsfaktor ist, $P$ ein Druckwert der Umgebung ist, in der sich der Reaktionsweg befindet, und $\alpha$ ein Druckbeeinflussungs-faktor entsprechend der Reaktionsregel entsprechend dem Reaktionsweg ist.

24. Verfahren nach einem der Ansprüche 17-23, wobei jede Mineralölverarbeitungsvorrichtung einem Satz von Reaktionsregeln entspricht.

25. Vorrichtung zum Erhalten eines Produktions- und Verarbeitungsschemas von Erdölraffination und -verarbeitung, wobei die Vorrichtung Folgendes umfasst:

eine Erwerbseinheit, die konfiguriert ist, um molekulare Zusammensetzung von Roherdöl zu erwerben;
eine erste Verarbeitungseinheit, die konfiguriert ist, um molekulare Zusammensetzung von verschiedenen Fraktionen zu erwerben, die durch Destillation des Roherdöls gemäß physikalischen Eigenschaften von verschiedenen einzelnen Molekülen in der molekularen Zusammensetzung des Roherdöls erhalten werden und den entsprechenden Fraktionen als Mineralölverarbeitungsausgangsstoffe dienen, jeweils Eingeben, gemäß einem voreingestellten Ausgangsstoffverhältnis, der molekularen Zusammensetzung der entsprechenden Fraktionen in ein Produktvorhersagemodell einer jeweiligen Mineralölverarbeitungsvorrichtung, um molekulare Zusammensetzung eines entsprechenden vorhergesagten Produkts und Gehalt von jedem einzelnen Molekül in dem vorhergesagten Produkt zu erhalten;
eine zweite Verarbeitungseinheit, die konfiguriert ist, um jedes von den vorhergesagten Produkten, das als ein Produktvermischungsausgangsstoff verwendet wird, gemäß einem voreingestellten Regelsatz zu vermischen, um molekulare Zusammensetzung von einer Vielzahl von gemischten Produkten und Gehalt von jedem einzelnen Molekül in jedem der gemischten Produkte zu erhalten, wobei jeder Satz von voreingestellten Regeln in dem voreingestellten Regelsatz die Art und Menge des verwendeten vorhergesagten Produkts beinhaltet;
eine dritte Verarbeitungseinheit, die konfiguriert ist, um jeweils eine Produkteigenschaft von jedem der gemischten Produkte gemäß der molekularen Zusammensetzung von jedem der gemischten Produkte und dem Gehalt von jedem einzelnen Molekül in jedem der gemischten Produkte zu berechnen; und Bestimmen, ob die Produkteigenschaft von jedem der gemischten Produkte einen beliebigen voreingestellten Standard in einem voreingestellten Standardsatz erfüllt; und
eine vierte Verarbeitungseinheit, die konfiguriert ist, um, wenn die Produkteigenschaft von jedem der gemischten Produkte einen beliebigen voreingestellten Standard in dem voreingestellten Standardsatz erfüllt, einen Zielparameter gemäß allen gemischten Produkten zu erwerben und zu bestimmen, ob der Zielparameter eine voreingestellte Bedingung erfüllt, und wenn der Zielparameter die voreingestellte Bedingung nicht erfüllt, das voreingestellte Ausgangsstoffverhältnis, einen Betriebsparameter in dem Produktvorhersagemodell und eine voreingestellte Regel in dem voreingestellten Regelsatz anzupassen, um eine Vielzahl von gemischten Produkten erneut zu erhalten, bis die Produkteigenschaft von jedem der gemischten Produkte einen beliebigen voreingestellten Standard in dem voreingestellten Standardsatz erfüllt und der Zielparameter die voreingestellte Bedingung erfüllt;
wenn der Zielparameter eine voreingestellte Bedingung erfüllt, Ausgeben des voreingestellten Ausgangsstoffverhältnisses, des Produktvorhersagemodells und des voreingestellten Regelsatzes als ein Produktions- und Verarbeitungsschema, und Verwenden des Produktions- und Verarbeitungsschemas, um Erdölraffinationsverarbeitung in einem tatsächlichen Produktionsprozess durchzuführen;
wobei der Zielparameter ein Gehalt von Substanzen in dem Produkt ist, welche die Umgebung schädigen, oder ein Anteil an Produkten, die einen voreingestellten Standard erfüllen, in allen gemischten Produkten zu allen der gemischten Produkte, wobei die Betriebsparameter in dem Produktvorhersagemodell Druck, Temperatur und Raumgeschwindigkeit umfassen;
die erste Verarbeitungseinheit insbesondere konfiguriert ist, um jedes einzelne Molekül in dem Roherdöl und den Gehalt von jedem einzelnen Molekül zu erwerben, jeweils einen Siedepunkt von jedem einzelnen Molekül zu berechnen, das Roherdöl durch Destillation gemäß einem voreingestellten fraktionierten Destillationsbereich zu schneiden, um mehrere Fraktionen zu erhalten, und ein einzelnes Molekül und Gehalt des einzelnen Moleküls, das in jeder der Fraktionen enthalten ist, gemäß dem Siedepunkt und dem Gehalt von jedem einzelnen Molekül in dem Roherdöl zu bestimmen;

die erste Verarbeitungseinheit ferner konfiguriert ist, um für zwei Fraktionen mit benachbarten Destillationsbereichen die Fraktion mit einer relativ hohen Temperatur in dem Destillationsbereich als eine erste Fraktion zu nehmen und die Fraktion mit einer relativ niedrigen Temperatur in dem Destillationsbereich als eine zweite Fraktion zu nehmen;

Berechnen eines Mindestwertes eines überlappenden Intervalls eines überlappenden Destillationsbereichs der ersten Fraktion und der zweiten Fraktion durch die folgende Formel:

$$T_{\min} = T_{\mathrm{cut}} \times (1 - SF)$$
;

; und

Berechnen eines Maximalwertes des überlappenden Intervalls des überlappenden Destillationsbereichs der ersten Fraktion und der zweiten Fraktion durch die folgende Formel:

$$T_{\max} = T_{\mathrm{cut}} \times (1 + SF)$$
;

wobei $T_{\min}$ der Mindestwert des überlappenden Intervalls ist, $T_{\max}$ der Maximalwert des überlappenden Intervalls ist, $T_{\mathrm{cut}}$ die Destillationsschnitttemperatur der ersten Fraktion und der zweiten Fraktion ist und $SF$ ein Trennungsindex der ersten Fraktion und der zweiten Fraktion ist;

die erste Verarbeitungseinheit ferner konfiguriert ist, um Gehalt von destilliertem Teil in die erste Fraktion von jedem einzelnen Molekül in dem überlappenden Intervall zu berechnen und Gehalt von destilliertem Teil in die zweite Fraktion von jedem einzelnen Molekül in dem überlappenden Intervall gemäß dem Gehalt von jedem einzelnen Molekül und jedem einzelnen Molekül entsprechend jedem Siedepunkt des überlappenden Intervalls zu berechnen und den Gehalt von jedem einzelnen Molekül und jedem einzelnen Molekül in jeder von der ersten Fraktion und der zweiten Fraktion zu erhalten, nachdem Roherdöl durch Destillation gemäß dem Gehalt von destilliertem Teil in die erste Fraktion von jedem einzelnen Molekül in dem überlappenden Intervall und dem Gehalt von destilliertem Teil in die zweite Fraktion von jedem einzelnen Molekül in dem überlappenden Intervall geschnitten ist,

wobei der Gehalt an destilliertem Teil in die erste Fraktion von jedem einzelnen Molekül in dem überlappenden Intervall und der Gehalt an destilliertem Teil in die zweite Fraktion von jedem einzelnen Molekül in dem überlappenden Intervall durch die folgende Gleichung berechnet werden:

$$C_h^i = \ln \left( \frac{T_i}{T_{\min}} \right) \times C^i$$
;

$$C_l^i = C^i - C_h^i$$
;

wobei $C_h^i$ der Gehalt an destilliertem Teil in die erste Fraktion des i-ten einzelnen Moleküls in allen Molekülen mit einem Siedepunkt ist, der sich in dem überlappenden Intervall befindet, wobei das i-te einzelne Molekül den sich in dem überlappenden Intervall befindlichen Siedepunkt aufweist, $C_l^i$ der Gehalt an destilliertem Teil in die erste Fraktion des i-ten einzelnen Moleküls in allen Molekülen ist, wobei sich ein Siedepunkt in dem überlappenden Intervall befindet, wobei das i-te einzelne Molekül den sich in dem überlappenden Intervall befindlichen Siedepunkt aufweist, $T_i$ der Siedepunkt des i-ten einzelnen Moleküls ist, $T_{\min}$ der Mindestwert des überlappenden Intervalls ist, und $C^i$ der Gehalt des i-ten einzelnen Moleküls in allen Molekülen ist, wobei sich ein Siedepunkt in dem überlappenden Intervall befindet, wobei das i-te einzelne Molekül den sich in dem überlappenden Intervall befindlichen Siedepunkt aufweist.

26. System zum Erhalten eines Produktions- und Verarbeitungsschemas von Erdölraffination, umfassend einen Prozessor, eine Kommunikationsschnittstelle, einen Speicher und einen Kommunikationsbus, wobei der Prozessor, die Kommunikationsschnittstelle und der Speicher in Kommunikation miteinander über den Kommunikationsbus sind;

der Speicher konfiguriert ist, um ein Computerprogramm zu speichern; und
der Prozessor konfiguriert ist, um das Verfahren nach einem der Ansprüche 1-24 durchzuführen, wenn das Programm, das in dem Speicher gespeichert ist, ausgeführt wird.

27. Computerlesbares Speichermedium, wobei das computerlesbaren Speichermedium ein oder mehrere darauf gespeicherte Programme aufweist, wobei das eine oder die mehreren Programme durch einen oder mehrere Prozessoren ausführbar sind, um das Verfahren nach einem der Ansprüche 1-24 zu implementieren.

**Revendications**

1. Procédé mis en œuvre par ordinateur permettant d'obtenir un schéma de production et de traitement de traitement de raffinerie de pétrole, dans lequel le procédé comprend :

l'acquisition de la composition moléculaire du pétrole brut ;
l'acquisition de la composition moléculaire de diverses fractions obtenues par distillation du pétrole brut en fonction des propriétés physiques de diverses molécules uniques dans la composition moléculaire du pétrole brut ;
l'entrée respective, en fonction d'un rapport de charge prédéfini, de la composition moléculaire des fractions correspondantes dans un modèle de prédiction de produit d'un dispositif de traitement pétrolier respectif en tant que charges de traitement pétrolier, pour obtenir la composition moléculaire d'un produit prédit correspondant et la teneur de chaque molécule unique dans le produit prédit ;
le mélange de chacun des produits prédits qui est utilisé en tant que charge de mélange de produits en fonction d'un ensemble de règles prédéfinies, pour obtenir la composition moléculaire d'une pluralité de produits mixtes et la teneur de chaque molécule unique dans chacun des produits mixtes, dans lequel chaque ensemble de règles prédéfinies dans l'ensemble de règles prédéfinies comprend le type et la quantité du produit prédit utilisé ;
le calcul respectif d'une propriété de produit de chacun des produits mixtes selon la composition moléculaire de chacun des produits mixtes et la teneur de chaque molécule unique dans chacun des produits mixtes ; et la détermination si la propriété de produit de chacun des produits mixtes respecte une quelconque norme prédéfinie dans un ensemble de normes prédéfinies ;
si la propriété de produit de chacun des produits mixtes respecte une quelconque norme prédéfinie dans l'ensemble de normes prédéfinies, l'acquisition d'un paramètre cible en fonction de tous les produits mixtes et la détermination si le paramètre cible respecte une condition prédéfinie ; le paramètre cible représente : une teneur en substances dans le produit qui causera des dommages à l'environnement ; ou une proportion de produits, qui respectent une norme prédéfinie, dans tous les produits mixtes par rapport à tous les produits mixtes ; et
si le paramètre cible ne respecte pas la condition prédéfinie, l'ajustement du rapport de charge prédéfini, d'un paramètre de fonctionnement dans le modèle de prédiction de produit et d'une règle prédéfinie dans l'ensemble de règles prédéfinies, pour ré-obtenir une pluralité de produits mixtes jusqu'à ce que la propriété de produit de chacun des produits mixtes respecte une quelconque norme prédéfinie dans l'ensemble de normes prédéfinies et que le paramètre cible respecte la condition prédéfinie, les paramètres de fonctionnement dans le modèle de prédiction de produit comprenant la pression, la température et la vitesse spatiale ;
si le paramètre cible respecte une condition prédéfinie, le rapport de charge prédéfini, le modèle de prédiction de produit et l'ensemble de règles prédéfinies sont sortis en tant que schéma de production et de traitement, et l'utilisation du schéma de production et de traitement pour effectuer le traitement de raffinerie de pétrole dans un processus de production réel ;
dans lequel l'acquisition de la composition moléculaire de diverses fractions obtenues par distillation du pétrole brut en fonction des propriétés physiques de diverses molécules uniques dans la composition moléculaire du pétrole brut comprend :

l'acquisition de chaque molécule unique dans le pétrole brut et de la teneur de chaque molécule unique ;
le calcul d'un point d'ébullition de chaque molécule unique, respectivement ; et
la coupe du pétrole brut par distillation selon une plage de distillation fractionnée prédéfinie pour obtenir des fractions multiples, et la détermination d'une molécule unique et de la teneur en chaque molécule unique contenue dans chacune des fractions en fonction du point d'ébullition et de la teneur en chaque molécule unique dans le pétrole brut ;

le procédé comprend en outre :

pour deux fractions ayant des plages de distillation adjacentes, l'adoption de la fraction ayant une température relativement élevée dans la plage de distillation en tant que première fraction, et l'adoption de la fraction ayant une température relativement basse dans la plage de distillation en tant que seconde fraction ;

le calcul d'une valeur minimale d'un intervalle de chevauchement d'une plage de distillation en chevauchement par la première fraction et la seconde fraction par la formule suivante :

$$T_{\min} = T_{\mathrm{cut}} \times (1 - SF)$$ ;

et

le calcul d'une valeur maximale de l'intervalle de chevauchement de la plage de distillation en chevauchement par la première fraction et la seconde fraction par la formule suivante :

$$T_{\max} = T_{\mathrm{cut}} \times (1 + SF)$$

où, $T_{\min}$ est la valeur minimale de l'intervalle de chevauchement, $T_{\max}$ est la valeur maximale de l'intervalle de chevauchement, $T_{\mathrm{cut}}$ est la température de coupe de distillation de la première fraction et de la seconde fraction, et $SF$ est un indice de séparation de la première fraction et de la seconde fraction ;

le procédé comprend en outre :

le calcul de la teneur en partie distillée dans la première fraction de chaque molécule unique dans l'intervalle de chevauchement et le calcul de la teneur en partie distillée dans la seconde fraction de chaque molécule unique dans l'intervalle de chevauchement en fonction de la teneur en chaque molécule unique et chaque molécule unique correspondant à chaque point d'ébullition de l'intervalle de chevauchement ;

dans lequel la teneur en partie distillée dans la première fraction de chaque molécule unique dans l'intervalle de chevauchement et la teneur en partie distillée dans la seconde fraction de chaque molécule unique dans l'intervalle de chevauchement sont calculées par l'équation suivante :

$$C_h^i = \ln\ (\frac{T_i}{T_{\min}}) \times C^i$$ ;

$$C_l^i = C^i - C_h^i$$ ;

où, $C_h^i$ est la teneur en partie distillée dans la première fraction de la i-ième molécule unique dans toutes les molécules avec un point d'ébullition situé dans l'intervalle de chevauchement, laquelle i-ième molécule unique a le point d'ébullition situé dans l'intervalle de chevauchement, $C_l^i$ est la teneur en partie distillée dans la première fraction de la i-ième molécule unique dans toutes les molécules avec un point d'ébullition situé dans l'intervalle de chevauchement, laquelle i-ième molécule unique a le point d'ébullition situé dans l'intervalle de chevauchement, $T_i$ est le point d'ébullition de la i-ième molécule unique, $T_{\min}$ est la valeur minimale de l'intervalle de chevauchement, et $C^i$ est la teneur de la i-ième molécule unique dans toutes les molécules avec un point d'ébullition situé dans l'intervalle de chevauchement, laquelle i-ième molécule unique a le point d'ébullition situé dans l'intervalle de chevauchement ; et

l'obtention de la teneur en chaque molécule unique et chaque molécule unique dans chacune de la première fraction et de la seconde fraction après que le pétrole brut est coupé par distillation en fonction de la teneur en partie distillée dans la première fraction de chaque molécule unique dans l'intervalle de chevauchement et de la teneur en partie distillée dans la seconde fraction de chaque molécule unique dans l'intervalle de chevauchement.

**2.** Procédé selon la revendication 1, dans lequel le procédé comprend en outre :

l'acquisition d'un flux d'entrée de charges de traitement pétrolier entré dans chacun des dispositifs de traitement pétrolier ;

la détermination pour savoir si chacun des flux d'entrée respecte une plage de flux d'entrée prédéfinie du dispositif de traitement pétrolier respectif ; et

l'ajustement du rapport de charge prédéfini si l'un quelconque des flux d'entrée ne respecte pas la plage de flux d'entrée prédéfinie du dispositif de traitement pétrolier respectif, et respectivement la ré-entrée, en fonction du rapport de charge prédéfini ajusté, des fractions correspondantes dans le modèle de prédiction de produit du dispositif de traitement pétrolier respectif en tant que charges de traitement pétrolier, jusqu'à ce que chacun des flux d'entrée respecte la plage de flux d'entrée prédéfinie du dispositif de traitement pétrolier respectif.

**3.** Procédé selon la revendication 1, dans lequel le procédé comprend en outre :

l'acquisition de la composition moléculaire des charges de traitement pétrolier entrées dans chacun des dispositifs de traitement pétrolier et de la teneur en chaque molécule unique dans les charges de traitement pétrolier ;

le calcul d'une propriété physique de chaque molécule unique dans les charges de traitement pétrolier, le calcul d'une propriété de charge des charges de traitement pétrolier en fonction de la propriété physique de chaque molécule unique et de la teneur en chaque molécule unique dans les charges de traitement pétrolier ;

la détermination pour savoir si chacune des propriétés de charge respecte un intervalle de restriction de propriété physique prédéfini du dispositif de traitement pétrolier respectif ; et

si l'une quelconque des propriétés de charge ne respecte pas l'intervalle de restriction de propriété physique prédéfini du dispositif de traitement pétrolier respectif, l'ajustement du rapport de charge prédéfini, et respectivement la ré-entrée, en fonction du rapport de charge prédéfini ajusté, des fractions correspondantes dans le modèle de prédiction de produit du dispositif de traitement pétrolier respectif en tant que charges de traitement pétrolier, jusqu'à ce que chacune des propriétés de charge respecte l'intervalle de restriction de propriété physique prédéfini du dispositif de traitement pétrolier respectif.

**4.** Procédé selon la revendication 1, dans lequel le procédé comprend en outre :

si la propriété de produit d'un quelconque produit mixte ne respecte aucune norme prédéfinie dans l'ensemble de normes prédéfinies, l'ajustement de la règle prédéfinie dans l'ensemble de règles prédéfinies et le mélange de chacune des charges de mélange de produits en fonction de l'ensemble de règles prédéfinies ajusté, pour ré-obtenir une pluralité de produits mixtes jusqu'à ce que la propriété de produit de chacun des produits mixtes respecte une quelconque norme prédéfinie dans l'ensemble de normes prédéfinies.

**5.** Procédé selon la revendication 1, dans lequel le calcul respectif d'une propriété de produit de chacun des produits mixtes en fonction de la composition moléculaire de chacun des produits mixtes et de la teneur en chaque molécule unique dans chacun des produits mixtes comprend :

l'acquisition de la première composition moléculaire de chaque ensemble des charges de mélange de produits et de la première teneur en composants de chaque molécule unique dans chaque ensemble des charges de mélange de produits ;

sur la base de l'ensemble de règles prédéfinies, l'obtention d'une seconde composition moléculaire de chacun des produits mixtes et d'une seconde teneur en composant de chaque molécule unique dans chacun des produits mixtes en fonction de la première composition moléculaire de chaque ensemble de la charge de mélange de produits et de la première teneur en composant de chaque molécule unique dans chaque ensemble des charges de mélange de produits ;

le calcul d'une propriété physique de chaque molécule unique dans chacun des produits mixtes en fonction du nombre de groupes de chaque groupe contenu dans chaque molécule unique dans chacun des produits mixtes et d'une valeur de contribution de chaque groupe à la propriété physique ; et

le calcul d'une propriété de produit de chacun des produits mixtes en fonction de la propriété physique et de la seconde teneur en composant de chaque molécule unique dans chacun des produits mixtes.

**6.** Procédé selon la revendication 5, dans lequel le calcul de la propriété physique de chaque molécule unique comprend :

pour chaque molécule unique, l'acquisition du nombre de groupes de chaque groupe constituant la molécule unique et d'une valeur de contribution de chaque groupe à la propriété physique ; et

l'entrée du nombre de groupes de chaque groupe constituant la molécule unique et de la valeur de contribution de chaque groupe à la propriété physique dans un modèle de calcul de propriété pré-entraîné, pour acquérir la propriété physique de la molécule unique sortie par le modèle de calcul de propriété pré-entraîné.

**7.** Procédé selon la revendication 6, dans lequel, avant l'entrée du nombre de groupes de chaque groupe constituant la molécule unique et de la valeur de contribution de chaque groupe à la propriété physique dans un modèle de calcul de propriété pré-entraîné, le procédé comprend en outre :

la comparaison du nombre de groupes de chaque groupe constituant la molécule unique à des informations moléculaires d'une molécule unique modèle avec des propriétés physiques connues pré-stockées dans une base de données, les informations moléculaires comprenant le nombre de groupes de chaque groupe constituant la molécule unique modèle ;

la détermination s'il existe une même molécule unique modèle que la molécule unique ;

s'il existe une même molécule unique modèle que la molécule unique, la sortie des propriétés physiques de la molécule unique modèle en tant que propriété physique de la molécule unique ; et

s'il n'existe pas de même molécule unique modèle que la molécule unique, alors l'exécution de l'étape d'entrée du nombre de groupes de chaque groupe constituant la molécule unique et de la valeur de contribution de chaque groupe à la propriété physique dans un modèle de calcul de propriété pré-entraîné.

**8.** Procédé selon la revendication 1, dans lequel le calcul d'un point d'ébullition de chaque molécule unique comprend :

pour chaque molécule unique, l'acquisition du nombre de groupes de chaque groupe constituant la molécule unique et d'une valeur de contribution de chaque groupe au point d'ébullition ; et

l'entrée du nombre de groupes de chaque groupe constituant la molécule unique et de la valeur de contribution de chaque groupe au point d'ébullition dans un modèle de calcul de propriété pré-entraîné, pour acquérir le point d'ébullition de la molécule unique sortie par le modèle de calcul de propriété pré-entraîné.

**9.** Procédé selon la revendication 8, dans lequel, avant l'entrée du nombre de groupes de chaque groupe constituant la molécule unique et de la valeur de contribution de chaque groupe au point d'ébullition dans un modèle de calcul de propriété pré-entraîné, le procédé comprend en outre :

la comparaison du nombre de groupes de chaque groupe constituant la molécule unique à des informations moléculaires d'une molécule unique modèle avec un point d'ébullition connu pré-stocké dans une base de données, les informations moléculaires comprenant le nombre de groupes de chaque groupe constituant la molécule unique modèle ;

la détermination s'il existe une même molécule unique modèle que la molécule unique ;

s'il existe une même molécule unique modèle que la molécule unique, la sortie du point d'ébullition de la molécule unique modèle en tant que point d'ébullition de la molécule unique ; et

s'il n'existe pas de même molécule unique modèle que la molécule unique, alors l'exécution de l'étape d'entrée du nombre de groupes de chaque groupe constituant la molécule unique et de la valeur de contribution de chaque groupe au point d'ébullition dans un modèle de calcul de propriété pré-entraîné.

**10.** Procédé selon la revendication 6 ou 8, dans lequel une étape d'entraînement du modèle de calcul de propriété comprend :

la construction d'un modèle de calcul de propriété d'une molécule unique ;

l'acquisition du nombre de groupes de chaque groupe constituant une molécule unique échantillon ; dans lequel la propriété physique de la molécule unique échantillon est connue ;

l'entrée du nombre de groupes de chaque groupe constituant la molécule unique échantillon dans le modèle de calcul de propriété ;

l'acquisition d'une propriété physique prédite de la molécule unique échantillon sortie par le modèle de calcul de propriété ;

si une valeur d'écart entre la propriété physique prédite et la propriété physique qui est connue est inférieure à un seuil d'écart prédéfini, la détermination que le modèle de calcul de propriété converge, l'acquisition d'une valeur de contribution correspondant à chaque groupe dans le modèle de calcul de propriété qui est convergé, et le stockage de la valeur de contribution en tant que valeur de contribution du groupe à la propriété physique ; et

si la valeur d'écart entre la propriété physique prédite et la propriété physique qui est connue est supérieure ou égale au seuil d'écart, l'ajustement d'une valeur de contribution correspondant à chaque groupe dans le modèle de calcul de propriété jusqu'à ce que le modèle de calcul de propriété converge.

**11.** Procédé selon la revendication 10, dans lequel le modèle de calcul de propriété est établi comme indiqué ci-dessous :

$$f = a + \sum_i n_i \Delta f_i \;;$$

où, $f$ est la propriété physique de la molécule unique, $n_i$ est le nombre de groupes du *i-ième* groupe, $\Delta f_i$ est la valeur de contribution du *i-ième* groupe à la propriété physique,
et $\alpha$ est une constante associée.

**12.** Procédé selon la revendication 10, dans lequel l'acquisition du nombre de groupes de chaque groupe constituant une molécule unique échantillon comprend :

la détermination d'un groupe primaire, du nombre de groupes du groupe primaire, d'un groupe à étages multiples et du nombre de groupes du groupe à étages multiples dans tous les groupes de la molécule unique ;
l'adoption de tous les groupes constituant la molécule unique en tant que groupe principal ; et
l'adoption de divers groupes qui coexistent et contribuent à une même propriété physique en commun en tant que groupe à étages multiples, et l'adoption du nombre des divers groupes en tant que niveau du groupe à étages multiples.

**13.** Procédé selon la revendication 12, dans lequel le modèle de calcul de propriété est établi comme indiqué ci-dessous :

$$f = a + \sum_i m_{1i} \Delta f_{1i} + \sum_j m_{2j} \Delta f_{2j} \ldots\ldots + \sum_l m_{Nl} \Delta f_{Nl}$$

où, $f$ est la propriété physique de la molécule unique, $m_{1i}$ est le nombre de groupes du *i-ième* groupe dans le groupe primaire, $\Delta f_{1i}$ est la valeur de contribution du *i-ième* groupe dans le groupe primaire à la propriété physique, $m_{2j}$ est le nombre de groupes du j-ième groupe dans un groupe secondaire, $\Delta f_{2j}$ est la valeur de contribution du j-ième groupe dans le groupe secondaire à la propriété physique, $m_{Nl}$ est le nombre de groupes du /-ième groupe dans un groupe à N étages, $\Delta f_{Nl}$ est la valeur de contribution du /-ième groupe dans le groupe à N étages à la propriété physique, $\alpha$ est une constante associée, et *N* est un entier positif supérieur ou égal à 2.

**14.** Procédé selon la revendication 8, dans lequel l'acquisition du nombre de groupes de chaque groupe constituant la molécule unique comprend :

la détermination d'un groupe primaire, du nombre de groupes du groupe primaire, d'un groupe à étages multiples et du nombre de groupes du groupe à étages multiples dans tous les groupes de la molécule unique ;
l'adoption de tous les groupes constituant la molécule unique en tant que groupe primaire ; et
l'adoption de divers groupes qui coexistent et contribuent à une même propriété physique en commun en tant que groupe à étages multiples, et l'adoption du nombre des divers groupes en tant que niveau du groupe à étages multiples.

**15.** Procédé selon la revendication 14, dans lequel,
l'entrée du nombre de groupes de chaque groupe constituant la molécule unique et de la valeur de contribution de chaque groupe au point d'ébullition dans un modèle de calcul de propriété pré-entraîné, pour acquérir le point d'ébullition de la molécule unique sortie par le modèle de calcul de propriété pré-entraîné comprend :

le calcul du point d'ébullition de la molécule unique en fonction du modèle de calcul de propriété suivant :

$$T = \frac{SOL \times GROUPE_1 + SOL \times GROUPE_2 + \ldots\ldots + SOL \times GROUPE_N}{(SOL \times N\text{umh})^d + b} + c \;;$$

où, *T* est le point d'ébullition de la molécule unique, *SOL* est un vecteur de molécule unique converti en fonction du nombre de groupes de chaque groupe constituant la molécule unique, $GROUPE_{11}$ est un premier vecteur de valeur de contribution converti en fonction d'une valeur de contribution du groupe primaire au point d'ébullition, $GROUPE_{12}$ est un second vecteur de valeur de contribution converti en fonction d'une valeur de contribution du groupe secondaire au point d'ébullition, $GROUPE_{1N}$ est un N-ième vecteur de valeur de contribution converti en fonction d'une valeur de contribution du groupe à N étages au point d'ébullition, Numh est le nombre d'atomes autres que l'atome d'hydrogène dans la molécule unique, d est une première constante prédéfinie, $b$ est une deuxième constante prédéfinie, c est une troisième constante prédéfinie, et $N$ est un entier positif supérieur ou égal à 2.

16. Procédé selon la revendication 15, dans lequel,
la conversion du vecteur de molécule unique en fonction du nombre de groupes de chaque groupe constituant la molécule unique comprend :

l'adoption du nombre d'espèces de groupes en tant que dimension du vecteur à molécule unique ; et
l'adoption du nombre de groupes de chaque groupe en tant que valeur d'élément de la dimension correspondante dans le vecteur moléculaire unique,
la conversion du premier vecteur de valeur de contribution en fonction d'une valeur de contribution du groupe primaire au point d'ébullition comprend :

l'adoption du nombre de types de groupes primaires en tant que dimension du premier vecteur de valeur de contribution ; et
l'adoption de la valeur de contribution de chaque groupe primaire au point d'ébullition en tant que valeur d'élément de la dimension correspondante dans le premier vecteur de valeur de contribution,
la conversion du second vecteur de valeur de contribution en fonction d'une valeur de contribution du groupe secondaire au point d'ébullition comprend :

l'adoption du nombre de types de groupes secondaires en tant que dimension du second vecteur de valeur de contribution ; et
l'adoption de la valeur de contribution de chaque groupe secondaire au point d'ébullition en tant que valeur d'élément de la dimension correspondante dans le second vecteur de valeur de contribution,
la conversion du N-ième vecteur de valeur de contribution en fonction d'une valeur de contribution de chaque groupe à N étages au point d'ébullition comprend :

l'adoption du nombre de types de groupes à N étages en tant que dimension du N-ième vecteur de valeur de contribution ; et
l'adoption de la valeur de contribution de chaque groupe à N étages au point d'ébullition en tant que valeur d'élément de la dimension correspondante dans le N-ième vecteur de valeur de contribution.

17. Procédé selon la revendication 1, dans lequel l'entrée respective, en fonction d'un rapport de charge prédéfini, des fractions correspondantes dans un modèle de prédiction de produit d'un dispositif de traitement pétrolier respectif comprend :

l'obtention de différentes quantités de chaque fraction en fonction du rapport de charge prédéfini, et l'entrée respective de chaque fraction dans le modèle de prédiction de produit du dispositif de traitement pétrolier respectif,
le dispositif de traitement pétrolier comprend une unité de craquage catalytique, une unité de cokéfaction retardée, une unité d'hydrotraitement des résidus, une unité d'hydrocraquage, une unité d'hydrogradation du diesel, une unité d'hydro-raffinage du diesel, une unité d'hydro-raffinage de l'essence, une unité de reformage catalytique et une unité d'alkylation.

18. Procédé selon la revendication 17, dans lequel une étape d'entraînement du modèle de prédiction de produit comprend :

l'établissement d'un modèle de prédiction de produit ; dans lequel le modèle de prédiction de produit comprend :
un ensemble de règles de réaction comprenant une pluralité de règles de réaction et un algorithme de vitesse de réaction ;
l'acquisition d'informations de charge échantillon pour une charge échantillon ;
l'entraînement de l'ensemble de règles de réaction en utilisant les informations de charge échantillon et la fixation de l'ensemble des règles de réaction qui ont été entraînées ; et

l'entraînement de l'algorithme de vitesse de réaction en utilisant les informations de charge échantillon et la fixation de l'algorithme de vitesse de réaction qui a été entraîné, pour obtenir le modèle de prédiction de produit qui a été entraîné.

19. Procédé selon la revendication 18, dans lequel les informations de charge échantillon de la charge échantillon comprennent : la composition moléculaire de la charge échantillon, la teneur moléculaire de chaque molécule dans la charge échantillon, la composition moléculaire d'un produit réel correspondant à la charge échantillon, et la teneur réelle en chaque molécule dans le produit réel.

20. Procédé selon la revendication 19, dans lequel l'entraînement de l'ensemble de règles de réaction en utilisant les informations de charge échantillon comprend :

le traitement de la composition moléculaire de la charge échantillon en fonction d'un ensemble prédéfini de règles de réaction, pour obtenir un chemin de réaction correspondant à chaque molécule dans la composition moléculaire de la charge échantillon ;

l'obtention de la première composition moléculaire d'un produit de sortie de dispositif comprenant la charge échantillon, un produit intermédiaire et un produit prédit en fonction du chemin de réaction correspondant à chaque molécule dans la composition moléculaire de la charge échantillon ; dans le produit de sortie de dispositif, comprenant : la charge échantillon, le produit intermédiaire et le produit prédit ;

le calcul d'un premier écart relatif en fonction de la première composition moléculaire du produit de sortie de dispositif et de la seconde composition moléculaire du produit réel ;

si le premier écart relatif respecte une condition prédéfinie, la fixation de l'ensemble de règles de réaction ; et

si le premier écart relatif ne respecte pas la condition prédéfinie, l'ajustement d'une règle de réaction dans l'ensemble de règles de réaction, et le recalcul du premier écart relatif en fonction de l'ensemble ajusté de règles de réaction jusqu'à ce que le premier écart relatif respecte la condition prédéfinie.

21. Procédé selon la revendication 20, dans lequel le calcul d'un premier écart relatif en fonction de la première composition moléculaire du produit de sortie de dispositif et de la seconde composition moléculaire du produit réel comprend :

l'acquisition d'espèces de molécules uniques dans la première composition moléculaire, pour constituer un premier ensemble

l'acquisition d'espèces de molécules uniques dans la seconde composition moléculaire, pour constituer un second ensemble ;

la détermination si le second ensemble est un sous-ensemble du premier ensemble ;

si le second ensemble n'est pas un sous-ensemble du premier ensemble, l'obtention d'une valeur d'écart relatif pré-stockée qui ne respecte pas la condition prédéfinie en tant que premier écart relatif ; et

si le second ensemble est un sous-ensemble du premier ensemble, le calcul du premier écart relatif par une formule de calcul comme suit :

$$x_1 = \frac{\mathrm{card}\big((M - M_1 - M_2) - M_3\big)}{\mathrm{card}\ (M - M_1 - M_2)} ;$$

où, $x_1$ est le premier écart relatif, $M$ est le premier ensemble, $M_1$ est un ensemble d'espèces de molécules uniques dans la composition moléculaire de la charge échantillon, $M_2$ est un ensemble d'espèces de molécules uniques dans la composition moléculaire du produit intermédiaire, $M_3$ est le second ensemble, et card représente le nombre d'éléments dans les ensembles.

22. Procédé selon la revendication 19, dans lequel l'entraînement de l'algorithme de vitesse de réaction en utilisant les informations de charge échantillon comprend :

le calcul d'une vitesse de réaction d'un chemin de réaction correspondant à chaque molécule dans la composition moléculaire de la charge échantillon, respectivement, en fonction de l'algorithme de vitesse de réaction ;

l'obtention de la teneur prédite de chaque molécule dans un produit prédit correspondant à la charge échantillon en fonction de la teneur moléculaire de chaque molécule dans la charge échantillon et la vitesse de réaction du chemin de réaction correspondant à la molécule ;

le calcul d'un second écart relatif en fonction de la teneur prédite de chaque molécule dans le produit prédit et la teneur réelle de chaque molécule dans le produit réel ;

si le premier écart relatif respecte une condition prédéfinie, la fixation de l'algorithme de vitesse de réaction ; et si le second écart relatif ne respecte pas la condition prédéfinie, l'ajustement d'un paramètre dans l'algorithme de vitesse de réaction, et le recalcul du second écart relatif en fonction de l'algorithme de vitesse de réaction ajusté jusqu'à ce que le second écart relatif respecte la condition prédéfinie.

23. Procédé selon la revendication 22, dans lequel le calcul d'une vitesse de réaction d'un chemin de réaction correspondant à chaque molécule dans la composition moléculaire de la charge échantillon, respectivement, selon l'algorithme de vitesse de réaction comprend :

le calcul d'une vitesse de réaction de chaque chemin de réaction selon une constante de vitesse de réaction dans l'algorithme de vitesse de réaction ;

dans lequel la constante de vitesse de réaction est déterminée en fonction d'une formule de calcul comme suit :

$$k = \frac{k_B E}{h} \exp(\frac{E\Delta S - \Delta E}{RE})\varphi \times P^{\alpha} ;$$

où, $k$ est la constante de vitesse de réaction, $k_B$ est la constante de Boltzmann, $h$ est la constante de Planck, R est une constante de gaz idéale, E est une valeur de température de l'environnement au niveau duquel le chemin de réaction se trouve, exp est une fonction exponentielle avec une base de constante naturelle, $\Delta S$ est un changement d'entropie avant et après la réaction correspondant à la règle de réaction correspondant au chemin de réaction, $\Delta E$ est une barrière d'énergie de réaction correspondant à la règle de réaction correspondant au chemin de réaction, $\varphi$ est un facteur d'activité de catalyseur, $P$ est une valeur de pression de l'environnement au niveau duquel le chemin de réaction se trouve, et $\alpha$ est un facteur influençant la pression correspondant à la règle de réaction correspondant au chemin de réaction.

24. Procédé selon l'une quelconque des revendications 17 à 23, dans lequel chaque dispositif de traitement pétrolier correspond à un ensemble de règles de réaction.

25. Appareil pour l'obtention d'un schéma de production et de traitement de traitement de raffinerie de pétrole, dans lequel l'appareil comprenant :

une unité d'acquisition configurée pour acquérir la composition moléculaire du pétrole brut ;

une première unité de traitement configurée pour acquérir la composition moléculaire de diverses fractions obtenues par distillation du pétrole brut en fonction des propriétés physiques de diverses molécules uniques dans la composition moléculaire du pétrole brut, et délivrer les fractions correspondantes en tant que charges de traitement pétrolier, respectivement entrées, en fonction d'un rapport de charge prédéfini, la composition moléculaire des fractions correspondantes dans un modèle de prédiction de produit d'un dispositif de traitement pétrolier respectif, pour obtenir la composition moléculaire d'un produit prédit correspondant et la teneur de chaque molécule unique dans le produit prédit ;

une deuxième unité de traitement configurée pour mélanger chacun des produits prédits qui est utilisé en tant que charge de mélange de produits en fonction d'un ensemble de règles prédéfinies, pour obtenir la composition moléculaire d'une pluralité de produits mixtes et la teneur en chaque molécule unique dans chacun des produits mixtes, dans lequel chaque ensemble de règles prédéfinies dans l'ensemble de règles prédéfinies comprend le type et la quantité du produit prédit utilisé ;

une troisième unité de traitement configurée pour calculer respectivement une propriété de produit de chacun des produits mixtes en fonction de la composition moléculaire de chacun des produits mixtes et de la teneur en chaque molécule unique dans chacun des produits mixtes ; et déterminer si la propriété de produit de chacun des produits mixtes respecte une quelconque norme prédéfinie dans un ensemble de normes prédéfinies ; et

une quatrième unité de traitement configurée pour, si la propriété de produit de chacun des produits mixtes respecte une quelconque norme prédéfinie dans l'ensemble de normes prédéfinies, acquérir un paramètre cible selon tous les produits mixtes et déterminer si le paramètre cible respecte une condition prédéfinie, et, si le paramètre cible ne respecte pas la condition prédéfinie, ajuster le rapport de charge prédéfini, un paramètre de fonctionnement dans le modèle de prédiction de produit et une règle prédéfinie dans l'ensemble de règles prédéfinies, pour ré-obtenir une pluralité de produits mixtes jusqu'à ce que la propriété de produit de chacun des produits mixtes respecte une quelconque norme prédéfinie dans l'ensemble de normes prédéfinies et que le paramètre cible respecte la condition prédéfinie ;

si le paramètre cible respecte une condition prédéfinie, sortir le rapport de charge prédéfini, le modèle de prédiction de produit et l'ensemble de règles prédéfinies en tant que schéma de production et de traitement, et utiliser le schéma de production et de traitement pour effectuer le traitement de raffinerie de pétrole dans un processus de production réel ;

le paramètre cible est une teneur en substances dans le produit qui causera des dommages à l'environnement, ou une proportion de produits, qui respecte une norme prédéfinie, dans tous les produits mixtes par rapport à tous les produits mixtes, les paramètres de fonctionnement dans le modèle de prédiction de produit comprenant la pression, la température et la vitesse spatiale ;

la première unité de traitement est, en particulier, configurée pour acquérir chaque molécule unique dans le pétrole brut et la teneur en chaque molécule unique, calculer un point d'ébullition de chaque molécule unique, respectivement, couper le pétrole brut par distillation en fonction d'une plage de distillation fractionnée prédéfinie pour obtenir des fractions multiples, et déterminer une molécule unique et la teneur de la molécule unique contenue dans chacune des fractions en fonction du point d'ébullition et de la teneur en chaque molécule unique dans le pétrole brut ;

la première unité de traitement est en outre configurée pour, pour deux fractions avec des plages de distillation adjacentes, adopter la fraction avec une température relativement élevée dans la plage de distillation en tant que première fraction, et adopter la fraction avec une température relativement basse dans la plage de distillation en tant que seconde fraction ;

calculer une valeur minimale d'un intervalle de chevauchement d'une plage de distillation en chevauchement par la première fraction et la seconde fraction par la formule suivante :

$$T_{\min} = T_{\text{cut}} \times (1 - SF)$$
;

et

calculer une valeur maximale de l'intervalle de chevauchement de la plage de distillation en chevauchement par la première fraction et la seconde fraction par la formule suivante :

$$T_{\max} = T_{\text{cut}} \times (1 + SF)$$
;

où, $T_{\min}$ est la valeur minimale de l'intervalle de chevauchement, $T_{\max}$ est la valeur maximale de l'intervalle de chevauchement, $T_{\text{cut}}$ est la température de coupe de distillation de la première fraction et de la seconde fraction, et $SF$ est un indice de séparation de la première fraction et de la seconde fraction ;

la première unité de traitement est en outre configurée pour calculer la teneur en partie distillée dans la première fraction de chaque molécule unique dans l'intervalle de chevauchement et pour calculer la teneur en partie distillée dans la seconde fraction de chaque molécule unique dans l'intervalle de chevauchement en fonction de la teneur en chaque molécule unique et en chaque molécule unique correspondant à chaque point d'ébullition de l'intervalle de chevauchement, et obtenir la teneur en chaque molécule unique et en chaque molécule unique dans chacune de la première fraction et de la seconde fraction après que le pétrole brut est coupé par distillation en fonction de la teneur en partie distillée dans la première fraction de chaque molécule unique dans l'intervalle de chevauchement et de la teneur en partie distillée dans la seconde fraction de chaque molécule unique dans l'intervalle de chevauchement,

dans lequel la teneur en partie distillée dans la première fraction de chaque molécule unique dans l'intervalle de chevauchement et la teneur en partie distillée dans la seconde fraction de chaque molécule unique dans l'intervalle de chevauchement sont calculées par l'équation suivante :

$$C_h^i = \ln \left( \frac{T_i}{T_{\min}} \right) \times C^i$$
;

$$C_l^i = C^i - C_h^i$$
;

où, $C_h^i$ est la teneur en partie distillée dans la première fraction de la i-ième molécule unique dans toutes les molécules avec un point d'ébullition situé dans l'intervalle de chevauchement, laquelle i-ième molécule unique a le point d'ébullition situé dans l'intervalle de chevauchement, $C_i^i$ est la teneur en partie distillée dans la première fraction de la i-ième molécule unique dans toutes les molécules avec un point d'ébullition situé dans l'intervalle de chevauchement, laquelle i-ième molécule unique a le point d'ébullition situé dans l'intervalle de chevauchement, $T_i$ est le point d'ébullition de la i-ième molécule unique, $T_{min}$ est la valeur minimale de l'intervalle de chevauchement, et $C^i$ est la teneur en i-ième molécule unique dans toutes les molécules avec un point d'ébullition situé dans l'intervalle de chevauchement, laquelle i-ième molécule unique a le point d'ébullition situé dans l'intervalle de chevauchement.

26. Système pour l'obtention d'un schéma de production et de traitement de raffinerie de pétrole comprenant un processeur, une interface de communication, une mémoire et un bus de communication, dans lequel le processeur, l'interface de communication et la mémoire sont en communication les uns avec les autres via le bus de communication ;

la mémoire est configurée pour stocker un programme informatique ; et
le processeur est configuré pour exécuter le procédé selon l'une quelconque des revendications 1 à 24 lors de l'exécution du programme stocké dans la mémoire.

27. Support de stockage lisible par ordinateur, dans lequel le support de stockage lisible par ordinateur possède dans celui-ci un ou plusieurs programmes, l'un ou plusieurs programmes étant exécutables par un ou plusieurs processeurs pour mettre en œuvre le procédé selon l'une quelconque des revendications 1 à 24.

```
┌─────────────────────────────────────────────────────────┐
│        molecular composition of crude oil is acquired     │
└─────────────────────────────────────────────────────────┘
                                                              S11
┌─────────────────────────────────────────────────────────┐
│ molecular composition of various fractions obtained by distillation of │
│  the crude oil is acquired according to physical properties of various  │
│     single molecules in the molecular composition of the crude oil      │
└─────────────────────────────────────────────────────────┘
                                                              S12
┌─────────────────────────────────────────────────────────┐
│ according to a preset feedstock ratio, the corresponding fractions are  │
│ respectively inputted into a product prediction model of a respective   │
│ petroleum processing device as petroleum processing feedstocks, to      │
│ obtain molecular composition of a corresponding predicted product       │
│     and content of each single molecule in the predicted product        │
└─────────────────────────────────────────────────────────┘
                                                              S13
┌─────────────────────────────────────────────────────────┐
│ each of the predicted products which is used as a product blending      │
│     feedstock is blended according to a preset rule set, to obtain       │
│ molecular composition of a plurality of mixed products and content      │
│     of each single molecule in each of the mixed products               │
└─────────────────────────────────────────────────────────┘
                                                              S14
```

a product property of each of the mixed products is respectively calculated according to the molecular composition of each of the mixed products and the content of each single molecule in each of the mixed products; and it is determined whether the product property of each of the mixed products meets any preset standard in a preset standard set — S15

Yes

a target parameter is acquired according to all mixed products and it is determined whether the target parameter meets a preset condition — S16

No — Yes

```
┌─────────────────────────┐        ┌─────────────────────────┐
│ the preset feedstock     │ S17b   │  the preset feed ratio, the │ S17a
│ ratio, a parameter in    │        │ product prediction model, and│
│ the product prediction   │        │ the preset rule set is outputted│
│ model and a preset rule  │        │ as a production and processing │
│ in the preset rule set   │        │         scheme               │
│ are adjusted             │        │                             │
└─────────────────────────┘        └─────────────────────────┘
```

FIG. 1

a product price of each of mixed products and a yield of each of mixed products are acquired ⟋ S21

↓

a product benefit of each of mixed products is calculated according to the yield of each of mixed products and the product price of each of mixed products ⟋ S22

↓

the product benefit of each of mixed products is accumulated to obtain a cumulative benefit ⟋ S23

↓

a feedstock price of each group of the petroleum processing feedstocks and an operating cost of each of the petroleum processing devices are acquired ⟋ S24

↓

feedstock prices of all petroleum processing feedstocks and operating costs of all petroleum processing devices are subtracted from the cumulative benefit to obtain a comprehensive benefit ⟋ S25

↓

the comprehensive benefit is served as the target parameter and it is determined whether the comprehensive benefit reaches a maximum value ⟋ S26

No — Yes

the target parameter meets the preset condition ⟋ S27a

the target parameter does not meet the preset condition ⟋ S27b

FIG. 2

an input flow of petroleum processing feedstocks input to each of the petroleum processing devices is acquired ⟍ S31

↓

it is determined whether each of the input flows meets a preset input flow range of the respective petroleum processing device ⟋ S32

Yes — No

S33b molecular composition of a corresponding predicted product and content of each single molecule in the predicted product are obtained

according to the adjusted preset feedstock ratio, the corresponding fractions are respectively inputted into the product prediction model of the respective petroleum processing device as petroleum processing feedstocks, until each of the input flows meets the preset input flow range of the respective petroleum processing device ⟋ S33a

FIG. 3

the molecular composition of the petroleum processing feedstocks input to each of the petroleum processing devices and content of each single molecule in the petroleum processing feedstocks are acquired ⟩ S41

a physical property of each single molecule in the petroleum processing feedstocks is calculated, and a feedstock property of the petroleum processing feedstocks is calculated according to the physical property of each single molecule and the content of each single molecule in the petroleum processing feedstocks ⟩ S42

it is determined whether each of the feedstock properties meets a preset physical property restriction interval of the respective petroleum processing device ⟩ S43

—Yes—          —No—

S44b ⟩ molecular composition of a corresponding predicted product and content of each single molecule in the predicted product are obtained

according to the adjusted preset feedstock ratio, the corresponding fractions are respectively re-input into the product prediction model of the respective petroleum processing device as petroleum processing feedstocks, until each of the feedstock properties meets the preset physical property restriction interval of the respective petroleum processing device ⟩ S44a

FIG. 4

first molecular composition of each group of the product blending feedstocks and first component content of each single molecule in each group of the product blending feedstocks are acquired ⟩ S51

based on the preset rule set, second molecular composition of each of mixed products and second component content of each single molecule in each of mixed products are obtained according to the first molecular composition of each group of the product blending feedstock and the first component content of each single molecule in each group of the product blending feedstocks ⟩ S52

a physical property of each single molecule is calculated according to the number of groups of each group contained in each single molecule in each of the mixed products and a contribution value of each group to the physical property ⟩ S53

a product property of each of the mixed products is calculated according to the physical property and the second component content of each single molecule in each of the mixed products ⟩ S54

FIG. 5

each single molecule in the crude oil and the content of each single molecule are acquired — S61

a boiling point of each single molecule is calculated respectively — S62

the crude oil is cut by distillation according to a preset fractional distillation range to obtain multiple fractions, and a single molecule and content of the single molecule contained in each of the fractions are determined according to the boiling point and the content of each single molecule in the crude oil — S63

FIG. 6

for two fractions with adjacent distillation ranges, the fraction with a relatively high temperature in the distillation range is taken as a first fraction, and the fraction with a relatively low temperature in the distillation range is taken as a second fraction — S71

a minimum value of an overlapping interval of an overlapping distillation range of the first fraction and the second fraction is calculated; and a maximum value of an overlapping interval of an overlapping distillation range of the first fraction and the second fraction is calculated — S72

— S73

the overlapping interval is obtained according to the minimum and maximum values

FIG. 7

for each of the single molecule, the number of groups of each group constituting the single molecule and a contribution value of the each group to the boiling point are acquired — S81

the number of groups of each group constituting the single molecule and the contribution value of each group to the boiling point are input into a pre-trained property calculation model, to acquire the boiling point of the single molecule outputted by the pre-trained property calculation model — S82

FIG. 8

a property calculation model of a single molecule is constructed ⟩ S91

the number of groups of each group constituting a sample single molecule is acquired; wherein the physical property of the sample single molecule is known ⟩ S92

the number of groups of each group constituting the sample single molecule is inputted into the property calculation model ⟩ S93

a predicted physical property of the sample single molecule outputted by the property calculation model is acquired ⟩ S94

it is determined whether a deviation value between the predicted physical property and the physical property which is known is less than a preset deviation threshold

Yes — No

it is determined that the property calculation model converges ⟩ S95a

a contribution value corresponding to each group in the property calculation model is adjusted, until the property calculation model converges ⟩ S95b

FIG. 9

the number of groups of each group constituting the single molecule is compared with molecular information of a template single molecule with known physical properties pre-stored in a database ⟩ S101

it is determined whether there is a same template single molecule as the single molecule ⟩ S102

if there is a same template single molecule as the single molecule, the physical properties of the template single molecule are outputted as a physical property of the single molecule ⟩ S103

if there is not a same template single molecule as the single molecule, then the step of the inputting the number of groups of each group constituting the single molecule and the contribution value of each group to the physical property into a pre-trained property calculation model is performed ⟩ S104

FIG. 10

| a product prediction model is established | S111 |

| sample feedstock information for a sample feedstock is acquired | S112 |

| the set of reaction rules is trained by using the sample feedstock information, and the set of reaction rules that has been trained is fixed | S113 |

| the reaction rate algorithm is trained by using the sample feedstock information, and the reaction rate algorithm that has been trained is fixed, to obtain the product prediction model that has been trained | S114 |

FIG. 11

| the molecular composition of the sample feedstock is processed according to a preset set of reaction rules, to obtain a reaction pathway corresponding to each molecule in the molecular composition of the sample feedstock | S121 |

| first molecule composition of a device output product is obtained according to the reaction path corresponding to each molecule in the molecular composition of the sample feedstock | S122 |

| a first relative deviation is calculated according to the first molecular composition of the device output product and second molecular composition of the actual product | S123 |

it is determined whether the first relative deviation meets a preset condition — S124

No → a reaction rule in the set of reaction rules is adjusted — S126

Yes → the set of reaction rules is fixed — S125

FIG. 12

a reaction rate of a reaction path corresponding to each molecule in the molecular composition of the sample feedstock is respectively calculated according to the reaction rate algorithm

~ S131

predicted content of each molecule in a predicted product corresponding to the sample feedstock is obtained according to molecular content of each molecule in the sample feedstock and the reaction rate of the reaction path corresponding to the molecule

~ S132

a second relative deviation is calculated according to the predicted content of each molecule in the predicted product and the actual content of each molecule in the actual product

~ S133

S134

it is determined whether the second relative deviation meets a preset condition

No — Yes

a parameter in the reaction rate algorithm is adjusted

S136

the reaction rate algorithm is fixed

S135

FIG. 13

11    12    13

acquisition unit — first processing unit — second processing unit

fourth processing unit — third processing unit

15    14

FIG. 14

FIG. 15

FIG. 16

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LI J. et al.** Data-driven mathematical modeling and global optimization framework for entire petrochemical planning operations. *AICHE JOURNAL*, 2016, vol. 62 (9), 3020-3040
- **KHOR C. S. et al.** Petroleum refinery optimization. *OPTIMIZATION AND ENGINEERING*, 2016, vol. 18 (4), 943-989
- **GUEDDAR T. et al.** Disaggregation-aggregation based model reduction for refinery-wide optimization. *COMPUTERS & CHEMICAL ENGINEERING*, 2011, vol. 35 (9), 1838-1856
- **CHERNYSHEVA E. A. et al.** Enhancing the Efficiency of Refinery Crude Oil Distillation Process by Optimized Preliminary Feedstock Blending (Review). *PETROLEUM CHEMISTRY*, 2020, vol. 60 (1), 1-15
- **REN Y. et al.** Molecular reconstruction: Recent progress toward composition modeling of petroleum fractions. *CHEMICAL ENGENEERING JOURNAL*, 2018, vol. 357, 761-775